(19) Europäisches Patentamt
European Patent Office
Office européen des brevets

(11) EP 4 071 170 A1

(12) **EUROPEAN PATENT APPLICATION**
published in accordance with Art. 153(4) EPC

(43) Date of publication:
**12.10.2022 Bulletin 2022/41**

(21) Application number: **20892076.9**

(22) Date of filing: **13.11.2020**

(51) International Patent Classification (IPC):
***C07K 16/28*** *(2006.01)* ***A61K 39/395*** *(2006.01)*
***C12N 15/13*** *(2006.01)* ***A61P 35/00*** *(2006.01)*
***A61P 19/02*** *(2006.01)* ***A61P 29/00*** *(2006.01)*
***A61P 37/02*** *(2006.01)*

(52) Cooperative Patent Classification (CPC):
**A61K 39/395; A61P 19/02; A61P 29/00; A61P 35/00; A61P 35/02; A61P 37/02; C07K 16/00; C07K 16/28; C07K 16/46**

(86) International application number:
**PCT/CN2020/128820**

(87) International publication number:
**WO 2021/104052 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.11.2019 CN 201911207085**

(71) Applicants:
- **Keymed Biosciences Co.,Ltd.**
  **Chengdu, Sichuan 610219 (CN)**
- **Shanghai Lingyue Biopharma Co., Ltd.**
  **Shanghai 201203 (CN)**

(72) Inventors:
- **YU, Juntao**
  **Shanghai 201203 (CN)**
- **XU, Gang**
  **Chengdu, Sichuan 610219 (CN)**
- **CHEN, Bo**
  **Chengdu, Sichuan 610219 (CN)**

(74) Representative: **Elzaburu S.L.P.**
**Edificio Torre de Cristal**
**Paseo de la Castellana 259 C, planta 28**
**28046 Madrid (ES)**

# (54) PHARMACEUTICAL COMPOSITION, PREPARATION METHOD THEREFOR AND USE THEREOF

(57) The present disclosure relates to an antibody that binds to the CD38 protein or an antigen-binding portion thereof, a preparation method therefor and the use thereof. The antibody can bind to human CD38 with high affinity, has an inhibitory effect on the CD38 enzyme, has a CDC, ADCC, and/or ADCP killing activity with regard to different tumor cells, and has an anti-tumor function. Moreover, the antibody does not cause red blood cell lysis.

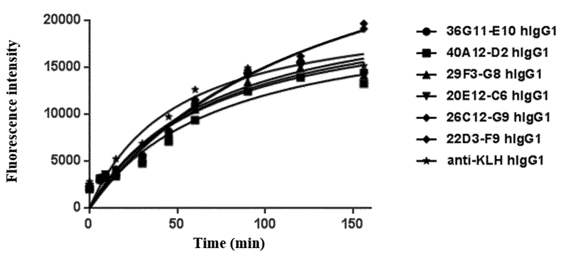


FIG. 4-1

EP 4 071 170 A1

## Description

### Technical Field

The present invention relates to a CD38 antibody, a preparation method and use thereof.

### Background Art

CD38 is a 46-kDa type-II transmembrane glycoprotein with an intracellular N-terminal cytoplasmic domain containing 21 amino acid residues, a transmembrane domain containing 21 amino acids, and a C-terminus extracellular domain containing 258 amino acid residues. CD38 regulates the migration and receptor-mediated adhesion through interaction with CD31 or hyaluronic acid, and in addition, CD38 has extracellular enzyme activity and is involved in the production of nucleotide metabolites, controlling intracellular calcium storage. CD38 is expressed at relatively low levels in some normal tissues, such as bone marrow and lymphoid cells, and in some non-hematopoietic cells; in contrast, it is highly expressed in plasma cells and some malignant cells, including multiple myeloma (MM) and chronic lymphocytic leukemia (CLL), thus CD38 is a target molecule for targeted multiple myeloma cell therapy due to the differences in expression abundance.

Multiple myeloma is considered a cancer of plasma cells, characterized by the accumulation of malignant cells in the bone marrow and the production of monoclonal immunoglobulins (M protein). Although the median overall survival of patients has improved significantly with the introducing of new therapies in recent years, patients typically relapse and develop resistance within one year. Daratumumab, a monoclonal antibody (mAb) that targets CD38, has been approved to be marketed for the treatment of relapsed/refractory multiple myeloma; several other mAbs in clinical trials such as isatuximab, MOR202, and TAK-079 target indications including newly diagnosed multiple myeloma in addition to relapsed/refractory multiple myeloma. CD38 antibodies also show good therapeutic effects in other malignancies, such as NK/T cell lymphoma, T cell acute lymphoblastic leukemia and immunoglobulin light chain amyloidosis.

Rheumatoid arthritis (RA) is an autoimmune disease in which a large number of B lymphocytes proliferate in the diseased synovial membrane. Flow cytometry analysis showed that the expression of CD38+, CD3+ CD38+ and CD56+ CD38+ subsets in the peripheral blood of RA patients was significantly increased, and the expression of CD38+ cells was significantly correlated with rheumatoid factor (RF) levels in RA patients; immunohistochemical results showed that CD38 was specifically and highly expressed in the synovial membrane of RA patients; the ELISA experiment demonstrated that after siRNA interference with CD38 gene, the expression levels of IL-1$\alpha$ and IL-$\beta$ in RASF medium are significantly decreased, and the increased expression of CD38 gene may be involved in the immune activation of RA patients (Expression of CD38 in peripheral blood and synovial tissue from patients with rheumatoid arthritis. Yue Longtao, et al. Current Immunology, pp. 89-93, 2014, No. 2). CD38 is significantly elevated in peripheral blood from patients with other autoimmune diseases such as systemic lupus erythematosus (SLE), and plays an important role in the occurrence and development of SLE. Although a variety of CD38 antibodies have shown very significant anti-tumor effects in vitro or preclinical animal studies, significant heterogeneity has been observed in clinical studies in the therapeutic effects and duration of response of the different antibodies, which may be related to the mechanism of action of the antibodies. For example, NK cells in the blood of patients treated with CD38 antibodies are rapidly depleted, and antibodies that primarily utilize antibody-dependent cellular cytotoxicity (ADCC) may be limited in function due to the lack of effector cells; antibodies with another mechanism of action (e. g., daratumumab has complement-dependent cytotoxicity, CDC) have been shown to be superior to other CD38 antibodies in clinical studies in overall response rate and survival time. It has been studied that CDC efficacy of an antibody is improved by introducing a mutation into IgG1 Fc or replacing the IgG1 hinge region with the IgG3 hinge region. Recently, it has also been studied that the mutation of IgG 1 Fc allows the monoclonal antibody to form a hexamer, which effectively enhances the killing efficacy of CD38 antibodies, but brings some difficulties in production and development.

### Summary of the Invention

The present inventors immunized mice with CD38 recombinant protein, and obtained a variety of novel high-affinity CD38 antibodies with the strongest CDC, ADCC, and antibody-dependent cellular phagocytosis (ADCP) activity of the similar antibodies, as well as the ability to inhibit CD38 extracellular enzyme activity and induce apoptosis under Fc cross-linking conditions. The present inventor further introduced new mutations in the Fc segment of the antibody, and the tumor killing efficiency of the novel CD38 antibody is far higher than that of the similar antibodies on the market at present. In one aspect, the present disclosure provides an antibody or antigen-binding portion thereof that binds to a CD38 protein.

In one aspect, the present disclosure provides a nucleic acid encoding the antibody or antigen-binding portion thereof according to the preceding aspect.

In one aspect, the present disclosure provides a vector comprising the nucleic acid according to the preceding aspect.

In one aspect, the present disclosure provides a cell comprising the vector according to the preceding aspect.

The antibody or antigen-binding portion thereof according to any one of the preceding aspects is provided, wherein the antibody or antigen-binding portion thereof is humanized.

In one aspect, the present disclosure provides a pharmaceutical composition or kit comprising an antibody or antigen-binding portion thereof or nucleic acids encoding the same according to any of the preceding aspects and a pharmaceutically acceptable carrier.

In one aspect, the present disclosure provides a method of treating the CD38-related disorders comprising the steps of administering to the mammal a therapeutically effective amount of an antibody or antigen-binding fragment thereof, a nucleic acid molecule, a vector, a cell and/or a pharmaceutical compositions according to any of the preceding aspects.

Use of the antibody or antigen-binding fragment thereof, the nucleic acid molecule, the vector, the cell and/or the pharmaceutical composition of any of the preceding aspects in the manufacture of a medicament or kit for the treatment of a CD38-related disorder in a mammal.

The antibodies may bind to human CD38 with high affinity, have an inhibitory effect on the CD38 enzyme, have CDC, ADCC, and/or ADCP killing activity on different tumor cells, and have an anti-tumor function. Also, the antibody does not cause lysis of red blood cells.

## Brief Description of the Drawings

FIG. 1 illustrates the recombinant expression of human CD38 extracellular domain protein in HEK 293 cells;
FIG. 2 illustrates flow cytometry analysis of human CD38/CHO stably transfected cells;
FIG. 3-1 illustrates CDC effects of chimeric antibodies on Duadi cells;
FIG. 3-2 illustrates CDC effects of chimeric antibodies on Duadi cells;
FIG. 4-1 illustrates inhibition of chimeric antibodies on CD38 enzymatic activity;
FIG. 4-2 illustrates inhibition of chimeric antibodies on CD38 enzymatic activity;
FIG. 5 illustrates ADCC killing activity of chimeric antibodies on Duadi cells;
FIG. 6 illustrates ADCP killing activity of chimeric antibodies on Duadi cells;
FIG. 7-1 illustrates CDC effects of different humanized antibodies on Daudi cells;
FIG. 7-2 illustrates CDC effects of different humanized antibodies on Daudi cells;
FIG. 8-1 illustrates ELISA affinity assay;
FIG. 8-2 illustrates binding of anti-CD38 humanized antibody to Daudi cells;
FIG. 8-3 illustrates binding of anti-CD38 humanized antibody to NALM-6 cells;
FIG. 8-4 illustrates binding of anti-CD38 humanized antibody to Ramos.2G6.4C10 cells;
FIG. 8-5 illustrates binding of anti-CD38 humanized antibody to TALL-1 cells;
FIG. 9 illustrates expression of CD38 on different tumor cells;
FIG. 10-1 illustrates CDC effects of anti-CD38 humanized antibody on Ramos.2G6.4C10;
FIG. 10-2 illustrates CDC effects of anti-CD38 humanized antibody on Daudi;
FIG. 10-3 illustrates CDC effects of anti-CD38 humanized antibody on TALL-1;
FIG. 11-1 illustrates ADCC effects of anti-CD38 humanized antibody on Ramos.2G6.4C10;
FIG. 11-2 illustrates ADCC effects of anti-CD38 humanized antibody on NALM-6;
FIG. 11-3 illustrates ADCC effects of anti-CD38 humanized antibody on RPMI-8226;
FIG. 11-4 illustrates ADCC effects of anti-CD38 humanized antibody on CCRF-CEM;
FIG. 11-5 illustrates ADCC effects of anti-CD38 humanized antibody on Daudi;
FIG. 11-6 illustrates ADCC effects of anti-CD38 humanized antibody on TALL-1;
FIG. 11-7 illustrates ADCC effects of anti-CD38 humanized antibody on MM.1S;
FIG. 11-8 illustrates ADCC effects of anti-CD38 humanized antibody on NCI-H929;
FIG. 11-9 illustrates ADCC effects of anti-CD38 humanized antibody on Raji;
FIG. 12-1 illustrates ADCP effects of anti-CD38 humanized antibody on Daudi;
FIG. 12-2 illustrates ADCP effects of anti-CD38 humanized antibody on TALL-1;
FIG. 12-3 illustrates ADCP effects of anti-CD38 humanized antibody on Ramos.2G6.4C10;
FIG. 12-4 illustrates ADCP effects of anti-CD38 humanized antibody on Raji;
FIG. 12-5 illustrates ADCP effects of anti-CD38 humanized antibody on NALM-6;
FIG. 13 illustrates the analysis of the inhibition of humanized CD38 antibodies on CD38 enzymatic activity;
FIG. 14 illustrates Daudi cell apoptosis induced by humanized CD38 antibody;

FIG. 15 illustrates human erythrocyte lysis assay induced by humanized CD38 antibody; and

FIG.16 illustrates effect of CD38 antibody on growth of human B-cell lymphoma Daudi subcutaneous xenografts.

**Detailed Description of the Invention**

**I. Definitions**

In the present invention, unless otherwise specified, scientific and technical terms used herein have the meanings commonly understood by those skilled in the art. Also, as used herein, protein and nucleic acid chemistry, molecular biology, cell and tissue culture, microbiology, immunology, and laboratory procedures used herein are terms and routine procedures widely used in the corresponding fields. Meanwhile, in order to better understand the present invention, definitions and explanations of related terms are provided below.

In one aspect, provided herein are antibodies (e. g., monoclonal antibodies) that specifically bind to CD38 and antigen-binding fragments thereof. In particular aspects, provided herein are anti-CD38 monoclonal antibodies that specifically bind to CD38, wherein the anti-CD38 antibodies include variants of the parent antibodies. In particular aspects, provided herein are antibodies that specifically bind to CD38 (e. g., human CD38). In particular aspects, provided herein are anti-CD38 antibodies comprising modifications in one or more amino acid residues (e. g., 5-13 amino acid substitutions in the framework region of the heavy chain variable region) that retain affinity for an antigen as compared to the parent antibody without the modification.

The term "about" or "approximately" as used herein means within plus or minus 10% of a given value or range, unless otherwise specified. Where integers are required, the term refers to integers within plus or minus 10% of a given value or range, rounded up or down to the nearest integer. The phrase "substantially identical" with respect to an antibody chain polypeptide sequence may be construed as an antibody chain exhibiting at least 60%, 65%, 70%, 75%, 80%, 85%, 90%, 95%, 96%, 97%, 98%, 99% or more sequence identity to a reference polypeptide sequence. The term with respect to a nucleotide sequence may be construed as a sequence of nucleotides exhibiting at least about 60%, 65%, 70%, 75%, 80%, 85%, 90%, 95%, 96%, 97%, 98%, 99% or more sequence identity to the reference nucleotide sequence.

Sequence "identical" or "identity" has the recognized meaning in this field, and the percentage of sequence identity between two nucleic acid or polypeptide molecules or regions can be calculated using published techniques. Sequence identity can be measured along the entire length of a polynucleotide or polypeptide or along a region of the molecule (see, for example: Computational Molecular Biology, Lesk, A.M., ed., Oxford University Pres, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M.and Devereux, J., eds., M Stockton Press, New York, 1991). While there are many methods of measuring identity between two polynucleotides or polypeptides, the term "identity" is well known to those skilled in the art (Carrillo, H. & Lipman, D. SIAM J Applied Math 48: 1073 (1988)).

"Substitution" variants are those in which at least one amino acid residue in the native sequence has been removed and inserted by a different amino acid at the same position. The substitution may be single, with only one amino acid in the molecule being substituted; or may be multiple, with two or more amino acids being substituted in the same molecule. Multiple substitutions may be at consecutive positions. Likewise, an amino acid may be substituted with multiple residues, wherein such variants include both substitutions and insertions. "Insertion" variants are those in which one or more amino acids are inserted immediately adjacent to an amino acid at a particular position in a native sequence. By immediately adjacent amino acid is meant attached to the $\alpha$-carboxy or $\alpha$-amino functional group of the amino acid. "Deletion" variants are those in which one or more amino acids in the native amino acid sequence have been removed. Typically, one or two amino acids are deleted in a particular region of the molecules of deletion variants.

With respect to the variable domains of antibodies, the term "variable" refers to certain portions of related molecules that differ widely in sequence between antibodies and are used for specifically recognizing and binding to a specific target of a particular antibody. However, the variability is not uniformly distributed throughout the variable domain of the antibody. Variability is concentrated on three segments called complementarity determining regions (CDR; i.e., CDR1, CDR2, and CDR3) or hypervariable regions, all of which are located within the variable domains of the light and heavy chains. The more conserved portions within the variable domains are referred to as framework (FR) regions or framework sequences. Each variable domain of native heavy and light chains comprises four FR regions, predominantly in a $\beta$ -sheet configuration, connected by three CDRs, which form loops connecting and in some cases forming part of the $\beta$ -sheet structure. The CDRs of each chain are typically joined together by adjacent FR regions and aid in the formation of antibody target binding sites (epitopes or determinants) by means of CDR from other chains (see Kabat et al. Sequences of Proteins of Immunological Interest, national Institute of Health, bethesda, MD (1987)). As used herein, the immunoglobulin amino acid residue numbering is in accordance with the Kabat numbering scheme (Kabat et al.) for numbering amino acid residues in the immunoglobulin, unless otherwise indicated. One CDR may have the ability to specifically bind to a cognate epitope.

As used herein, an "antibody fragment" or "antigen-binding fragment" of an antibody refers to any portion of a full-length antibody that is less than full-length, but that comprises at least a portion of the variable region (e. g., one or more CDRs and/or one or more antibody binding sites) of the antibody that binds antigen, thus retaining binding specificity as well as at least a portion of the specific binding capacity of the full-length antibody. Thus, an antigen-binding fragment refers to an antibody fragment that comprises an antigen-binding portion that binds the same antigen as the antibody from which the antibody fragment was derived. Antibody fragments include antibody derivatives produced by enzymatic treatment of full-length antibodies, as well as synthetic derivatives, e. g., recombinant derivatives. Antibodies include antibody fragments. Examples of antibody fragments include, but are not limited to, Fab, Fab', $F(ab')_2$, single chain Fv (scFv), Fv, dsFv, diabodies, Fd, and Fd' fragments and other fragments, including modified fragments (see, e. g., Methods in Molecular Biology, Vol 207: Recombinant Antibodies for Cancer Therapy Methods and Protocols (2003); Chapter 1; p 3-25, Kipriyanov). The fragments may comprise multiple chains linked together, for example, by disulfide bonds and/or by peptide linkers. Antibody fragments generally comprise at least or about 50 amino acids, and typically at least or about 200 amino acids. Antigen-binding fragments include any antibody fragment that, when inserted into an antibody framework (e. g., by displacement of the corresponding region), results in an antibody that immunospecifically binds (i. e., exhibiting a Ka of at least or at least about $10^7$-$10^8$M-1) an antigen. A "functional fragment" or "analog of an anti-CD38 antibody" is a fragment or analog that prevents or substantially reduces the ability of the receptor to bind a ligand or initiate signal transduction. As used herein, functional fragments generally have the same meaning as "antibody fragments" and, in the case of antibodies, can refer to fragments that prevent or substantially reduce the ability of the receptor to bind a ligand or initiate signal transduction, e. g., Fv, Fab, $F(ab')_2$, and the like. An "Fv" fragment consists of a dimer ($V_H$-$V_L$ dimer) of a variable domain of a heavy chain and a variable domain of a light chain formed by non-covalent association. In this configuration, the three CDRs of each variable domain interact to define a target binding site on the surface of the $V_H$-$V_L$ dimer, as is the case with intact antibodies. The six CDRs together confer target binding specificity to the intact antibody. However, even a single variable domain (or half of an Fv comprising only three target-specific CDRs) may still have the ability to recognize and bind to targets.

As used herein, the term "bispecific antibody (BsAb)" refers to an antibody and/or antigen-binding molecule capable of specifically binding to two different antigenic determinants. Generally, a bispecific antibody and/or antigen-binding molecule comprises two antigen-binding sites, each of which is specific for different antigenic determinants. In some embodiments, the bispecific antibody and/or antigen binding molecule is capable of binding two antigenic determinants simultaneously, particularly two antigenic determinants expressed on two different cells.

As used herein, "monoclonal antibody" refers to a population of identical antibodies, meaning that each individual antibody molecule in the population of monoclonal antibodies is identical to another antibody molecule. This property is in contrast to the property of a population of polyclonal antibodies comprising antibodies having a plurality of different sequences. Monoclonal antibodies can be prepared by a number of well-known methods (Smith et al. (2004) J.Clin.Pathol.57, 912-917; and Nelson et al., J Clin Pathol (2000), 53, 111-117). For example, monoclonal antibodies can be prepared by immortalizing B cells, for example, by fusing B cells with myeloma cells to generate hybridoma cell lines or by infecting B cells with a virus such as EBV. Recombinant techniques can also be used to prepare antibodies from clonal populations of host cells in vitro by transforming host cells with plasmids carrying artificial sequences encoding the nucleotides of the antibodies.

As used herein, the term "hybridoma" or "hybridoma cell" refers to a cell or cell line (typically a myeloma or lymphoma cell) resulting from the fusion of antibody-producing lymphocytes and non-antibody-producing cancer cells. As known to those of ordinary skill in the art, hybridomas can be propagated and continuously supplied to produce a particular monoclonal antibody. Methods for producing hybridomas are known in the art (see, e. g., Harlow & Lane, 1988). When referring to the term "hybridoma" or "hybridoma cell", it also includes subclones and progeny cells of hybridomas.

As used herein, a full-length antibody is an antibody having two full-length heavy chains (e. g. VH-CH1-CH2-CH3 or VH-CH1-CH2-CH3-CH4) and two full-length light chains (VL-CL) and a hinge region, e. g., an antibody naturally produced by an antibody secreting B cell as well as a synthetically produced antibody having the same domain.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to a form of non-human (e. g., mouse) antibody that is a chimeric immunoglobulin, immunoglobulin chain or fragment thereof (e. g., Fv, Fab, Fab', $F(ab')_2$ or other antigen-binding subsequence of an antibody), containing minimal sequence derived from a non-human immunoglobulin. Preferably, the humanized antibody is a human immunoglobulin (recipient antibody) in which residues from a complementarity determining region (CDR) of the recipient antibody are replaced by CDR residues from a non-human species (donor antibody) such as mouse, rat, or rabbit having the desired specificity, affinity, and capacity.

Furthermore, in humanization it is also possible to mutate amino acid residues within the CDR1, CDR2 and/or CDR3 regions of VH and/or VL, thereby improving one or more binding properties (e. g., affinity) of the antibody. Mutations can be introduced, e. g. by PCR-mediated mutagenesis, and their effect on antibody binding or other functional properties can be assessed using the in vitro or in vivo assays described herein. Typically, conservative mutations are introduced. Such mutations may be amino acid substitutions, additions, or deletions. In addition, mutations within CDR typically do not exceed one or two. Thus, humanized antibodies of the present invention also encompass antibodies comprising one or two amino acid mutations within CDR.

As used herein, the term "CDR" refers to a complementarity-determining region known to have three CDRs per heavy and light chain of an antibody molecule. CDR is also known as a hypervariable region and is present in the variable region of each of the heavy and light chains of an antibody with a very high site of variability in the primary structure of CDR. In the present specification, the CDR of the heavy chain is represented by CDR1, CDR2, CDR3 from the amino terminus of the amino terminal sequence of the heavy chain, and the CDR of the light chain is represented by CDR1, CDR2, CDR3 from the amino terminus of the amino terminal sequence of the light chain. These sites are adjacent to each other in the tertiary structure and determine the specificity of the antigen to which the antibody binds.

As used herein, the term "epitope" refers to any antigenic determinant on an antigen to which the paratope of an antibody binds. Epitopic determinants usually comprise chemically active surface types of molecules, such as amino acids or sugar side chains, and usually have specific three-dimensional structural characteristics as well as specific charge characteristics.

As used herein, "specific binding" or "immunospecifically binding" with respect to an antibody or antigen-binding fragment thereof is used interchangeably herein and refers to the ability of an antibody or antigen-binding fragment to form one or more non-covalent bonds with the same antigen through non-covalent interactions between the antibody and the antibody binding site of the antigen. The antigen may be an isolated antigen or present in a tumor cell. Typically, an antibody that immunospecifically binds (or specifically binds) to an antigen is one that binds to the antigen with an affinity constant Ka of about or $1\times10^{7}M^{-1}$ or $1\times10^{8}M^{-1}$ or more (or a dissociation constant (Kd) of $1\times10^{-7}M$ or $1\times10^{-8}M$ or less). Affinity constants can be determined by standard kinetic methods for antibody reactions, e. g., immunoassays, surface plasmon resonance (SPR) (Rich and Myszka (2000) Curr. Opin. Biotechnol 11:54; Englebienne (1998) Analyst. 123:1599), isothermal titration calorimetry (ITC), or other kinetic interaction assays known in the art (see, e. g., Paul, ed., Fundamental Immunology, 2nd ed., Raven Press, New York, pages 332-336 (1989); see also U.S. Patent No. 7, 229, 619) which describes an exemplary SPR and ITC method for calculating the binding affinity of an antibody. Instruments and methods for real-time detecting and monitoring the rate of binding are known and commercially available (see, BiaCore 2000, Biacore AB, Upsala, Sweden and GE Healthcare Life Sciences; Malmqvist (2000) Biochem.Soc.Trans. 27:335). As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), typically linked together by phosphodiester bonds. As used herein, the term "nucleic acid molecule" is intended to include DNA molecules and RNA molecules. The nucleic acid molecule may be single-stranded or doublestranded, and may be cDNA.

As used herein, an isolated nucleic acid molecule is a nucleic acid molecule isolated from other nucleic acid molecules present in the natural source of the nucleic acid molecule. An "isolated" nucleic acid molecule, such as a cDNA molecule, can be substantially free of other cellular material or culture medium when prepared by recombinant techniques, or substantially free of chemical precursors or other chemical components when chemically synthesized. Exemplary isolated nucleic acid molecules provided herein include isolated nucleic acid molecules encoding a provided antibody or antigen-binding fragment.

As used herein, "operably linked" with respect to a nucleotide sequence, region, element or domain means that the nucleic acid regions are functionally related to one another. For example, a promoter may be operably linked to a nucleic acid encoding a polypeptide such that the promoter regulates or mediates transcription of the nucleic acid.

Also provided are "conservative sequence modifications" of the sequences set forth in the sequence listings described herein, i.e., nucleotide and amino acid sequence modifications that do not eliminate binding of an antibody encoded by a nucleotide sequence or containing an amino acid sequence to an antigen. These conservative sequence modifications include substitutions of conservative nucleotide and amino acid and additions and deletions of nucleotide and amino acid. For example, modifications can be introduced into the sequence listings described herein by standard techniques known in the art, such as site-directed mutagenesis and PCR-mediated mutagenesis. Conservative sequence modifications include conservative amino acid substitutions in which an amino acid residue is replaced with an amino acid residue having a similar side chain. Families of amino acid residues having similar side chains have been defined in the art. These families include amino acids containing basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, a predicted nonessential amino acid residue in an anti-CD38 antibody is preferably replaced with another amino acid residue from the same side chain family. Methods for identifying conservative substitutions of nucleotides and amino acids that do not eliminate antigen binding are well known in the art (see, e. g., Brummell et al., Biochem. 32: 1180-1187 (1993); Kobayashi et al., Protein Eng. 12(10): 879-884 (1999); Burks et al., Proc. Natl. Acad. Sci. USA 94: 412-417 (1997)). Alternatively, in another embodiment, mutations can be randomly introduced along all or a portion of the anti-CD38 antibody coding sequence, e.g., by saturation mutagenesis, and the resulting modified anti-CD38 antibodies can be screened for improved binding activity.

As used herein, "expression" refers to a process of producing a polypeptide by transcription and translation of a polynucleotide. The level of expression of a polypeptide can be assessed using any method known in the art, including, for example, methods of determining the amount of polypeptide produced from a host cell. Such methods may include, but are not limited to, quantitation of polypeptides in cell lysates by ELISA, gel electrophoresis followed by Coomassie blue staining, Lowry protein assay, and Bradford protein assay.

As used herein, a "host cell" is a cell for receiving, maintaining, replicating, and expanding a vector. The host cell may also be used to express a polypeptide encoded by the vector. The nucleic acid contained in the vector replicates during the host cell division, thereby amplifying the nucleic acid. The host cell may be a eukaryotic cell or a prokaryotic cell. Suitable host cells include, but are not limited to, CHO cells, various COS cells, HeLa cells, HEK cells such as HEK 293 cells.

As used herein, a "vector" is a replicable nucleic acid from which one or more heterologous proteins can be expressed when the vector is transformed into an appropriate host cell. References to vectors include those into which a nucleic acid encoding a polypeptide or fragment thereof may be introduced, typically by restriction digestion and ligation. References to vectors also include those comprising a nucleic acid encoding a polypeptide. Vectors are used to introduce a nucleic acid encoding a polypeptide into a host cell, to amplify the nucleic acid, or to express/display the polypeptide encoded by the nucleic acid. The vector generally remains episomal, but can be designed to allow integration of a gene or portion thereof into the chromosome of the genome. Also contemplated are vectors for artificial chromosomes, such as yeast artificial vectors and mammalian artificial chromosomes. The selection and use of such vehicles is well known to those skilled in the art.

As used herein, a vector also includes a "virus vector" or a "viral vector". A viral vector is an engineered virus that is operably linked to a foreign gene to transfer (as a vehicle or shuttle) the foreign gene into a cell.

As used herein, an "expression vector" includes a vector capable of expressing a DNA operably linked to regulatory sequences, such as a promoter region, capable of affecting the expression of such DNA fragments. Such additional fragments may include promoter and terminator sequences, and optionally may include one or more origins of replication, one or more selectable markers, enhancers, polyadenylation signals, etc. Expression vectors are typically derived from plasmid or viral DNA, or may contain elements of both. Thus, an expression vector refers to a recombinant DNA or RNA construct, such as a plasmid, phage, recombinant virus or other vectors, which results in the expression of a cloned DNA when introduced into an appropriate host cell. Appropriate expression vectors are well known to those skilled in the art and include expression vectors that are replicable in eukaryotic and/or prokaryotic cells as well as expression vectors that remain episomal or that are integrated into the host cell genome.

As used herein, "treating" a subject with a disease or condition means that the subject's symptoms are partially or completely alleviated or remain unchanged after the treatment. Thus, treatment includes prevention, treatment and/or cure. The prevention refers to preventing the underlying disease and/or preventing the worsening of symptoms or the progression of the disease. The treatment also includes any antibody or antigen-binding fragment thereof provided and any pharmaceutical use of the compositions provided herein.

As used herein, "therapeutic effect" refers to an effect resulting from treatment of a subject that alters, typically improves or ameliorates a symptom of a disease or condition, or cures a disease or condition.

As used herein, a "therapeutically effective amount" or "therapeutically effective dose" refers to an amount of a substance, compound, material, or composition comprising a compound that is at least sufficient to produce a therapeutic effect after being administered to a subject. Thus, it is an amount necessary to prevent, cure, ameliorate, block, or partially block the symptoms of a disease or disorder. As used herein, a "prophylactically effective amount" or "prophylactically effective dose" refers to an amount of a substance, compound, material, or composition comprising a compound that, when administered to a subject, has the desired prophylactic effect, e. g., preventing or delaying the occurrence or recurrence of a disease or condition, reducing the likelihood of the occurrence or recurrence of a disease or condition. A fully prophylactically effective dose need not occur by administration of one dose, and may occur only after administration of a series of doses. Thus, a prophylactically effective amount may be administered one or more times

As used herein, the term "patient" refers to a mammal, such as a human.

## II. **Detailed Description of Embodiments**

In one aspect, the present disclosure provides an antibody or antigen-binding portion thereof that binds to CD38, comprising heavy chain CDRs selected from the amino acid sequences of SEQ ID NOs: 2-4, 12-14, 22-24, 32-34, 42-44, 52-54, 62-64, 72-74, 82-84, 92-94, 102-144, 112-114, 122-124, 132-34, 142-144, 152-154, 162-164, 172-174, 182-184, 187-189, 192-194, 197-199, 202-204, 207-209, 212-214, 217-219, 222-224, 227-229, 232-234, or any variant thereof, and/or light chain CDRs selected from the amino acid sequences of SEQ ID NOs: 7-9, 17-19, 27-29, 37-39, 47-49, 57-59, 67-69, 77-79, 87-89, 97-99, 107-109, 117-119, 127-129, 137-139, 147-149, 157-159, 167-169, 177-179, 237-239, 242-244, 247-249, 252-254, 257-259, 262-264, 267-269, or any variant thereof. The antibody or antigen-binding portion thereof according to the preceding aspect comprises a heavy chain CDR1 selected from the amino acid sequences of SEQ ID NOs: 2, 12, 22, 32, 42, 52, 62, 72, 82, 92, 102, 112, 122, 132, 142, 152, 162, 172, 182, 187, 192, 197, 202, 207, 212, 217, 222, 227, 232, or any variant thereof, a heavy chain CDR2 selected from the amino acid sequences of SEQ ID NOs: 3, 13, 23, 33, 43, 53, 63, 73, 83, 93, 103, 113, 123, 133, 143, 153, 163, 173, 183, 188, 193, 198, 203, 208, 213, 218, 223, 228, 233, or any variant thereof, and a heavy chain CDR3 selected from the amino acid sequences of SEQ ID NOs: 4, 14, 24, 34, 44, 54, 64, 74, 84, 94, 104, 114, 124, 134, 144, 154, 164, 174, 189, 194, 199, 204, 209, 214, 219, 224, 229, 234, or any variant thereof; and/or a light chain CDR1 selected from the amino acid sequences of SEQ ID NOs: 7, 17, 27, 37, 47, 57, 67, 77, 87, 97, 107, 117, 127, 137, 147, 157, 167, 177, 237, 242, 247, 252, 257, 262, 267, or any variant thereof, a light chain CDR2 selected from the amino acid sequences of SEQ ID NOs: 8, 18, 28, 38, 48, 58, 68, 78, 88, 98, 108, 118, 128, 138, 148, 158, 168, 178, 238, 243, 248, 253, 258, 263, 268, or any variant thereof, and a light chain CDR3 selected from the amino acid sequences of SEQ ID NOs: 9, 19, 29, 39, 49, 59, 69, 79, 89, 99, 109, 119, 129, 139, 149, 159, 169, 179, 239, 244, 249, 254, 259, 264, 269, or any variant thereof.

The antibody or antigen-binding portion thereof according to the preceding aspect comprises a CDR combination of heavy and light chains selected from the group consisting of:

(1) the heavy chain CDR1, CDR2, and CDR3 sequences including SEQ ID NOs: 2-4, respectively, and/or the light chain CDR1, CDR2, and CDR3 sequences including SEQ ID NOs: 7-9, respectively;
(2) the heavy chain CDR1, CDR2, and CDR3 sequences including SEQ ID NOs: 12-14, respectively, and/or the light chain CDR1, CDR2, and CDR3 sequences including SEQ ID NOs: 17-19, respectively;
(3) the heavy chain CDR1, CDR2, and CDR3 sequences including SEQ ID NOs: 22-24, respectively, and/or the light chain CDR1, CDR2, and CDR3 sequences including SEQ ID NOs: 27-29, respectively;
(4) the heavy chain CDR1, CDR2, and CDR3 sequences including SEQ ID NOs: 32-34, respectively, and/or the light chain CDR1, CDR2, and CDR3 sequences including SEQ ID NOs: 37-39, respectively;
(5) the heavy chain CDR1, CDR2, and CDR3 sequences including SEQ ID NOs: 42-44, respectively, and/or the light chain CDR1, CDR2, and CDR3 sequences including SEQ ID NOs: 47-49, respectively;
(6) the heavy chain CDR1, CDR2, and CDR3 sequences including SEQ ID NOs: 52-54, respectively, and/or the light chain CDR1, CDR2, and CDR3 sequences including SEQ ID NOs: 57-59, respectively;
(7) the heavy chain CDR1, CDR2, and CDR3 sequences including SEQ ID NOs: 62-64, respectively, and/or the light chain CDR1, CDR2, and CDR3 sequences including SEQ ID NOs: 67-69, respectively;
(8) the heavy chain CDR1, CDR2, and CDR3 sequences including SEQ ID NOs: 72-74, respectively, and/or the light chain CDR1, CDR2, and CDR3 sequences including SEQ ID NOs: 77-79, respectively;
(9) the heavy chain CDR1, CDR2, and CDR3 sequences including SEQ ID NOs: 82-84, respectively, and/or the light chain CDR1, CDR2, and CDR3 sequences including SEQ ID NOs: 87-89, respectively;
(10) the heavy chain CDR1, CDR2, and CDR3 sequences including SEQ ID NOs: 92-94, respectively, and/or the light chain CDR1, CDR2, and CDR3 sequences including SEQ ID NOs: 97-99, respectively;
(11) the heavy chain CDR1, CDR2, and CDR3 sequences including SEQ ID NOs: 102-104, respectively, and/or the light chain CDR1, CDR2, and CDR3 sequences including SEQ ID NOs: 107-109, respectively;
(12) the heavy chain CDR1, CDR2, and CDR3 sequences including SEQ ID NOs: 112-114, respectively, and/or the light chain CDR1, CDR2, and CDR3 sequences including SEQ ID NOs: 117-119, respectively;
(13) the heavy chain CDR1, CDR2, and CDR3 sequences including SEQ ID NOs: 122-124, respectively, and/or the light chain CDR1, CDR2, and CDR3 sequences including SEQ ID NOs: 127-129, respectively;
(14) the heavy chain CDR1, CDR2, and CDR3 sequences including SEQ ID NOs: 132-134, respectively, and/or the light chain CDR1, CDR2, and CDR3 sequences including SEQ ID NOs: 137-139, respectively;
(15) the heavy chain CDR1, CDR2, and CDR3 sequences including SEQ ID NOs: 142-144, respectively, and/or the light chain CDR1, CDR2, and CDR3 sequences including SEQ ID NOs: 147-149, respectively;
(16) the heavy chain CDR1, CDR2, and CDR3 sequences including SEQ ID NOs: 152-154, respectively, and/or the light chain CDR1, CDR2, and CDR3 sequences including SEQ ID NOs: 157-159, respectively;
(17) the heavy chain CDR1, CDR2, and CDR3 sequences including SEQ ID NOs: 162-164, respectively, and/or the light chain CDR1, CDR2, and CDR3 sequences including SEQ ID NOs: 167-169, respectively;
(18) the heavy chain CDR1, CDR2, and CDR3 sequences including SEQ ID NOs: 172-174, respectively, and/or the light chain CDR1, CDR2, and CDR3 sequences including SEQ ID NOs: 177-179, respectively;
(19) the heavy chain CDR1, CDR2, and CDR3 sequences including SEQ ID NOs: 182-184, respectively, and/or the light chain CDR1, CDR2, and CDR3 sequences including SEQ ID NOs: 237-239, respectively;

(20) the heavy chain CDR1, CDR2, and CDR3 sequences including SEQ ID NOs: 182-184, respectively, and/or the light chain CDR1, CDR2, and CDR3 sequences including SEQ ID NOs: 242-244, respectively;
(21) the heavy chain CDR1, CDR2, and CDR3 sequences including SEQ ID NOs: 182-184, respectively, and/or the light chain CDR1, CDR2, and CDR3 sequences including SEQ ID NOs: 247-249, respectively;
(22) the heavy chain CDR1, CDR2, and CDR3 sequences including SEQ ID NOs: 182-184, respectively, and/or the light chain CDR1, CDR2, and CDR3 sequences including SEQ ID NOs: 252-254, respectively;
(23) the heavy chain CDR1, CDR2, and CDR3 sequences including SEQ ID NOs: 182-184, respectively, and/or the light chain CDR1, CDR2, and CDR3 sequences including SEQ ID NOs: 257-259, respectively;
(24) the heavy chain CDR1, CDR2, and CDR3 sequences including SEQ ID NOs: 187-189, respectively, and/or the light chain CDR1, CDR2, and CDR3 sequences including SEQ ID NOs: 237-239, respectively;
(25) the heavy chain CDR1, CDR2, and CDR3 sequences including SEQ ID NOs: 187-189, respectively, and/or the light chain CDR1, CDR2, and CDR3 sequences including SEQ ID NOs: 242-244, respectively;
(26) the heavy chain CDR1, CDR2, and CDR3 sequences including SEQ ID NOs: 187-189, respectively, and/or the light chain CDR1, CDR2, and CDR3 sequences including SEQ ID NOs: 247-249, respectively;
(27) the heavy chain CDR1, CDR2, and CDR3 sequences including SEQ ID NOs: 187-189, respectively, and/or the light chain CDR1, CDR2, and CDR3 sequences including SEQ ID NOs: 252-254, respectively;
(28) the heavy chain CDR1, CDR2, and CDR3 sequences including SEQ ID NOs: 187-189, respectively, and/or the light chain CDR1, CDR2, and CDR3 sequences including SEQ ID NOs: 257-259, respectively;
(29) the heavy chain CDR1, CDR2, and CDR3 sequences including SEQ ID NOs: 192-194, respectively, and/or the light chain CDR1, CDR2, and CDR3 sequences including SEQ ID NOs: 237-239, respectively;
(30) the heavy chain CDR1, CDR2, and CDR3 sequences including SEQ ID NOs: 192-194, respectively, and/or the light chain CDR1, CDR2, and CDR3 sequences including SEQ ID NOs: 242-244, respectively;
(31) the heavy chain CDR1, CDR2, and CDR3 sequences including SEQ ID NOs: 192-194, respectively, and/or the light chain CDR1, CDR2, and CDR3 sequences including SEQ ID NOs: 247-249, respectively;
(32) the heavy chain CDR1, CDR2, and CDR3 sequences including SEQ ID NOs: 192-194, respectively, and/or the light chain CDR1, CDR2, and CDR3 sequences including SEQ ID NOs: 252-254, respectively;
(33) the heavy chain CDR1, CDR2, and CDR3 sequences including SEQ ID NOs: 192-194, respectively, and/or the light chain CDR1, CDR2, and CDR3 sequences including SEQ ID NOs: 257-259, respectively;
(34) the heavy chain CDR1, CDR2, and CDR3 sequences including SEQ ID NOs: 197-199, respectively, and/or the light chain CDR1, CDR2, and CDR3 sequences including SEQ ID NOs: 237-239, respectively;
(35) the heavy chain CDR1, CDR2, and CDR3 sequences including SEQ ID NOs: 197-199, respectively, and/or the light chain CDR1, CDR2, and CDR3 sequences including SEQ ID NOs: 242-244, respectively;
(36) the heavy chain CDR1, CDR2, and CDR3 sequences including SEQ ID NOs: 197-199, respectively, and/or the light chain CDR1, CDR2, and CDR3 sequences including SEQ ID NOs: 247-249, respectively;
(37) the heavy chain CDR1, CDR2, and CDR3 sequences including SEQ ID NOs: 197-199, respectively, and/or the light chain CDR1, CDR2, and CDR3 sequences including SEQ ID NOs: 252-254, respectively;
(38) the heavy chain CDR1, CDR2, and CDR3 sequences including SEQ ID NOs: 197-199, respectively, and/or the light chain CDR1, CDR2, and CDR3 sequences including SEQ ID NOs: 257-259, respectively;
(39) the heavy chain CDR1, CDR2, and CDR3 sequences including SEQ ID NOs: 202-204, respectively, and/or the light chain CDR1, CDR2, and CDR3 sequences including SEQ ID NOs: 237-239, respectively;
(40) the heavy chain CDR1, CDR2, and CDR3 sequences including SEQ ID NOs: 202-204, respectively, and/or the light chain CDR1, CDR2, and CDR3 sequences including SEQ ID NOs: 242-244, respectively;
(41) the heavy chain CDR1, CDR2, and CDR3 sequences including SEQ ID NOs: 202-204, respectively, and/or the light chain CDR1, CDR2, and CDR3 sequences including SEQ ID NOs: 247-249, respectively;
(42) the heavy chain CDR1, CDR2, and CDR3 sequences including SEQ ID NOs: 202-204, respectively, and/or the light chain CDR1, CDR2, and CDR3 sequences including SEQ ID NOs: 252-254, respectively;
(43) the heavy chain CDR1, CDR2, and CDR3 sequences including SEQ ID NOs: 202-204, respectively, and/or the light chain CDR1, CDR2, and CDR3 sequences including SEQ ID NOs: 257-259, respectively;
(44) the heavy chain CDR1, CDR2, and CDR3 sequences including SEQ ID NOs: 207-209, respectively, and/or the light chain CDR1, CDR2, and CDR3 sequences including SEQ ID NOs: 237-239, respectively;
(45) the heavy chain CDR1, CDR2, and CDR3 sequences including SEQ ID NOs: 207-209, respectively, and/or the light chain CDR1, CDR2, and CDR3 sequences including SEQ ID NOs: 242-244, respectively;
(46) the heavy chain CDR1, CDR2, and CDR3 sequences including SEQ ID NOs: 207-209, respectively, and/or the light chain CDR1, CDR2, and CDR3 sequences including SEQ ID NOs: 247-249, respectively;
(47) the heavy chain CDR1, CDR2, and CDR3 sequences including SEQ ID NOs: 207-209, respectively, and/or the light chain CDR1, CDR2, and CDR3 sequences including SEQ ID NOs: 252-254, respectively;
(48) the heavy chain CDR1, CDR2, and CDR3 sequences including SEQ ID NOs: 207-209, respectively, and/or the light chain CDR1, CDR2, and CDR3 sequences including SEQ ID NOs: 257-259, respectively;

(49) the heavy chain CDR1, CDR2, and CDR3 sequences including SEQ ID NOs: 212-214, respectively, and/or the light chain CDR1, CDR2, and CDR3 sequences including SEQ ID NOs: 262-264, respectively;
(50) the heavy chain CDR1, CDR2, and CDR3 sequences including SEQ ID NOs: 212-214, respectively, and/or the light chain CDR1, CDR2, and CDR3 sequences including SEQ ID NOs: 267-269, respectively;
(51) the heavy chain CDR1, CDR2, and CDR3 sequences including SEQ ID NOs: 217-219, respectively, and/or the light chain CDR1, CDR2, and CDR3 sequences including SEQ ID NOs: 262-264, respectively;
(52) the heavy chain CDR1, CDR2, and CDR3 sequences including SEQ ID NOs: 217-219, respectively, and/or the light chain CDR1, CDR2, and CDR3 sequences including SEQ ID NOs: 267-269, respectively;
(53) the heavy chain CDR1, CDR2, and CDR3 sequences including SEQ ID NOs: 222-224, respectively, and/or the light chain CDR1, CDR2, and CDR3 sequences including SEQ ID NOs: 262-264, respectively;
(54) the heavy chain CDR1, CDR2, and CDR3 sequences including SEQ ID NOs: 222-224, respectively, and/or the light chain CDR1, CDR2, and CDR3 sequences including SEQ ID NOs: 267-269, respectively;
(55) the heavy chain CDR1, CDR2, and CDR3 sequences including SEQ ID NOs: 227-229, respectively, and/or the light chain CDR1, CDR2, and CDR3 sequences including SEQ ID NOs: 262-264, respectively;
(56) the heavy chain CDR1, CDR2, and CDR3 sequences including SEQ ID NOs: 227-229, respectively, and/or the light chain CDR1, CDR2, and CDR3 sequences including SEQ ID NOs: 267-269, respectively;
(57) the heavy chain CDR1, CDR2, and CDR3 sequences including SEQ ID NOs: 232-234, respectively, and/or the light chain CDR1, CDR2, and CDR3 sequences including SEQ ID NOs: 262-264, respectively;
(58) the heavy chain CDR1, CDR2, and CDR3 sequences including SEQ ID NOs: 232-234, respectively, and/or the light chain CDR1, CDR2, and CDR3 sequences including SEQ ID NOs: 267-269, respectively.

The antibody or antigen-binding portion thereof according to the preceding aspect comprises a heavy chain variable region selected from the amino acid sequence of SEQ ID NOs: 1, 11, 21, 31, 41, 51, 61, 71, 81, 91, 101, 111, 121, 131, 141, 151, 161, 171, 181, 186, 191, 196, 201, 206, 211, 216, 221, 226, 231, or any variant thereof, and/or a light chain variable region selected from the amino acid sequence of SEQ ID NOs: 6, 16, 26, 36, 46, 56, 66, 76, 86, 96, 106, 116, 126, 136, 146, 156, 166, 176, 236, 241, 246, 251, 256, 261, 266, or any variant thereof.

In one aspect, the present disclosure relates to an antibody or antigen-binding portion thereof that binds human CD38, comprising a heavy chain variable region of the amino acid sequence of SEQ ID NO: 1 or any variant thereof, and a light chain variable region of the amino acid sequence of SEQ ID NO: 6 or any variant thereof.

In one aspect, the present disclosure relates to an antibody or antigen-binding portion thereof that binds human CD38, comprising a heavy chain variable region of the amino acid sequence of SEQ ID NO: 11 or any variant thereof, and a light chain variable region of the amino acid sequence of SEQ ID NO: 16 or any variant thereof.

In one aspect, the present disclosure relates to an antibody or antigen-binding portion thereof that binds human CD38, comprising a heavy chain variable region of the amino acid sequence of SEQ ID NO: 21 or any variant thereof, and a light chain variable region of the amino acid sequence of SEQ ID NO: 26 or any variant thereof.

In one aspect, the present disclosure relates to an antibody or antigen-binding portion thereof that binds human CD38, comprising a heavy chain variable region of the amino acid sequence of SEQ ID NO: 31 or any variant thereof, and a light chain variable region of the amino acid sequence of SEQ ID NO: 36 or any variant thereof.

In one aspect, the present disclosure relates to an antibody or antigen-binding portion thereof that binds human CD38, comprising a heavy chain variable region of the amino acid sequence of SEQ ID NO: 41 or any variant thereof, and a light chain variable region of the amino acid sequence of SEQ ID NO: 46 or any variant thereof.

In one aspect, the present disclosure relates to an antibody or antigen-binding portion thereof that binds human CD38, comprising a heavy chain variable region of the amino acid sequence of SEQ ID NO: 51 or any variant thereof, and a light chain variable region of the amino acid sequence of SEQ ID NO: 56 or any variant thereof.

In one aspect, the present disclosure relates to an antibody or antigen-binding portion thereof that binds human CD38, comprising a heavy chain variable region of the amino acid sequence of SEQ ID NO: 61 or any variant thereof, and a light chain variable region of the amino acid sequence of SEQ ID NO: 66 or any variant thereof.

In one aspect, the present disclosure relates to an antibody or antigen-binding portion thereof that binds human CD38, comprising a heavy chain variable region of the amino acid sequence of SEQ ID NO: 71 or any variant thereof, and a light chain variable region of the amino acid sequence of SEQ ID NO: 76 or any variant thereof.

In one aspect, the present disclosure relates to an antibody or antigen-binding portion thereof that binds human CD38, comprising a heavy chain variable region of the amino acid sequence of SEQ ID NO: 81 or any variant thereof, and a light chain variable region of the amino acid sequence of SEQ ID NO: 86 or any variant thereof.

In one aspect, the present disclosure relates to an antibody or antigen-binding portion thereof that binds human CD38, comprising a heavy chain variable region of the amino acid sequence of SEQ ID NO: 91 or any variant thereof, and a light chain variable region of the amino acid sequence of SEQ ID NO: 96 or any variant thereof.

In one aspect, the present disclosure relates to an antibody or antigen-binding portion thereof that binds human CD38, comprising a heavy chain variable region of the amino acid sequence of SEQ ID NO: 101 or any variant thereof, and a light chain variable region of the amino acid sequence of SEQ ID NO: 106 or any variant thereof.

In one aspect, the present disclosure relates to an antibody or antigen-binding portion thereof that binds human CD38, comprising a heavy chain variable region of the amino acid sequence of SEQ ID NO: 111 or any variant thereof, and a light chain variable region of the amino acid sequence of SEQ ID NO: 116 or any variant thereof.

In one aspect, the present disclosure relates to an antibody or antigen-binding portion thereof that binds human CD38, comprising a heavy chain variable region of the amino acid sequence of SEQ ID NO: 121 or any variant thereof, and a light chain variable region of the amino acid sequence of SEQ ID NO: 126 or any variant thereof.

In one aspect, the present disclosure relates to an antibody or antigen-binding portion thereof that binds human CD38, comprising a heavy chain variable region of the amino acid sequence of SEQ ID NO: 131 or any variant thereof, and a light chain variable region of the amino acid sequence of SEQ ID NO: 136 or any variant thereof.

In one aspect, the present disclosure relates to an antibody or antigen-binding portion thereof that binds human CD38, comprising a heavy chain variable region of the amino acid sequence of SEQ ID NO: 141 or any variant thereof, and a light chain variable region of the amino acid sequence of SEQ ID NO: 146 or any variant thereof.

In one aspect, the present disclosure relates to an antibody or antigen-binding portion thereof that binds human CD38, comprising a heavy chain variable region of the amino acid sequence of SEQ ID NO: 151 or any variant thereof, and a light chain variable region of the amino acid sequence of SEQ ID NO: 156 or any variant thereof.

In one aspect, the present disclosure relates to an antibody or antigen-binding portion thereof that binds human CD38, comprising a heavy chain variable region of the amino acid sequence of SEQ ID NO: 161 or any variant thereof, and a light chain variable region of the amino acid sequence of SEQ ID NO: 166 or any variant thereof.

In one aspect, the present disclosure relates to an antibody or antigen-binding portion thereof that binds human CD38, comprising a heavy chain variable region of the amino acid sequence of SEQ ID NO: 171 or any variant thereof, and a light chain variable region of the amino acid sequence of SEQ ID NO: 176 or any variant thereof.

In one aspect, the present disclosure relates to an antibody or antigen-binding portion thereof that binds human CD38, comprising a heavy chain variable region of the amino acid sequence of SEQ ID NO: 181 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 181 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 181 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 181 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 181 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 186 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 186 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 186 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 186 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 186 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 191 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 191 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 191 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 191 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 191 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 196 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 196 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 196 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 196 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 196 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 201 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 201 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 201 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 201 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 201 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 206 or any variant thereof, and a light chain variable region of the amino acid sequence of SEQ ID NO: 236 or any variant thereof.

In one aspect, the present disclosure relates to an antibody or antigen-binding portion thereof that binds human CD38, comprising a heavy chain variable region of the amino acid sequence of SEQ ID NO: 206 or any variant thereof, and a light chain variable region of the amino acid sequence of SEQ ID NO: 241 or any variant thereof.

In one aspect, the present disclosure relates to an antibody or antigen-binding portion thereof that binds human CD38, comprising a heavy chain variable region of the amino acid sequence of SEQ ID NO: 206 or any variant thereof, and a light chain variable region of the amino acid sequence of SEQ ID NO: 246 or any variant thereof.

In one aspect, the present disclosure relates to an antibody or antigen-binding portion thereof that binds human CD38, comprising a heavy chain variable region of the amino acid sequence of SEQ ID NO: 206 or any variant thereof, and a light chain variable region of the amino acid sequence of SEQ ID NO: 251 or any variant thereof.

In one aspect, the present disclosure relates to an antibody or antigen-binding portion thereof that binds human CD38, comprising a heavy chain variable region of the amino acid sequence of SEQ ID NO: 206 or any variant thereof, and a light chain variable region of the amino acid sequence of SEQ ID NO: 256 or any variant thereof.

In one aspect, the present disclosure relates to an antibody or antigen-binding portion thereof that binds human CD38, comprising a heavy chain variable region of the amino acid sequence of SEQ ID NO: 211 or any variant thereof, and a light chain variable region of the amino acid sequence of SEQ ID NO: 261 or any variant thereof.

In one aspect, the present disclosure relates to an antibody or antigen-binding portion thereof that binds human CD38, comprising a heavy chain variable region of the amino acid sequence of SEQ ID NO: 211 or any variant thereof, and a light chain variable region of the amino acid sequence of SEQ ID NO: 266 or any variant thereof.

In one aspect, the present disclosure relates to an antibody or antigen-binding portion thereof that binds human CD38, comprising a heavy chain variable region of the amino acid sequence of SEQ ID NO: 216 or any variant thereof, and a light chain variable region of the amino acid sequence of SEQ ID NO: 261 or any variant thereof.

In one aspect, the present disclosure relates to an antibody or antigen-binding portion thereof that binds human CD38, comprising a heavy chain variable region of the amino acid sequence of SEQ ID NO: 216 or any variant thereof, and a light chain variable region of the amino acid sequence of SEQ ID NO: 266 or any variant thereof.

In one aspect, the present disclosure relates to an antibody or antigen-binding portion thereof that binds human CD38, comprising a heavy chain variable region of the amino acid sequence of SEQ ID NO: 221 or any variant thereof, and a light chain variable region of the amino acid sequence of SEQ ID NO: 261 or any variant thereof.

In one aspect, the present disclosure relates to an antibody or antigen-binding portion thereof that binds human CD38, comprising a heavy chain variable region of the amino acid sequence of SEQ ID NO: 221 or any variant thereof, and a light chain variable region of the amino acid sequence of SEQ ID NO: 266 or any variant thereof.

In one aspect, the present disclosure relates to an antibody or antigen-binding portion thereof that binds human CD38, comprising a heavy chain variable region of the amino acid sequence of SEQ ID NO: 226 or any variant thereof, and a light chain variable region of the amino acid sequence of SEQ ID NO: 261 or any variant thereof.

In one aspect, the present disclosure relates to an antibody or antigen-binding portion thereof that binds human CD38, comprising a heavy chain variable region of the amino acid sequence of SEQ ID NO: 226 or any variant thereof, and a light chain variable region of the amino acid sequence of SEQ ID NO: 266 or any variant thereof.

In one aspect, the present disclosure relates to an antibody or antigen-binding portion thereof that binds human CD38, comprising a heavy chain variable region of the amino acid sequence of SEQ ID NO: 231 or any variant thereof, and a light chain variable region of the amino acid sequence of SEQ ID NO: 261 or any variant thereof.

In one aspect, the present disclosure relates to an antibody or antigen-binding portion thereof that binds human CD38, comprising a heavy chain variable region of the amino acid sequence of SEQ ID NO: 231 or any variant thereof, and a light chain variable region of the amino acid sequence of SEQ ID NO: 266 or any variant thereof.

In one aspect, the present disclosure provides a bispecific or multispecific molecule comprising an antibody or antigen-binding portion thereof of any of the preceding aspects.

A nucleic acid molecule encodes the antibody or antigen-binding portion thereof or bispecific or multispecific molecule according to any of the preceding aspects. Preferably, the nucleic acid molecule comprises the nucleotide sequence of the antibody heavy chain selected from SEQ ID NOs: 5, 15, 25, 35, 45, 55, 65, 75, 85, 95, 105, 115, 125, 135, 145, 155, 165, 175, 185, 190, 195, 200, 205, 210, 215, 220, 225, 230, 235, or any variant thereof, and/or the nucleotide sequence of the antibody light chain selected from SEQ ID NOs: 10, 20, 30, 40, 50, 60, 70, 80, 90, 100, 110, 120, 130, 140, 150, 160, 170, 180, 240, 245, 250, 255, 260, 265, 270, or any variant thereof.

An antibody or antigen-binding portion thereof that binds to human CD38 has at least greater than 60%, 65%, 70%, 75%, 80%, 85%, 90%, 95%, 96%, 97%, 98%, 99%, or more sequence identity to the antibody or antigen-binding portion thereof of any of the preceding aspects.

A nucleic acid molecule encodes the antibody or antigen-binding portion thereof according to any one of the preceding aspects, or a nucleic acid molecule has at least greater than 60%, 65%, 70%, 75%, 80%, 85%, 90%, 95%, 96%, 97%, 98%, 99%, or more sequence identity thereto.

A vector comprises the nucleic acid according to any one of the preceding aspects.

A cell comprises the vector according to any one of the preceding aspects.

A pharmaceutical composition comprises the antibody or antigen-binding portion thereof or nucleic acid encoding the same according to any one of the preceding aspects and a pharmaceutically acceptable carrier.

The antibodies of the present invention are useful as therapeutic or diagnostic tools in a variety of diseases in which CD38 is undesirably expressed or found. Diseases and conditions that are particularly suitable for the treatment with the CD38 antibodies of the present invention are tumors or autoimmune diseases, preferably said tumors are selected from the group consisting of multiple myeloma (MM) and chronic lymphocytic leukemia (CLL), chronic myelogenous leukemia (CML), T-cell acute lymphocytic leukemia, acute myelogenous leukemia (AML), and acute lymphocytic leukemia (ALL). Preferably, the autoimmune disease is selected from rheumatoid arthritis (RA) or systemic lupus erythematosus (SLE).

The method of treating diseases and conditions with the CD38 antibodies of the present invention includes the steps of administering to the mammal a therapeutically effective amount of an antibody or antigen-binding fragment thereof or a nucleic acid molecule or a vector or a cell or a pharmaceutical composition of any of the preceding aspects.

Use of the antibody or antigen-binding fragment thereof or the nucleic acid molecule or the vector or the cell or the pharmaceutical composition according to any of the preceding aspects in the manufacture of a medicament for the treatment of a CD38-related disorder in a mammal. According to any one of the preceding aspects, optionally, the antibody is conjugated to other drugs, such as a labeled or cytotoxic conjugate.

In one aspect, the present disclosure also includes kits, e.g., comprising the antibodies, the fragments, the homologues, derivatives thereof, etc., of the disclosure, e.g., the labeled or cytotoxic conjugate, as well as the instructions for use of the antibody, the conjugate that kills a particular type of cell, and the like. The instructions can include directions for using the antibody, conjugate, etc. in vitro, in vivo, or ex vivo. The antibodies may be in liquid form or in solid form, usually lyophilized. The kit may contain other suitable reagents, such as buffers, reconstitution solutions and other necessary components for the intended use. Combinations of reagents packaged in predetermined amounts with instructions for their use, e. g. for therapeutic use or for conducting diagnostic assays, are contemplated. When the antibody is labeled, e. g. with an enzyme, then the kit can include a substrate and cofactors required for the enzyme (e. g. a substrate precursor providing a detectable chromophore or fluorophore). In addition, other additives such as stabilizers, buffers (e. g. blocking buffers or lysis buffers), and the like may also be included. The relative amounts of the various reagents can be varied to provide a concentrate of reagent solution, which provides user flexibility, and savings space and reagent. These reagents may also be provided in dry powder form, usually in lyophilized form, including excipients which, when dissolved, provide a reagent solution having the appropriate concentration.

Use of the antibody or functional fragment thereof or the nucleic acid molecule or the vector or the cell or the pharmaceutical composition or the kit according to any of the preceding aspects in the manufacture of a reagent for inhibiting the binding of CD38 to CD38R.

In addition, the antibodies of the present invention may be used in immunoassays, purification methods, and other methods using immunoglobulins or fragments thereof. Such uses are well known in the art.

Accordingly, the present invention also provides compositions comprising anti-CD38 antibody of the present invention, or fragment thereof, conveniently in combination with a pharmaceutically acceptable carrier, diluent, or excipient, as is conventional in the art.

As used herein, the term "pharmaceutical composition" refers to formulations of various preparations. Formulations containing therapeutically effective amounts of multivalent antibodies are in sterile liquid solution, liquid suspension, or lyophilized form, optionally containing stabilizers or excipients.

The antibodies of the present invention may be used as compositions for administration alone, or can be used in combination with other active agents.

It should be appreciated that therapeutic agents according to the described embodiments will be administered with suitable pharmaceutically acceptable carriers, excipients, and other agents incorporated into formulations to provide improved transfer, delivery, tolerability, and the like. A large number of suitable formulations can be found in all pharmacopoeias known to pharmaceutical chemists: Remington's Pharmaceutical Sciences (15th ed. Mack Publishing Company, Easton, Pa. (1975)), particularly Chapter 87: Blaug, Seymour. These formulations include, for example, powders, pastes, ointments, gels, waxes, oils, lipids, lipid-containing (cationic or anionic) carriers (e. g., Lipofectin™), DNA conjugates, anhydrous syrups, oil-in-water and water-in-oil emulsions, emulsion polyethylene glycols (polyethylene glycols of various molecular weights), semi-solid gels, and semi-solid mixtures containing polyethylene glycol. Any of the foregoing mixtures may be suitable for the treatment or therapy according to the present invention, provided that the active ingredient in the formulation is not inactivated by the formulation and that the formulation is physiologically compatible and tolerates the route of administration.

In one embodiment, the antibody can be used as a therapeutic agent. Such agents will generally be used to treat, alleviate, and/or prevent a disease or pathology associated with aberrant expression, activity, and/or signaling of CD38 in a subject. The therapeutic regimens may be implemented using standard methods by identifying a subject, e. g., a human patient, having (or at risk of or developing) a disease or disorder associated with aberrant expression, activity, and/or signaling of CD38, e. g., a CD38-related disorder. An antibody preparation, preferably one with high specificity and affinity for its target antigen, is administered to a subject, which will generally have an effect due to its binding to the target. The administered antibody can eliminate or inhibit or interfere with expression, activity, and/or signaling function of the target (e. g., CD38). The administered antibody can eliminate or inhibit or interfere with the binding of the target (e. g., CD38) to the endogenous ligand to which it naturally binds. For example, an antibody binds to a target and modulates, blocks, inhibits, reduces, antagonizes, neutralizes, and/or otherwise interferes with the expression, activity, and/or signaling of CD38. In some embodiments, to treat a disease or disorder associated with aberrant expression of CD38, an antibody having heavy and light chain CDR can be administered to a subject. In another embodiment, antibodies against CD38 can be used in methods known in the art relating to localization and/or quantitation of CD38 (e. g., for determining CD38 and/or levels of CD38 in an appropriate physiological sample, for diagnostic methods, for protein imaging, etc.). In a given embodiment, an antibody comprising an antibody-derived antigen-binding domain specific for CD38 or a derivative, fragment, analog or homolog thereof is used as a pharmaceutically active compound (hereinafter "therapeutic agent").

In another embodiment, CD38 polypeptides can be isolated using antibodies specific for CD38 by standard techniques such as immunoaffinity, chromatography, or immunoprecipitation. The protein in a biological sample can be detected with antibodies (or fragments thereof) against the CD38 protein. In some embodiments, CD38 can be detected in a biological sample as part of a clinical testing procedure, for example, to determine the efficacy of a given therapeutic regimen. Detection may be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazine aminofluorescein, dansyl chloride, or phycoerythrin; one example of a luminescent material includes luminol; examples of bioluminescent materials include luciferase, luciferin and aequorin, and examples of suitable radioactive materials include $^{125}I$, $^{131}I$, $^{35}S$, or $^{3}H$.

In another embodiment, antibodies according to the present disclosure can be used as reagents for detecting the presence of CD38 or a protein fragment thereof in a sample. In some embodiments, the antibody comprises a detectable label. The antibody is a polyclonal antibody, or more preferably a monoclonal antibody. Intact antibodies or fragments thereof (e. g., Fab, scFv or $F(ab')_2$) are used. The term "labeled" with respect to an antibody is intended to encompass direct labeling of the antibody by coupling (i. e., physically linking) a detectable substance to the antibody, as well as indirect labeling of the antibody by reaction with another reagent that is directly labeled. Examples of indirect labeling include detection of a primary antibody using a fluorescently labeled second antibody, and end-labeling of the antibody with biotin to enable detection with fluorescently labeled streptavidin. The term "biological sample" is intended to include tissues, cells, and biological fluids isolated from a subject, as well as tissues, cells, and fluids present in a subject. Thus, the term "biological sample" as used includes blood and fractions or components in blood, including serum, plasma, or lymph. In other words, the detection method of the described embodiments can be used to detect the analyte mRNA, protein, or genomic DNA in a biological sample both in vitro and in vivo. For example, in vitro techniques for detection of mRNA analytes include Norhtern hybridization and in situ hybridization. In vitro techniques for detection of protein analytes include enzyme-linked immunosorbent assays (ELISA), Western blot, immunoprecipitations, and immunofluorescence. In vitro techniques for detection of genomic DNA analytes include Southern hybridization. Procedures for performing immunoassays are described, for example, in "ELISA: Theory and Practice: Methods in Molecular Biology", vol. 42, J. R. Crowther (ed.) Human Press, Totowa, N. J. 1995; "Immunoassay", E. Diamandis and T. Christopoulus, Academic Press, Inc., San Diego, Calif., 1996; and "Practice and Theory of Enzyme Immunoassays", P. Tijssen, Elsevier Science Publishers, Amsterdam, 1985. In addition, in vivo techniques for detection of protein analytes include introducing into a subject a labeled anti-analyte protein antibody. For example, an antibody can be labeled with a radiolabel, and the presence and location of the radiolabel in the subject can then be detected by standard imaging techniques.

The antibodies described herein and derivatives, fragments, analogs, and homologs thereof can be incorporated into pharmaceutical compositions suitable for administration. The principles and considerations involved in preparing such compositions, as well as guidance in selecting components, are well known in the art, for example, see Remington's Pharmaceutical Sciences: The Science and Practice of Pharmacy, 19th edition (Alfonso R. Gennaro et al. Eds.) Mack Pub. Co. Easton, Pa: 1995; Drug Absorption Enhancement: Concepts, Possibilities, Limitations, and Trends, Harwood Academic Publishers, Langhorne, Pa. 1994; and Peptide and Protein Drug Delivery (Advances In Parenteral Sciences, vol. 4), 1991, M. Dekker, New York.

Such compositions typically comprise the antibody and a pharmaceutically acceptable carrier. When antibody fragments are used, minimal inhibitory fragments that specifically bind to the target protein binding domain may be preferred. For example, based on the variable region sequence of an antibody, peptide molecules can be designed that retain the ability to bind to a target protein sequence. Such peptides can be chemically synthesized and/or produced by recombinant DNA techniques (see, e. g., Marasco et al. Proc. Natl. Acad. Sci. USA, 90:7889-7893 (1993)).

As used herein, the term "pharmaceutically acceptable carrier" is intended to include any and all solvents, dispersion media, coatings, antibacterial and antifungal agents, isotonic and absorption delaying agents, etc., compatible with pharmaceutical administration. Suitable pharmaceutically acceptable carriers are described in the latest edition of Remington's Pharmaceutical Sciences, a standard bibliography in the art, which is incorporated herein by reference. Preferred examples of such carriers or diluents include, but not limited to, water, saline, Ringer's solution, dextrose solution, and 5% human serum albumin. Liposomes and non-aqueous vehicles such as fixed oils can also be used. The use of such media and agents for pharmaceutically active substances is well known in the art. Except insofar as any conventional media or agent is incompatible with the antibody, its use in the compositions is contemplated.

The pharmaceutical compositions of the embodiments are formulated to be compatible with their intended route of administration. Examples of routes of administration include parenteral, e. g., intravenous, intradermal, subcutaneous, oral (e. g., inhalation), transdermal (i. e., topical), transmucosal, and rectal administration. Solutions or suspensions for parenteral, intradermal or subcutaneous administration may include the following components: sterile diluents for injection such as water, saline solutions, fixed oils, polyethylene glycols, glycerol, propylene glycol or other synthetic solvents; antibacterial agents such as benzyl alcohol or methyl p-hydroxybenzoate; antioxidants such as ascorbic acid or sodium bisulfite; chelating agents such as EDTA; buffers, such as acetates, citrates, or phosphates, and reagents to adjust the osmotic pressure, such as sodium chloride or dextrose. The pH can be adjusted with acids or bases, such as hydrochloric acid or sodium hydroxide. The parenteral preparation can be packaged in ampoules, disposable syringes, or multiple dose vials made of glass or plastic.

Pharmaceutical compositions suitable for injectable use include sterile aqueous solutions (water soluble) or dispersions and sterile powders for the extemporaneous preparation of sterile injectable solutions or dispersion. For intravenous administration, suitable pharmaceutically acceptable carriers include physiological saline, bacteriostatic water, Cremophor EL™ (BASF, Parsippany, N. J.) or phosphate buffered saline (PBS). In all cases, the composition must be sterile and should be fluid to the extent that easy syringability exists. It must be stable under the conditions of manufacture and storage and must be preserved against the contaminating from microorganisms such as bacteria and fungi. The carrier can be a solvent or dispersion medium containing, for example, water, ethanol, polyol (for example, glycerol, propylene glycol, and liquid polyethylene glycol, etc.), and suitable mixtures thereof. For example, the desired particle size can be maintained in the case of dispersions by the use of coatings such as lecithin, and proper fluidity can be maintained by the use of surfactants. Prevention of the action of microorganisms can be achieved by various antibacterial and antifungal agents, for example, parabens, chlorobutanol, phenol, ascorbic acid, thimerosal, etc. In many cases, it will be preferable to include isotonic agents, for example, sugars, polyalcohols such as mannitol, sorbitol, and sodium chloride in the composition. The absorption of the injectable compositions can be prolonged by encompassing in the composition an agent which delays absorption, for example, aluminum monostearate and gelatin.

Sterile injectable solutions can be prepared by incorporating the antibody in the required amount in an appropriate solvent with one or a combination of ingredients enumerated above, as required, followed by filtered sterilization. Generally, dispersions are prepared by incorporating the antibody into a sterile vehicle that contains a basic dispersion medium and the required other ingredients from those enumerated above. The resulting powders are vacuum-dried and freeze-dried to prepare sterile powder for injection containing the active ingredient and any additional desired ingredient from a sterile-filtered solution of those ingredients previously described.

For administration by inhalation, the compounds are delivered in the form of an aerosol spray from a pressurized container or dispenser or a nebulizer containing a suitable propellant, such as carbon dioxide and other gas.

Systemic administration can also be performed by transmucosal or transdermal means. For transmucosal or transdermal administration, penetrants appropriate to permeate the barrier are used in the formulation. Such penetrants are generally known in the art, and include, for example, detergents, bile salts, and fusidic acid derivatives for transmucosal administration. Transmucosal administration can be accomplished through the use of nasal sprays or suppositories. For transdermal administration, one or more of the antibodies can be formulated into ointments, salves, gels, or creams as generally known in the art.

The compounds may also be prepared in the form of suppositories (e. g., with conventional suppository bases such as cocoa butter or other glycerides) or retention enemas for rectal delivery. In one embodiment, the antibodies can be prepared with carriers that prevent their rapid elimination from the body, such as sustained/controlled release formulations, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparing such formulations will be apparent to those skilled in the art.

It is especially advantageous to formulate parenteral compositions in dosage unit form for ease of administration and uniformity of dosage. Dosage unit form, as used herein, refers to physically discrete units suited as unitary dosages for the subject to be treated; each unit contains a predetermined quantity of one or more of the antibodies calculated to produce the desired therapeutic effect in association with the required pharmaceutical carrier. The specification for the dosage unit forms of the described embodiments are dictated by and directly dependent on: the unique characteristics of antibodies and the specific therapeutic effects to be achieved, and the limitations inherent in the art of formulating such antibodies for treating individuals.

The pharmaceutical compositions can be placed in a container, pack, or dispenser together with instructions for administration.

The formulations described herein may also contain more than one of the antibodies, preferably those with complementary activities but without adversely affecting each other, depending on the particular situation to be treated. Alternatively or additionally, the composition may, for example, comprise an agent that enhances the function thereof, such as a cytotoxic agent, a cytokine, a chemotherapeutic agent, or a growth inhibitor. Such molecules are suitably present in combination in amounts effective for the intended purpose. For example, they may be present in combination in a kit or in combination for use.

In one embodiment, one or more of the antibodies can be administered in combination therapy, i. e., in combination with other agents, such as therapeutic agents, which can be used to treat pathological conditions or disorders, such as various forms of cancer, autoimmune disorders, and inflammatory diseases. The term "in combination" means herein that the agents are administered substantially synchronously, simultaneously or sequentially. If administered sequentially, the first of the two compounds is still preferably detected at an effective concentration at the treatment site when the second compound is initially administered. In one aspect, a "combination" can also include both an antibody of the present invention and another therapeutic agent in a kit.

For example, combination therapy can comprise coformulation and/or coadministration of one or more antibodies described herein with one or more additional therapeutic agents as described in more detail below, for example, one or more cytokine and growth factor inhibitors, immunosuppressants, anti-inflammatory agents, metabolic inhibitors, enzyme inhibitors, and/or cytotoxins or cytostatic agents. Such combination therapy may advantageously utilize lower dosages of the administered therapeutic agents, thus avoiding possible toxicities or complications associated with each monotherapy.

For purposes of clarity and conciseness, features are described herein as part of the same or separate embodiments, however, it will be understood that the scope of the present invention may include some embodiments having a combination of all or some of the features described.

## Example

### Example 1: Preparation of CD38 recombinant proteins

Human CD38 (UniProt KB: P28907) extracellular region (Val 43-Ile 300) nucleotide sequences were synthesized, to which IgG kappa signal peptide and 6 × his tag were fused at N-terminal, the fused sequences were digested and cloned into eukaryotic expression plasmid CMV downstream of promoter to obtain a pSect-Nhis-CD38; HEK293.6E cells were transfected with the plasmid and cultured for 7 days, the supernatant of culture medium was collected and purified by nickel chelate column to obtain recombinant human CD38 extracellular region protein. See FIG. 1 for SDS-PAGE.

### **Example 2. Construction of human CD38/CHO stably transfected** cell line

A lentiviral vector plasmid containing a full-length sequence of human CD38 was constructed, and the constructed lentiviral plasmid and packaging plasmid were co-transfected into HEK 293T cells for lentiviral packaging, according to the lentiviral packaging kit instructions (Lenti-Pac HIV Expression Packaging Kit, Gene Copoeia, Cat: HPK-LvTR-20). Culture medium was collected in 48 hours after transfection, and centrifuged at 500 * g for 10 minutes to remove cell debris to obtain culture supernatant containing lentivirus particles, which was filtered through 0.45 μm PES membrane and dispensed into 1.5 mL EP tubes; $1\times10^6$ CHO cells were infected with 10 μL filtrate, and 10 μg/mL puromycin was added to the culture medium for screening positive clones, which was limiting dilution to obtain stably transfected cells CHO-CD38-1H7 expressing human full-length CD38, with the results shown in FIG. 2.

### Example 3 Preparation of the polyclonal antibody against human CD38

1) Immunization of mice

Human recombinant CD38 protein or CHO-CD38-1H7 cells were used as an antigen and mixed with an equal amount of immune adjuvant (Freund's adjuvant), and each group of 6 female Balb/c mice of 6 weeks were taken for subcutaneous immunization. A booster immunization was performed two weeks after the primary immunization. After three-time immunization, orbital bleeds were taken to detect serum titers.

2) Cell fusion and hybridoma screening

Before fusion, $1\times10^6$ CHO-CD38-1H7 transfected cells were injected into the tail vein challenging.

Three days later, the mice were sacrificed by neck dissection. The mouse spleen and some peripheral lymph nodes were taken, centrifuged after milling in DMEM medium; after the supernatant was poured, the spleen cell mass was gently dispersed, and 5ml of red blood cell lysis buffer was added. 40ml DMEM was added after lysis for 50s to centrifuge, a spleen cell suspension without red blood cells was obtained. After mixing an appropriate amount of lymph node and the spleen cell suspension with SP2/0, the BTX electrofusion instrument was used for cell fusion. The fusion cells were seeded in a 96-well plate, and cultured in a complete DMEM medium containing HAT under 5% $CO_2$ condition at 37°C. The growth of hybridoma cells was observed in about a week, and the supernatant was taken out for detection when the hybridoma cells grew to more than 60%.

### Example 4. Screening of monoclonal antibodies against human CD38 and acquisition of antibody sequences

1) ELISA screening

Human CD38 recombinant protein was coated onto a 96-well microtiter plate at 1 μg/mL and incubated overnight at 4°C. The plate was washed for 3 times with PBS next day, blocked with 200 μL 2% skimmed milk powder/PBS for 2 hours at room temperature, and washed once with PBS, followed by the addition of 50 μL hybridoma supernatant, the mixture was incubated for 1 hour at room temperature, and repeatedly washed for 3 times with PBST and PBS, followed by the addition of 100 μL/well HRP labeled anti-mouse IgG Fc secondary antibody, the mixture was incubated for 60 minutes at room temperature. To the mixture washed for 3 times with PBST and PBS respectively was added 100 μL of chromogenic reagent (TMB solution, Sigma Cat: T2885), followed by standing at 37°C for 5 minutes, the reaction was terminated by adding 50 μL of 2M concentrated sulfuric acid solution, and OD450 value was read immediately with a microplate reader.

2) Cell screening anti-CD38 specific antibodies (FACS)

Supernatants of positive clones that bound to CD38 antibodies detected by ELISA were further verified for binding to CD38 positive cells. CHO cells and CHO-CD38 stably transfected cells were taken out and added into a 96-well plate at $5\times10^4$ cells/well, respectively, 50 μL hybridoma supernatant was added per well, the mixture was incubated at 4°C for 60 minutes, then centrifuged to discard the supernatant by pipetting, sediment was washed with 0.5% BSA/PBS, 50 μL secondary antibody solution (anti-mouse IgG-Fc-AF647, Jackson ImmunoResearch, Cat: 115-606-071) was added, the mixture was incubated at 4°C for 45 minutes, then washed off with 0.5% BSA/PBS to remove excess secondary antibody, the cells were resuspended by adding PBS, and detected by Flow cytometry.

3) Gene cloning and expression of chimeric anti-human CD38 antibody

Positive monoclonal hybridoma against CD38 was screened, total cell RNA was extracted by a TRNzol lysis method, then single-chain cDNA was synthesized using reverse transcription kit (Invitrogen, Cat. No. 18080051), and used as a template to amplify the variable region sequence of antibody in monoclonal hybridoma cells; after sequencing, the obtained candidate positive clone heavy chain and light chain variable region sequences were as follows respectively:

Clones: 21H12-G12 SEQ ID NOs:1-10
Heavy chain VH

<-------------FR1-----------> CDR1 <----FR2-----> CDR2 <--

QIQLVQSGPELKKPGETVKISCKASGYTF**TNHGMN**WVKQAPGKGLKWMG**WINTYTGE**

**PTYGEDFKG**RFA

------------FR3-------------> CDR3<---FR4--->

FSLETSASTAYLQINNLKNEDLATYFCAR**KGFVY**WGQGTLVTVSV

Nucleotide sequence

CAGATCCAGTTGGTGCAGTCTGGACCTGAGCTGAAGAAGCCTGGAGAGACAGTC

AAGATCTCCTGCAAGGCTTCTGGGTATACTTTCACAAACCATGGAATGAACTGGG

TGAAGCAGGCTCCAGGAAAGGGCTTAAAGTGGATGGGCTGGATAAACACCTACA

CTGGAGAGCCAACATATGGTGAAGACTTCAAGGGACGGTTTGCCTTCTCTTTGGA

AACCTCTGCCAGCACTGCCTATTTGCAGATCAACAACCTCAAAAATGAGGACCTG

GCTACATATTTCTGTGCAAGAAAGGGGTTTGTTTACTGGGGCCAAGGGACTCTGG

TCACTGTCTCTGTA

Light chain VK

<----------FR1--------> CDR1 <-----FR2-----> CDR2 <--------

DIVLTQSPASLAVSLGQRATMSC**RASESVDIYGNSFIY**WYQQKPGQPPKLLIY**RASILES**G IPARFSGS

-----------FR3--------> CDR3 <---FR4--->

GSRTDFTLTINPVEADDVATYYC**QQINEDPFT**FGGGTKLEIK

Nucleotide sequence

GACATTGTGCTGACCCAATCTCCAGCTTCTTTGGCTGTGTCTCTAGGGCAGAGGGC
CACCATGTCCTGCAGAGCCAGTGAAAGTGTTGATATTTATGGCAATAGTTTTATAT
ACTGGTACCAACAGAAACCAGGACAGCCACCCAAACTCCTCATCTATCGTGCATC
CATCCTAGAATCTGGGATCCCTGCCAGGTTCAGTGGCAGTGGGTCTAGGACAGAC
TTCACCCTCACCATTAATCCTGTGGAGGCTGATGATGTTGCAACCTATTACTGTCA
GCAAATTAATGAAGATCCATTCACGTTCGGAGGGGGGACCAAGCTGGAAATAAA
A

Clones: 7C10-G5 SEQ ID NOs:11-20
Heavy chain VH

<-------------FR1-----------> CDR1 <----FR2-----> CDR2 <--

QAYLQQSGAELVRSGASVKMSCKASGYTF**TSYNIH**WVKQTPGQGLKWIG**YIYPGNGGT NYNQKFKV**KAT

------------FR3-------------> CDR3 <---FR4--->

LTADTSSSTAYMQISSLTSEDSAVYFCAR**RGSDYDGGFAS**WGQGTLVTVSA

Nucleotide sequence

CAGGCTTATCTACAGCAGTCTGGGGCTGAGCTGGTGAGGTCTGGGGCCTCAGTGA
AGATGTCCTGCAAGGCTTCTGGCTACACATTTACCAGTTACAATATACACTGGGT
AAAGCAGACACCTGGACAGGGCCTGAAATGGATTGGATATATCTATCCTGGAAAT
GGTGGTACTAACTACAATCAGAAGTTCAAGGTCAAGGCCACATTGACTGCAGACA
CATCCTCCAGCACAGCCTACATGCAGATCAGCAGCCTGACATCTGAAGACTCTGC
GGTCTATTTCTGTGCAAGAAGGGGGTCTGATTACGACGGGGGGTTTGCTTCCTGG
GGCCAAGGGACTCTGGTCACTGTCTCTGCA

Light chain VK

<----------FR1--------> CDR1 <-----FR2-----> CDR2 <--------

DILLTQSPASLAVSLGQRATISC**RASKSVDIYGNSFIH**WYQQKPGQSPKLLIY**LASNLDS**G VPARFSGS

-----------FR3--------> CDR3 <---FR4--->

GSRTDFTLTIDPVEADDAATYYC**QQNNEDPLT**FGAGTKLELK

Nucleotide sequence

GACATTTTGCTGACCCAGTCTCCAGCTTCTTTGGCTGTGTCTCTAGGGCAGAGGGC CACCATATCCTGCAGAGCCAGTAAAAGTGTTGATATTTATGGCAATAGTTTTATA CACTGGTACCAGCAGAAACCAGGACAGTCACCCAAACTCCTCATCTATCTTGCAT CCAACCTAGATTCTGGGGTCCCTGCCAGGTTCAGTGGCAGTGGGTCTAGGACAGA CTTCACCCTCACCATTGATCCTGTGGAGGCTGATGATGCTGCAACCTATTACTGTC AGCAAAATAATGAGGATCCGCTCACGTTCGGTGCTGGGACCAAGCTGGAGCTGA AA

Clones: 15A1-G1 SEQ ID NOs:21-30

Heavy chain VH

<-------------FR1-----------> CDR1 <----FR2-----> CDR2 <---

QLQLKQSGPGLVQPSQSLSITCTVSGFSL**TSFGVH**WIRQSPGKGLEWLG**VIWRGGSTDYN AAFMS**RLSI

------------FR3------------> CDR3 <---FR4--->

TKDNSKSQVFFKMNSLQADDTAIYYCAK**TMITRGYTMDY**WGQGTSVTVSS

Nucleotide sequence

CAGTTGCAGCTGAAGCAGTCAGGACCTGGCCTAGTGCAGCCCTCACAGAGCCTGT
CCATAACCTGCACAGTCTCTGGTTTCTCATTAACTAGCTTTGGTGTACACTGGATT
CGCCAGTCTCCAGGAAAGGGTCTGGAGTGGCTGGGAGTGATTTGGAGAGGTGGA
AGCACAGACTACAATGCAGCTTTCATGTCCAGACTGAGCATCACCAAGGACAACT
CCAAGAGCCAAGTTTTCTTTAAAATGAACAGTCTGCAAGCTGATGACACTGCCAT
ATATTACTGTGCCAAAACTATGATTACGAGGGGGTATACTATGGACTACTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQKPGNAPRLLIS**GATTLET**GVPSR
FSGSGSGK

--------FR3-------> CDR3 <---FR4--->

DYTLSITSLQTEDVATYYC**QQFWSTPYT**FGGGTKLEMI

Nucleotide sequence

GACATCCAGATGACACAATCCTCATCCTCCTTTTCTGTATCTCTAGGAGACAGGGT

CACCATTACTTGCAAGGCAAGTGAGGACATATATAATCGGTTAGCCTGGTATCAG
CAGAAACCAGGAAATGCTCCTAGGCTCTTAATATCTGGTGCAACCACTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTACTGTCAACAGTTTTGGA
GTACTCCGTACACGTTCGGAGGGGGGACCAAGCTGGAAATGATA

Clones: 51G7-D3 SEQ ID NOs:31-40
Heavy chain VH

<-------------FR1-----------> CDR1 <----FR2-----> CDR2 <--

EVQLVESGGGLVKPGGSLKLSCEGSGFTF**NNYAMS**WVRQSPEKRLEWVA**EISTGGTFTY
YVDTVTG**RFT

------------FR3-------------> CDR3 <---FR4--->

ISRDNAKNTLYLEMSSLRSEDTARYYCAR**GYRYDAADY**WGQGISVTVSS

Nucleotide sequence

GAAGTGCAGCTGGTGGAGTCTGGGGGAGGCTTAGTGAAGCCTGGAGGGTCCCTG
AAACTCTCCTGTGAAGGCTCTGGATTCACTTTCAACAACTATGCCATGTCTTGGGT
TCGCCAGTCTCCAGAGAAGAGGCTGGAGTGGGTCGCAGAGATTTCTACTGGTGGT
ACTTTCACCTACTATGTAGACACTGTGACGGGCCGATTCACCATCTCCAGAGACA
ATGCCAAGAACACCCTGTACCTGGAAATGAGTAGTCTGAGGTCTGAGGACACGG
CCAGGTATTATTGTGCAAGAGGCTATAGGTACGACGCGGCGGACTACTGGGGTCA
AGGAATCTCAGTCACCGTCTCCTCA

Light chain VK

<----------FR1--------> CDR1 <-----FR2-----> CDR2 <------------

DIQMTQSPSSLSASLGERVSLTC**RASQEISGYLI**WLQQKPDGTIKRLIY**ATSTLDS**GVPKRF
SGSRSGS

--------FR3-------> CDR3 <---FR4--->

DYTLTISSLESEDFADYYC**LQYANYFT**FGAGTKLELK

Nucleotide sequence

GACATCCAGATGACCCAGTCCCCCTCCTCCCTCTCCGCCTCCCTCGGCGAGCGCGT
CTCCCTCACCTGCCGCGCCTCCCAGGAGATCTCCGGCTACCTCATCTGGCTCCAGC
AGAAGCCCGACGGCACCATCAAGCGCCTCATCTACGCCACCTCCACCCTCGACTC
CGGCGTCCCCAAGCGCTTCTCCGGCTCCCGCTCCGGCTCCGACTACACCCTCACCA
TCTCCTCCCTCGAGTCCGAGGACTTCGCCGACTACTACTGCCTCCAGTACGCCAAC
TACTTCACCTTCGGCGCCGGCACCAAGCTCGAGCTCAAG

Clones: 17A4-H9 SEQ ID NOs:41-50
Heavy chain VH

<-------------FR1-----------> CDR1 <----FR2-----> CDR2 <---

QVQLNQSGPGLVQPSQSLSITCTVSGFS**LTSYGVH**WLRQSAGKGLEWLGVIWRGGSTDY
NVGFMSRLTI

------------FR3------------> CDR3 <---FR4--->

SKDNSKSQVFFKMNSLQGDDTAIYFCAK**SMITTGYTMDY**WGQGTSVTVSS

Nucleotide sequence

CAGGTGCAGCTGAACCAGTCAGGACCTGGCCTAGTGCAGCCCTCACAGAGCCTGT
CCATAACCTGCACAGTCTCTGGTTTCTCATTAACTAGCTATGGTGTACATTGGCTT
CGCCAGTCTGCAGGAAAGGGTCTGGAGTGGCTGGGAGTGATATGGAGAGGTGGA
AGCACAGACTACAATGTAGGTTTCATGTCCAGACTGACCATTAGTAAGGACAATT
CCAAGAGCCAAGTTTTCTTTAAAATGAACAGTCTGCAAGGTGATGACACTGCCAT
ATACTTCTGTGCCAAAAGTATGATTACGACGGGCTATACTATGGACTACTGGGGT
CAAGGAACCTCAGTCACCGTCTCCTCA

Light chain VK

<----------FR1--------> CDR1 <-----FR2-----> CDR2 <------------

AIQMTQSSSSFSVSLGDRVTITC**KASEDIYNRLA**WYQQKSGNAPRLLIS**GSTSLET**GVPSR
FSGSGSGK

--------FR3-------> CDR3 <---FR4--->

DYTLSIISLQTEDVATYYC**QQFWSTPWT**FGGGTKLEIK

Nucleotide sequence

GCCATCCAGATGACACAATCTTCATCCTCCTTTTCTGTATCTCTAGGAGACAGAGT
CACCATTACTTGCAAGGCAAGTGAGGACATATATAATCGGTTAGCTTGGTATCAG
CAGAAATCAGGAAATGCTCCAAGGCTCTTAATATCTGGTTCAACCAGTTTGGAAA
CTGGGGTTCCTTCAAGATTCAGTGGCAGTGGATCTGGAAAGGATTACACTCTCAG
CATTATCAGTCTTCAGACTGAAGATGTTGCTACTTATTACTGTCAACAGTTTTGGA

GTACTCCGTGGACGTTCGGTGGAGGCACCAAGCTGGAAATAAAA

Clones: 25C5-E9 SEQ ID NOs:51-60

Heavy chain VH

<-------------FR1-----------> CDR1 <----FR2-----> CDR2 <---

QVQLKQSGPGLVQPSQSLSITCTVSGFSLTSYGVHWVRQSPGKGLEWLG**VMWRGGSTD**
**YNAPFMS**RLSI

------------FR3------------> CDR3 <---FR4--->

TKDNSKSQVFFKMNSLQADDTAIYYCAK**SLITTGYAMDY**WGQGTSVTVSS

Nucleotide sequence

CAGGTCCAGCTGAAGCAGTCAGGACCTGGCCTAGTGCAGCCCTCACAGAGCCTGT
CCATAACCTGCACAGTCTCTGGTTTCTCATTAACTAGCTATGGTGTACACTGGGTT
CGCCAGTCTCCAGGAAAGGGTCTGGAGTGGCTGGGAGTGATGTGGAGAGGTGGA
AGCACAGACTACAATGCACCTTTCATGTCCAGACTGAGCATCACCAAGGACAACT
CCAAGAGCCAAGTTTTCTTTAAAATGAACAGTCTGCAAGCTGATGACACTGCCAT
ATACTACTGTGCCAAAAGTCTGATTACGACTGGGTATGCTATGGACTACTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQKPGNAPRLLIS**GATSLET**GVPSR
FSGSGSGK

--------FR3-------> CDR3 <---FR4--->

DYTLSITSLQTEDVATYYC**QQYWSNPYT**FGGGTKLEIK

Nucleotide sequence

GACATCCAGATGACACAATCTTCATCCTCCTTTTCTGTATCTCTAGGAGACAGAGT
CACCATTACTTGCAAGGCAAGTGAGGACATATATAATCGGTTAGCCTGGTATCAG
CAGAAACCAGGAAATGCTCCTAGGCTCTTAATATCTGGTGCAACCAGTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTACTGTCAACAGTATTGGA
GTAATCCGTACACGTTCGGAGGGGGGACCAAGCTGGAAATAAAA

Clones: 25F8-C4 SEQ ID NOs:61-70

Heavy chain VH

<-------------FR1-----------> CDR1 <----FR2-----> CDR2 <--

QAYLQQSGAELVRSGASVKMSCKASGYTF**SSYNMH**WVKQTPGQGLEWIG**YVYPGNGG**

**TNYNQKFKG**KAT

------------FR3-------------> CDR3 <---FR4--->

LTADTSSSTAYMQISSLTSEESAVYFCAR**PESSFHPWFAY**WGQGTLVTVAP

Nucleotide sequence

CAGGCTTATCTACAGCAGTCTGGGGCTGAGCTGGTGAGGTCTGGGGCCTCAGTGA
AGATGTCCTGCAAGGCTTCTGGCTACACATTTTCCAGTTACAATATGCACTGGGTA
AAGCAGACACCTGGACAGGGCCTGGAATGGATTGGATATGTTTATCCTGGAAATG
GTGGTACGAACTACAATCAGAAGTTCAAGGGCAAGGCCACATTGACTGCAGACA
CATCCTCCAGCACAGCCTACATGCAGATCAGCAGCCTGACATCTGAAGAGTCTGC
GGTCTATTTCTGTGCAAGACCAGAGAGTAGTTTCCACCCCTGGTTTGCTTACTGGG
GCCAAGGGACTCTGGTCACTGTCGCGCCA

Light chain VK

<----------FR1--------> CDR1 <-----FR2-----> CDR2 <--------

NIVLTQSPASLAVSLGQRATISCRASESVNIYGNIFMYWYQQKPGQPPKLLIY**LASNLAS**G

VPARFSGS

--------FR3-----------> CDR3 <---FR4--->

GSRTGFTLTIDPVEADDAATYYC**QQNAKDPWT**FGGGTKLEIK

Nucleotide sequence

AACATTGTGCTGACCCAATCTCCAGCTTCTTTGGCTGTGTCTCTAGGGCAGAGGGC
CACCATATCCTGCAGAGCCAGTGAAAGTGTTAATATTTATGGCAATATTTTTATGT
ACTGGTACCAGCAGAAACCAGGACAGCCACCCAAACTCCTCATCTATCTTGCATC
CAACCTAGCATCTGGGGTCCCTGCCAGGTTCAGTGGCAGTGGGTCTAGGACAGGC
TTCACCCTCACCATTGATCCTGTGGAGGCTGATGATGCTGCAACCTATTACTGTCA
GCAAAATGCTAAGGATCCGTGGACGTTCGGTGGAGGCACCAAGCTGGAAATCAA
A

Clones: 40A12-D2 SEQ ID NOs:71-80
Heavy chain VH

<-------------FR1-----------> CDR1 <----FR2-----> CDR2 <---

QVQLKQSGPGLVQPSQSLSITCTVSGFSL**TSYGIH**WVRQSPGKGLEWLG**VMWRGGSTDYNAPFMS**RLSI

-----------FR3-------------> CDR3 <---FR4--->

TKDNSKSQVFFKMNSLQADDTAIYYCAKSLITTGYAMDFWGQGTSVTVSS

Nucleotide sequence

CAGGTCCAGCTGAAGCAGTCAGGACCTGGCCTAGTGCAGCCCTCACAGAGCCTGT
CCATAACCTGCACAGTCTCTGGTTTCTCATTAACTAGCTATGGTATACACTGGGTT
CGCCAGTCTCCAGGAAAGGGTCTGGAGTGGCTGGGAGTGATGTGGAGAGGTGGA
AGCACAGACTACAATGCACCTTTTATGTCCAGACTGAGCATCACCAAGGACAACT
CCAAGAGCCAAGTTTTCTTTAAAATGAACAGTCTGCAAGCTGATGACACTGCCAT
ATATTACTGTGCCAAAAGTCTGATTACGACTGGGTATGCTATGGACTTCTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQKPGNAPRLLIS**GATSLET**GVPSRFSGSGSGK

-----FR3----------> CDR3 <---FR4--->

DYTLSITSLQTEDVATYYC**QQYWSNPYT**FGGGTKLEIK

Nucleotide sequence

GACATCCAGATGACACAATCTTCATCCTCCTTTTCTGTATCTCTAGGAGACAGAGT
CACCATTACTTGCAAGGCAAGTGAGGACATATATAATCGGTTAGCCTGGTATCAG
CAGAAACCAGGAAATGCTCCTAGGCTCTTAATATCTGGTGCAACCAGTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTACTGTCAACAGTATTGGA
GTAATCCGTACACGTTCGGAGGGGGGACCAAGCTGGAAATAAAA

Clones: 36G11-E10 SEQ ID NOs:81-90
Heavy chain VH

<-------------FR1-----------> CDR1 <----FR2-----> CDR2 <---

QVQLKQSGPSPVQPSQSLSITCTVSGFSL**TSYGVH**WVRQSPGKGLEWLG**VIWRGGSTDY**

**NAVFMS**RLSI

-----------FR3-------------> CDR3 <---FR4--->

TKDNSKSQVFFKMNSLQTDDTAIYYCAK**SLVTTGYTMDY**WGQGTSVTVSS

Nucleotide sequence

CAGGTCCAGCTGAAGCAGTCAGGACCTAGCCCAGTGCAGCCCTCACAGAGCCTGT
CCATAACCTGCACAGTCTCTGGTTTCTCATTAACGAGCTATGGGGTACACTGGGTT
CGCCAGTCTCCAGGAAAGGGTCTGGAGTGGCTGGGAGTGATATGGAGAGGTGGA
AGTACAGACTACAATGCAGTTTTCATGTCCAGACTGAGCATCACCAAGGACAACT
CCAAGAGCCAAGTTTTCTTTAAAATGAACAGTCTGCAAACTGATGACACTGCCAT
ATACTACTGTGCCAAAAGTCTTGTTACTACGGGCTATACTATGGACTACTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITCKASEDIYNRLAWYQQRPGNAPRLLIS**GTTSLET**GVPSRF SGSGSGK

-----FR3----------> CDR3 <---FR4--->

DYTLSITSLQTEDVATYYC**QQYWSIPWT**FGGGTKLEIK

Nucleotide sequence

GACATCCAGATGACACAGTCTTCATCCTCCTTTTCTGTATCTCTAGGAGACAGAGT
CACCATTACTTGCAAGGCAAGTGAGGACATATATAATCGGTTAGCCTGGTATCAG
CAGAGACCAGGAAATGCTCCTAGGCTCTTAATATCTGGTACAACCAGTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTACTGTCAACAGTATTGGA
GTATTCCGTGGACGTTCGGTGGAGGCACCAAGCTGGAAATAAAA

Clones: 26C12-G9 SEQ ID NOs:91-100
Heavy chain VH

<-------------FR1-----------> CDR1 <----FR2-----> CDR2 <---

QVQLKQSGPGLVQPSQSLSIICTVSGFSL**SSYGIH**WVRQSPGKGLEWLG**VIWRGGNTDYS VTFMS**RLSI

-----------FR3-------------> CDR3 <---FR4--->

TKDNSKSQVFFKMNSLQADDTAIYYCAK**SLITTGYAMDY**WGQGTSVTVSS

Nucleotide sequence

CAGGTCCAGCTGAAGCAGTCAGGACCTGGCCTAGTGCAGCCCTCACAGAGCCTGT
CCATAATCTGCACAGTCTCTGGTTTCTCCTTAAGTAGTTATGGTATTCACTGGGTT
CGCCAGTCTCCAGGAAAGGGTCTGGAGTGGCTGGGAGTGATATGGAGAGGTGGA
AACACAGACTACAGTGTAACTTTCATGTCCAGACTGAGCATCACCAAGGACAACT
CCAAGAGCCAAGTTTTCTTTAAAATGAACAGTCTGCAAGCTGATGACACTGCCAT
ATATTATTGTGCCAAAAGTTTGATTACGACAGGGTATGCTATGGACTACTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQKPGTTPRLLIS**GATSLET**GVPSR FSGSGSGK

-----FR3----------> CDR3 <---FR4--->

DYTLSITSLQTEDVGTYYC**QQYWSNPRT**FGGGTKLEIK

Nucleotide sequence

GACATCCAGATGACACAATCTTCATCCTCCTTTTCTGTTTCTCTAGGAGACAGAGT
CACCATTACTTGCAAGGCAAGTGAAGACATATATAATCGGTTAGCCTGGTATCAG
CAGAAACCAGGAACTACTCCTCGGCTCTTAATATCTGGTGCAACCAGTTTGGAAA
CTGGGGTTCCTTCAAGATTCAGTGGCAGTGGATCTGGAAAAGATTACACTCTCAG
CATTACCAGTCTTCAGACTGAAGATGTTGGTACTTATTACTGTCAACAGTATTGGA
GTAATCCGCGGACGTTCGGTGGAGGCACCAAGCTGGAAATAAAA

Clones: 20E12-C6 SEQ ID NOs:101-110
Heavy chain VH

<-------------FR1-----------> CDR1 <----FR2-----> CDR2 <---

QVQLKQSGPGLVQPSQTLSITCTVSGFSL**TSYGVH**WVRQSPGKGLEWLG**VIWRGGGTEY NAAFMS**RLSI

-----------FR3-------------> CDR3 <---FR4--->

TKDNSKSQVFLKMNSLQADDTAIYYCAK**SMITTGYAMDF**WGQGTSVTVSS

Nucleotide sequence

CAGGTCCAGCTGAAGCAGTCAGGACCTGGCCTAGTGCAGCCCTCACAGACCCTGT
CCATAACCTGCACAGTCTCTGGTTTCTCATTAACTAGCTATGGTGTACACTGGGTT
CGCCAGTCTCCAGGAAAGGGTCTGGAGTGGCTGGGAGTGATATGGAGAGGTGGA
GGCACAGAGTACAATGCAGCTTTCATGTCCAGACTGAGCATCACCAAGGACAACT
CCAAGAGCCAAGTTTTCTTAAAGATGAACAGTCTGCAAGCTGATGACACTGCCAT
ATACTACTGTGCCAAGTCGATGATTACGACAGGTTATGCTATGGACTTCTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITC**KASEDIYKRLA**WYQQKPGNAPRLLIS**GATSLET**GIPSR FSGSGSGK

-----FR3----------> CDR3 <---FR4--->

DYTLSLTSLQTEDVATYYC**QQYWSSPRT**FGGGAKLEIK

Nucleotide sequence

GACATCCAGATGACACAATCTTCATCCTCCTTTTCTGTATCTCTAGGAGACAGAGT
CACCATCACTTGCAAGGCAAGTGAGGACATATATAAGCGGTTAGCCTGGTATCAG
CAGAAACCAGGAAATGCTCCTAGGCTCTTAATATCTGGTGCAACCAGTTTGGAAA
CTGGGATTCCTTCAAGATTCAGTGGCAGTGGATCTGGAAAGGATTACACTCTCAG
CCTTACCAGTCTTCAGACTGAAGATGTTGCTACTTATTACTGTCAACAGTATTGGA
GTTCTCCTCGGACGTTCGGTGGAGGCGCCAAGCTGGAAATAAAA

Clones: 22D3-F9 SEQ ID NOs:111-120

Heavy chain VH

<-------------FR1-----------> CDR1 <----FR2-----> CDR2 <---

QVQLKQSGPGLVQPSQSLSITCTVSGFSL**TTYGIH**WIRQSPGRGLEWLG**VIWRGGSTDYN AAFMS**RLSF

-----------FR3-------------> CDR3 <---FR4--->

TKDNSKSQVFFKMNSLQADDTAIYYCAK**SMITTGFTMDY**WGQGTSVTVSS

Nucleotide sequence

CAGGTCCAGCTGAAGCAGTCAGGACCTGGCCTAGTGCAGCCCTCACAGAGCCTGT
CCATAACCTGCACAGTCTCTGGTTTCTCATTGACTACCTATGGTATACACTGGATT

CGCCAGTCTCCTGGAAGGGGTCTGGAGTGGCTGGGAGTGATTTGGAGAGGTGGA
AGCACAGACTACAATGCAGCTTTCATGTCCAGACTGAGCTTCACCAAGGACAACT
CCAAGAGCCAAGTTTTCTTTAAAATGAACAGTCTGCAAGCTGATGACACTGCCAT
ATACTACTGTGCCAAAAGTATGATTACGACTGGATTTACTATGGACTACTGGGGT
CAAGGAACCTCAGTCACCGTCTCCTCAg

Light chain VK

<----------FR1--------> CDR1 <-----FR2-----> CDR2 <------------

DIQMTQSSSSFSVSLGDRVTITC**RASEDIYNRLA**WYQQKPGNAPRLLIS**GATSLET**GVPSRFSGSGSGK

-----FR3----------> CDR3 <---FR4--->

DYTLSITSLQTEDVATYYC**QQFWSTPYT**FGGGTKLEIK

Nucleotide sequence

GACATCCAGATGACACAATCTTCATCCTCCTTTTCTGTATCTCTAGGAGACAGAGT
CACCATTACTTGCAGGGCAAGTGAGGACATATATAACCGGTTAGCCTGGTATCAG
CAGAAACCAGGAAATGCTCCTAGGCTCTTAATATCTGGTGCAACCAGTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTATTGTCAACAGTTTTGGA
GTACTCCGTACACGTTCGGAGGGGGGACCAAGCTGGAAATAAAA

Clones: 12C7-A11 SEQ ID NOs:121-130 Heavy chain VH

<-------------FR1-----------> CDR1 <----FR2-----> CDR2 <---

QVQLKQSGPGLVQPSQSLSISCSVSGFSL**TSYGVH**WVRQSPGKGLEWLG**VIWRGGSTDYNAAFSS**RLSI

-----------FR3-------------> CDR3 <---FR4--->

TKDNSKSQVFFKMNSLQADDTAIYYCAK**SMITTGRYFDV**WGAGTTVTVSS

Nucleotide sequence

CAGGTCCAGCTGAAGCAGTCAGGACCTGGCCTAGTGCAGCCCTCACAGAGCCTGT
CCATATCCTGCTCAGTCTCTGGTTTCTCATTAACTAGTTATGGTGTACACTGGGTT
CGCCAGTCTCCAGGAAAGGGTCTGGAGTGGCTGGGAGTGATATGGAGAGGAGGA
AGCACAGACTACAATGCAGCTTTCTCGTCCAGACTGAGCATCACCAAGGACAACT
CCAAGAGCCAAGTTTTCTTTAAAATGAACAGTCTGCAAGCTGATGACACTGCCAT
ATATTACTGTGCCAAATCTATGATTACGACGGGGAGGTACTTCGATGTCTGGGGC
GCAGGGACCACGGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQKPGNAPRLLIS**GAASLET**GIPSR
FSGSGSGK

-----FR3----------> CDR3 <---FR4--->

NYTLSITSLQTEDVATYYC**QQYWSTPWT**FGGGTKLEIK

Nucleotide sequence

GACATCCAGATGACACAATCTTCATCCTCCTTTTCTGTATCTCTCGGAGACAGAGT
CACCATTACTTGCAAGGCAAGTGAGGACATATATAATCGGTTAGCCTGGTATCAG
CAGAAACCAGGAAATGCTCCTAGGCTCTTAATATCTGGTGCAGCCAGTTTGGAAA
CTGGGATTCCTTCAAGATTCAGTGGCAGTGGATCTGGAAAGAATTACACTCTCAG
CATTACCAGTCTTCAGACTGAAGATGTTGCTACTTATTACTGTCAACAGTATTGGA
GTACTCCGTGGACGTTCGGTGGAGGCACCAAGCTGGAAATAAAA

Clones: 11F12-E1 SEQ ID NOs:131-140
Heavy chain VH

<-------------FR1-----------> CDR1 <----FR2-----> CDR2 <---

QVQLKQSGPGLVQPSQSLSITCSVSGFSLTSYGVHWVRQSPGKGLEWLG**VIWRGGSTDY
NAAFMS**RLSI

-----------FR3-------------> CDR3 <---FR4--->

TKDNSKSQVFFKMNSLQADDTAIYYCAK**SMITTGRYFDV**WGAGTTVTVSS

Nucleotide sequence

CAGGTCCAGCTGAAGCAGTCAGGACCTGGCCTAGTGCAGCCCTCACAGAGCCTGT
CCATAACCTGCTCAGTCTCTGGTTTCTCATTAACTAGCTATGGTGTACACTGGGTT
CGCCAGTCTCCAGGAAAGGGTCTGGAGTGGCTGGGAGTGATATGGAGAGGAGGA
AGCACAGACTACAATGCAGCTTTCATGTCCAGACTGAGCATCACCAAGGACAACT
CCAAGAGCCAAGTTTTCTTTAAAATGAACAGTCTGCAAGCTGATGACACTGCCAT
ATACTACTGTGCCAAATCTATGATTACGACGGGGAGGTACTTCGATGTCTGGGGC
GCAGGGACCACGGTCACCGTCTCCTCA

Light chain VK

<----------FR1--------> CDR1 <-----FR2-----> CDR2 <------------

DIQMTQSSSSFSVSLGDRVTITC**KASEDIYNRLA**WYQQRPGNAPRLLIS**GATSLET**GVPSR
FSGSGSGK

-----FR3----------> CDR3 <---FR4--->

DYTLSITSLQTEDVATYYC**QQYWSAPWT**FGGGTKLEIK

Nucleotide sequence

GACATCCAGATGACACAATCTTCATCCTCCTTTTCTGTATCTCTCGGAGACAGAGT
CACCATTACTTGCAAGGCAAGTGAGGACATATATAATCGGTTAGCCTGGTATCAG
CAGAGACCAGGAAATGCTCCTAGGCTCTTAATATCTGGTGCAACCAGTTTGGAAA
CTGGGGTTCCTTCAAGATTCAGTGGCAGTGGCTCTGGAAAGGATTACACTCTCAG
CATTACCAGTCTTCAGACTGAAGATGTTGCTACTTATTACTGTCAACAGTATTGGA
GTGCTCCGTGGACGTTCGGTGGAGGCACCAAGCTGGAAATAAAA

Clones: 23C4-D1 SEQ ID NOs: 141-150
Heavy chain VH

<-------------FR1-----------> CDR1 <----FR2-----> CDR2 <---

DVHLVESGGGLVQPGGSRKLSCAASGFTF**SSFGMH**WVRQAPERGLEWVA**YISGDSNTIY YTDTVK**GRFT

-----------FR3--------------> CDR3 <---FR4--->

ISRDNPKNTLFLQMTSLRSEDTAMYYCAK**SFDYAMDY**WGQGTSVTVSS

Nucleotide sequence

GAtGTGCAtCTGGTGGAGTCTGGGGGAGGCTTAGTGCAGCCTGGAGGGTCCCGGA
AACTCTCCTGTGCAGCCTCTGGATTCACTTTCAGTAGCTTTGGAATGCACTGGGTT
CGTCAGGCTCCAGAGAGGGGGCTGGAGTGGGTCGCATATATCAGTGGTGACAGT
AATACCATCTACTATACAGACACAGTGAAGGGCCGGTTCACCATCTCCAGAGACA
ATCCCAAGAACACCCTGTTCCTGCAAATGACCAGTCTAAGGTCTGAGGACACGGC
CATGTATTACTGTGCAAAATCGTTCGACTATGCTATGGACTACTGGGGTCAGGGA
ACCTCAGTCACCGTCTCCTCA

Light chain VK

<----------FR1--------> CDR1 <-----FR2-----> CDR2 <------

DIVMSQSPSSLAVSVGEKVTLSC**KSSQSLLYSTNQKNYLA**WYLQKPGQSPKLLIY**WAST RES**GVPDRFS

-------FR3-----------> CDR3 <---FR4--->

GSGSGTDFALTINTVKAEDLAV**YYCQQYYTYPYT**FGGGTKLEIK

Nucleotide sequence

GACATTGTGATGTCACAGTCTCCATCCTCCCTAGCTGTGTCAGTTGGAGAGAAGG
TTACTTTGAGCTGCAAGTCCAGTCAGAGCCTTTTATATAGTACCAATCAAAAGAA
CTACTTGGCCTGGTACCTGCAGAAACCAGGGCAGTCTCCTAAACTGCTGATTTAC
TGGGCATCCACTAGGGAATCTGGGGTCCCTGATCGCTTCTCAGGCAGTGGATCTG
GGACAGATTTCGCTCTCACCATCAATACTGTGAAGGCTGAAGACCTGGCAGTTTA
TTACTGTCAGCAATATTATACCTATCCGTACACGTTCGGAGGGGGGACCAAGCTG
GAAATAAAA

Clones: 29F3-G8 SEQ ID NOs:151-160 Heavy chain VH

<-------------FR1-----------> CDR1 <----FR2-----> CDR2 <---

QVQLKQSGPGLVQPSQSLSITCTVSGFSL**TSYGIH**WLRQSPGKGLEWLG**VIWRGGSTDY NAAFMS**RLSI

-----------FR3-------------> CDR3 <---FR4--->

TKDNSKSQVFFKMNSLQADDTAIYYCAK**MRVTTGFTMDY**WGQGTSVTVSS

Nucleotide sequence

CAGGTCCAGCTGAAGCAGTCAGGACCTGGCCTAGTGCAGCCCTCACAGAGCCTGT
CCATAACCTGCACAGTCTCTGGTTTCTCATTAACTAGCTACGGTATACACTGGCTT
CGCCAGTCTCCAGGAAAGGGTCTGGAGTGGCTGGGAGTGATATGGAGAGGTGGA
AGCACAGACTACAATGCAGCTTTCATGTCCAGACTGAGCATCACCAAGGACAACT
CCAAGAGCCAAGTTTTCTTTAAAATGAACAGTCTGCAAGCTGATGACACTGCCAT
ATACTACTGTGCCAAAATGAGAGTTACGACGGGGTTTACTATGGACTACTGGGGT
CAAGGAACCTCAGTCACCGTCTCCTCA

Light chain VK

<----------FR1--------> CDR1 <-----FR2-----> CDR2 <------------

DIQMTQSSSSFSVSLGDRVTITCKASEDIYNRLAWYQQKPGNAPRLLIS**GATSLET**GVPSR FSGSGSGK

-----FR3----------> CDR3 <---FR4--->

DYTLSITSLQTEDVATYYC**QQYWSNPYT**FGGGTKLEIK

Nucleotide sequence

GACATCCAGATGACACAATCTTCATCCTCCTTTTCTGTATCTCTAGGAGACAGAGT
CACCATTACTTGCAAGGCAAGTGAGGACATATATAATCGGTTAGCCTGGTATCAG
CAGAAACCAGGAAATGCTCCTAGGCTCTTAATATCTGGTGCAACCAGTTTGGAAA
CTGGGGTTCCTTCAAGATTCAGTGGCAGTGGATCTGGAAAGGATTACACTCTCAG
CATTACCAGTCTTCAGACTGAAGATGTTGCTACTTATTACTGTCAACAGTATTGGA
GTAATCCGTACACGTTCGGAGGGGGGACCAAGCTGGAAATAAAA

Clones: 27F8 SEQ ID NOs:161-170
Heavy chain VH

<-------------FR1-----------> CDR1 <----FR2-----> CDR2 <---

QVLQQSGPELEKPGASVKISCKASGYSF**TVYNIN**WVKQSNGKSLEWIG**NIDPYFGGTIY**
**NQKFK**DKAT

-----------FR3--------------> CDR3 <---FR4--->

LTVDKSSSTAYMELKSLTSEDSAVYYCER**SRGTWRFAYW**GQGTLVTVSA

Nucleotide sequence

CAGGTCCAGCTGCAGCAGTCTGGACCTGAGTTGGAGAAGCCTGGCGCTTCAGTGA
AGATATCCTGCAAGGCTTCTGGTTACTCATTCACTGTCTACAACATAAACTGGGTG
AAACAGAGCAATGGAAAGAGCCTTGAGTGGATTGGAAATATTGATCCTTACTTTG
GTGGTACTATTTACAACCAGAAATTCAAGGACAAGGCCACATTGACTGTCGACAA
ATCCTCCAGCACAGCCTACATGGAGCTCAAGAGCCTGACATCTGAGGACTCTGCA
GTCTATTACTGTGAAAGATCGAGGGGGACGTGGAGGTTTGCTTACTGGGGCCAAG
GGACTCTGGTCACTGTCTCTGCA

Light chain VK

<----------FR1--------> CDR1 <-----FR2-----> CDR2 <--------

DIVMTQSPSSMSVSLGDTVTITC**HASQGISSNIG**WLQQKPGKSFKGLIY**HGATLED**GIPSR FSGSGSGA

-----FR3----------> CDR3 <---FR4--->

DYSLTISSLDSEDFADYYCVQYAQFPYTFGGGTKLEIK

Nucleotide sequence

GACATTGTGATGACCCAGTCTCCATCCTCCATGTCTGTATCTCTGGGAGACACAGT CACCATCACTTGCCATGCAAGTCAGGGCATTAGCAGTAATATAGGGTGGTTGCAG CAGAAACCAGGGAAATCATTTAAGGGCCTGATCTATCATGGAGCCACCTTGGAAG ATGGAATTCCATCAAGGTTCAGTGGCAGTGGATCTGGAGCAGATTATTCTCTCAC CATCAGCAGCCTGGACTCTGAAGATTTTGCAGACTATTACTGCGTACAGTATGCT CAGTTTCCGTACACGTTCGGAGGGGGGACCAAGCTGGAAATCAAA

Clones: 48H1-B11 SEQ ID NOs:171-180
Heavy chain VH

<-------------FR1-----------> CDR1 <----FR2-----> CDR2 <---

EVQLKQSGPGLMQPSQSLSITCTVSGFSL**TSYGIH**WLRQSPGKGLEWLG**VIWRGGSTDY NAAFMS**RLSI

-----------FR3-------------> CDR3 <---FR4--->

TKDNSKSQVFFKMNSLQGDDTAIYYCAK**GKVTTGFYFDF**WGQGTTLTVSS

Nucleotide sequence

GAGGTGCAGCTGAAGCAGTCAGGACCTGGCCTAATGCAGCCCTCACAGAGCCTGT CCATAACCTGCACAGTCTCTGGTTTCTCATTAACTAGCTATGGTATACACTGGCTT CGTCAGTCTCCAGGAAAGGGTCTGGAGTGGCTGGGAGTGATATGGAGAGGTGGA AGCACAGACTACAATGCAGCTTTCATGTCCAGACTGAGCATCACCAAGGACAACT CCAAGAGCCAAGTTTTCTTTAAAATGAACAGTCTGCAAGGTGATGACACTGCCAT ATACTACTGTGCCAAAGGGAAGGTTACGACGGGGTTCTACTTTGACTTCTGGGGC CAAGGCACCACTCTCACAGTCTCCTCA

Light chain VK

<----------FR1--------> CDR1 <-----FR2-----> CDR2 <------------

DIQMTQSSSSFSVSLGDRVTITC**KASEDIYNRLV**WYQQKPGNAPGLLIS**GVTSLET**GVPSR FSGSGSGK

-----FR3----------> CDR3 <---FR4--->

DYTLTITSLQTEDVATYYC**QQYWSTPYT**FGGGTKLEIK

Nucleotide sequence

GACATCCAGATGACACAATCTTCATCCTCCTTTTCTGTATCTCTAGGAGACAGAGT
CACCATTACTTGCAAGGCAAGTGAGGACATATATAATCGGTTAGTCTGGTATCAG
CAGAAACCAGGAAATGCTCCTGGGCTCTTAATATCTGGTGTAACCAGTTTGGAAA
CTGGAGTTCCTTCAAGATTCAGTGGCAGTGGATCTGGAAAGGATTACACTCTCAC
TATTACCAGTCTTCAGACTGAAGATGTTGCTACTTATTACTGTCAACAGTATTGGA
GTACTCCGTACACGTTCGGAGGGGGAACCAAGCTGGAAATAAAA

The heavy chain variable region was cloned into a vector containing a human heavy chain constant region and regulatory elements to express the intact IgG heavy chain in mammalian cells. Similarly, the light chain variable region was cloned into a vector containing a human light chain constant region and regulatory elements to express the intact IgG light chain in mammalian cells. The vectors were verified by sequencing and then transfected into CHO-S mammalian cells, and IgG was expressed and secreted into the culture medium, the supernatant was combined and collected, purified after being filtered. IgG was purified by Protein A chromatography, the supernatant was loaded onto a Protein A column of appropriate size, washed with 50 mM Tris-HCl pH 8.0, 250 mM NaCl, and the bound IgG was eluted with 0.1 M Glycine-HCl (pH 3.0). The protein was ultrafiltered and concentrated using a concentration tube, OD280 was measured, and the concentration of IgG was determined by spectrophotometry.

4) CDC activity of chimeric antibodies on Daudi cells

Daudi cells were resuspended and plated into a 96-well plate at 50 μL/well and 50 μL of antibodies was added to each well. The humanized antibodies were diluted to 10 μg/mL as a starting concentration, and 3-fold serial dilutions was performed, with a total of 3 dilution series. 50 μL of complement mix was added to each well and the 96-well plate was placed in a 5% $CO_2$ incubator at 37°C for 2 hours. Then 50 μL of 40% CCK-8 solution was added to each well, the plate was shaken for 10 seconds and incubated in a 5% $CO_2$ incubator for 3 hours. The 96-well plate was placed in a microplate reader (BioTek, Synergy HT) and OD450 values were read and the collected data were calculated using GraphPad Prism 5 software. The results were shown in FIGs. 3-1 and 3-2.

5) Inhibition of chimeric antibodies on CD38 enzyme activity

The synthesis and degradation of cyclic ADP-ribose (cADPR) were catalyzed by CD38, nicotinamide adenine dinucleotide (NAD+) was cyclized to cyclic adenosine-diphosphate-ribose (cADPR), which was hydrolyzed to ADPR. Due to the instability of cADPR, the catalytically produced cGDPR was often detected using nicotinamide guanine dinucleotide (NGD+) as a substrate in CD38 enzymatic activity studies. The anti-CD38 antibody may bind to CD38 and inhibit the production of cGDPR from NGD+ catalyzed by CD38 protease. The Daudi cells were mixed well with the serially diluted chimeric antibodies and incubated at room temperature for 15 minutes; NGD substrate was added at different time and mixed well for detection; the results were shown in Table 1 and FIG.s 4-1 and 4-2.

Table 1: Screening of CD38 chimeric antibodies for biological activity

| CD38 antibody | CDC activity | Inhibition of CD38 enzyme activity |
|---|---|---|
| 21H12 G2 | - | + |
| 7C10-G5 | - | + |
| 15A1-G1 | - | + |
| 51G7-D3 | - | + |
| 17A4-H9 | + | + |
| 25C5-E9 | + | + |
| 25F8-C4 | + | + |
| 40A12-D2 | + | + |
| 36G11-E10 | + | + |
| 26C12-G9 | + | + |
| 20E12-C6 | + | + |
| 22D3-F9 | + | + |
| 12C7-A11 | + | + |
| 11F12-E1 | + | + |
| 23C4-D1 | + | + |
| 29F3-G8 | + | + |
| 27F8 | + | + |
| 48H1-B11 | ++ | + |

6) ADCC activity of chimeric antibodies on Daudi cells

Daudi cells were plated at a density of 1×$10^6$/mL in a U-shaped 96-well plate at 50 μL per well, 50 μL antibodies were added per well, and the plate was incubated in a 5% $CO_2$ incubator at 37°C for 30 minutes. PBMC cells were fetched from a liquid nitrogen container, quickly thawed in water bath at 37°C, and quickly transferred into a 15 mL centrifuge tube, RPMI 1640 culture medium was added, followed by counting and centrifuging at 300 * g for 10 minutes. The supernatant was discarded, and RPMI-1640 culture medium was resuspended to the cell density to 1×$10^7$/mL; the 96-well plate was taken out, and 50 μL PBMC cells were added to each well, and the plate was incubated in a 5% $CO_2$ incubator for 4 hours. Thirty minutes prior to assay, 2 μL of cell lysate (100×) was added to the maximal well of target cells, and the mixture was cultured at 37°C. The culture solution was centrifuged at 300 * g for 3 minutes, 50 μL of supernatant was added to a black ELISA plate, 50 μL of LDH detection substrate was added to each well (dissolving at room temperature in advance), and the plate was gently shaken and mixed well; after 10 minutes, the reaction was terminated by adding 25 μL stop solution to each well, and the plate was shaken for 10 seconds, followed by plate reading by selecting fluorescence (excitation wavelength of 560 nm, emission wavelength 590 nm). The results were shown in FIG. 5, wherein the chimeric antibodies 48H1-B11 had significant ADCC killing effect on Daudi cells in a dose-dependent manner.

7) ADCP activity of chimeric antibodies on Daudi cells

Daudi cells labeled with 1 μM CFSE were used as CD38+ target cells, 50 μL of the cells were seeded into a 96-well plate at 1×$10^5$/well, the antibodies were diluted 4-fold, 50 μL of the antibodies were added to the plate, finally 100 μL macrophages induced to mature for 7 days with GM-CSF1 at a density of 2.5×$10^5$/ml were added, with a ratio of macrophage : Daudi = 1:4, the mixture was incubated in 5% $CO_2$ incubator at 37°C for 4 hours, and finally the cells were collected and detected by flow cytometry. The results were shown in FIG. 6, wherein the chimeric antibodies 48H1-B11 had significant ADCP killing effect on Daudi cells in a dose-dependent manner.

**Example 5. Humanization of anti-CD38 antibody**

mAb48H1-B11 murine antibody light chain was mouse IMGT_mVK13-84; firstly, the humanized framework region was screened, and IMGT_hVK_1_39, IMGT_hVK_2D_28, IMGT_hVK_3_7, IMGT_hVK_3_20, and IMGT_hVK_4_1 were selected respectively for CDR transplantation to obtain five humanized light chain variants h48H1-Vkv1-v5; the heavy chain of murine antibody was IGHV 2-5, and IMGT_hVH_1_69, IMGT_hVH_2_26, IMGT_hVH_3_23, IMGT_hVH_4_59, IMGT_hVH_5_51, and IMGT_hVH_7_4_1 were selected for CDR transplantation to obtain 6 humanized heavy chain variants h48H1-VHv1-v6. The light and heavy chain variants were paired in combination, then transiently expressed in CHO-S cells, and tested for affinity to the antibody and CDC activity.

Human IMGT_hVK3-20 with highest homology to light chain framework region of murine antibody mAb48H1-B11 from light and heavy chain variants completely retaining CDC activity of chimeric antibodies was selected for performing humanized sequence optimization, and human IGKJ4 with highest homology was selected for FM4; the human heavy chain germline gene IMGT_hVH 4-59 was selected for CDR transplantation, and human IGHJ4*01 with the highest homology was selected for FM4. In silico homology modeling was conducted, the CDR region and its surrounding framework amino acid was analyzed to avoid the concentrated distribution of molecular surface charge or hydrophobic region. A total of five heavy chain variants h48H1-VHv7-v11 and two light chain variants h48H1-VKv7-v8 were designed; after full-sequence synthesis of the light and heavy chains, respectively, they were cloned into eukaryotic expression vectors containing antibody kappa chain constant region Ckappa or human IgG1 constant region CH1-CH3; the light and heavy chain plasmids were paired in combination, then transfected into CHO-S cells and expressed at 37°C for 5-6 days; the culture supernatant was collected and purified by Protein A column.

The humanized antibody heavy/light chain variable region sequences are as follows:

**VH v1:**

<-----------FR1---------> CDR1 <----FR2-----> CDR2 <---

QVQLVQSGAELKKPGSSVKVSCKVS**GFSLTSYGIH**WLRQAPGQGLEWMG**VIWRGGSTD**

**YNAKFQG**RVTI

-----------FR3-------------> CDR3 <---FR4--->

TKDNSKSTVYMELSSLRSEDTAVYYCAK**GKVTTGFYFDF**WGQGTLVTVSS

Nucleotide sequence

CAGGTTCAGCTGGTTCAGTCTGGCGCCGAGCTGAAGAAACCTGGCAGCTCTGTGAAGGTG

TCCTGCAAGGTGTCCGGCTTCAGCCTGACAAGCTACGGCATCCACTGGCTGAGACAGGCC

CCTGGACAAGGCTTGGAATGGATGGGAGTGATTTGGAGAGGCGGCAGCACCGACTACAA

CGCCAAGTTTCAGGGCAGAGTGACCATCACCAAGGACAACAGCAAGAGCACCGTGTACAT

GGAACTGAGCAGCCTGAGAAGCGAGGACACCGCCGTGTACTACTGCGCCAAGGGCAAAG

TGACAACCGGCTTCTACTTCGACTTCTGGGGCCAGGGAACCCTGGTCACAGTCTCTTCT

**VH v2:**

<----------FR1----------> CDR1 <-----FR2----> CDR2 <----

QVTLKESGPVLVKPTETLTLTCTVS**GFSLTSYGIH**WLRQPPGKALEWLA**VIWRGGSTDYNASLKS**RLTIS

K

FR3---------------------> CDR3 <---FR4--->

DNSKSQVVLTMTNMDPVDTATYYCAK**GKVTTGFYFDF**WGQGTLVTVSS

Nucleotide sequence

CAAGTGACCCTGAAAGAAAGCGGCCCTGTGCTGGTCAAGCCCACCGAAACACTGACCCTGACCTGTACCG
TGTCCGGCTTCAGCCTGACAAGCTACGGAATCCACTGGCTGAGACAGCCTCCAGGCAAGGCTCTGGAATG
GCTGGCCGTTATTTGGAGAGGCGGCAGCACAGACTACAACGCCAGCCTGAAGTCCAGACTGACCATCAGC
AAGGACAACAGCAAGAGCCAGGTGGTGCTGACCATGACCAACATGGACCCTGTGGACACCGCCACCTACT
ACTGCGCTAAGGGCAAAGTGACCACCGGCTTCTACTTCGACTTTTGGGGCCAGGGCACCCTGGTCACAGTC
TCTTCT

**VH v3:**

<-----------FR1---------> CDR1 <-----FR2----> CDR2 <-----

EVQLLESGGGLVQPGGSLRLSCAVS**GFSLTSYGIH**WLRQAPGKGLEWVS**VIWRGGSTDYNASVKG**RFT
ISK

--------FR3--------------> CDR3 <----FR4--->

DNSKSTVYLQMNSLRAEDTAVYYCAK**GKVTTGFYFDF**WGQGTLVTVSS

Nucleotide sequence

GAAGTGCAGCTGCTGGAATCTGGCGGAGGACTGGTTCAACCTGGCGGCTCTCTGAGACTGAGCTGTG
CTGTGTCTGGCTTCAGCCTGACCAGCTACGGAATCCACTGGCTGAGACAGGCCCCTGGCAAAGGACT
GGAATGGGTGTCCGTGATTTGGAGAGGCGGCAGCACAGACTACAACGCCTCTGTGAAGGGCAGATTC
ACCATCAGCAAGGACAACAGCAAGAGCACCGTGTACCTGCAGATGAACAGCCTGAGAGCCGAGGAC
ACCGCCGTGTACTACTGTGCCAAGGGCAAAGTGACCACCGGCTTCTACTTCGACTTTTGGGGCCAGG
GCACCCTGGTCACAGTCTCTTCT

**VH v4:**

<-----------FR1---------> CDR1 <----FR2-----> CDR2 <-----

QVQLQESGPGLVKPSETLSLTCTVS**GFSLTSYGIH**WLRQPPGKGLEWIG**VIWRGGSTDYNASLKS**RVTIS
K

--------FR3--------------> CDR3 <---FR4--->

DNSKSQVSLKLSSVTAADTAVYYCAK**GKVTTGFYFDF**WGQGTLVTVSS

Nucleotide sequence

CAGGTTCAGCTGCAAGAGTCTGGACCTGGCCTGGTCAAGCCTAGCGAGACACTGAGCCTGACCTGTA
CCGTGTCTGGCTTCAGCCTGACCAGCTACGGAATCCACTGGCTGAGACAGCCTCCAGGCAAAGGCCT
GGAATGGATCGGAGTGATTTGGAGAGGCGGCAGCACCGACTACAACGCCAGCCTGAAGTCCAGAGT
GACCATCAGCAAGGACAACAGCAAGAGCCAGGTGTCCCTGAAGCTGAGCAGCGTGACAGCCGCTGA
TACCGCCGTGTACTACTGCGCCAAGGGCAAAGTGACCACCGGCTTCTACTTCGACTTTTGGGGCCAG
GGCACCCTGGTCACAGTCTCTTCT

**VH v5:**

<-----------FR1---------> CDR1 <----FR2-----> CDR2 <----

EVQLVQSGAELKKPGESLKISCKVS**GFSLTSYGIH**WLRQMPGKGLEWMG**VIWRGGSTDYNASFQG**QV
TISK

--------FR3--------------> CDR3 <---FR4--->

DNSKSTVYLQWSSLKASDTAMYYCAK**GKVTTGFYFDF**WGQGTLVTVSS

Nucleotide sequence

GAAGTGCAGCTGGTTCAGTCTGGCGCCGAGCTGAAGAAGCCTGGCGAGAGCCTGAAGATCAGCTGC
AAGGTGTCCGGCTTCAGCCTGACCTCTTACGGCATCCACTGGCTGAGACAGATGCCTGGCAAAGGCC
TGGAATGGATGGGAGTGATTTGGAGAGGCGGCAGCACCGACTACAACGCCAGCTTTCAGGGCCAAG
TGACCATCAGCAAGGACAACAGCAAGAGCACCGTGTACCTGCAGTGGTCCAGCCTGAAGGCCAGCG
ACACAGCCATGTACTACTGCGCCAAGGGCAAAGTGACAACCGGCTTCTACTTCGACTTCTGGGGCCA
GGGAACCCTGGTCACAGTCTCTTCT

**VH v6:**

<-----------FR1---------> CDR1 <----FR2-----> CDR2 <----

QVQLVQSGSELKKPGASVKVSCKVS**GFSLTSYGIH**WLRQAPGQGLEWMG**VIWRGGSTDYNAGFTG**RF
VISK

--------FR3--------------> CDR3 <---FR4--->

DNSKSTVYLQICSLKAEDTAVYYCAK**GKVTTGFYFDF**WGQGTLVTVSS

Nucleotide sequence

CAGGTTCAGCTGGTGCAGTCTGGCAGCGAGCTGAAGAAACCTGGCGCCTCTGTGAAGGTGTCCTGCA

AGGTGTCCGGCTTCAGCCTGACAAGCTACGGCATCCACTGGCTGAGACAGGCCCCTGGACAAGGCTT

GGAATGGATGGGAGTGATTTGGAGAGGCGGCAGCACCGATTACAACGCCGGCTTCACAGGCAGATT

CGTGATCAGCAAGGACAACAGCAAGAGCACCGTGTACCTGCAGATCTGTAGCCTGAAGGCCGAGGA

CACCGCCGTGTACTACTGTGCCAAGGGCAAAGTGACCACCGGCTTCTACTTCGACTTTTGGGGCCAG

GGCACCCTGGTCACAGTCTCTTCT

**VH v7:**

<-----------FR1---------> CDR1 <----FR2-----> CDR2 <-----

QVQLQESGPGLVKPSETLSLTCTVS**GFSLTSYGIH**WIRQPPGKGLEWIG**VIWRGGSTDYNPSLKS**RVTIS

K

--------FR3--------------> CDR3 <---FR4--->

DNSKSQVSLKLSSVTAADTAVYYCAK**GKVTTGFYFDF**WGQGTLVTVSS

Nucleotide sequence

CAGGTTCAGCTGCAAGAGTCTGGACCTGGCCTGGTCAAGCCTAGCGAGACACTGAGCCTGACCTGTA

CCGTGTCTGGCTTCAGCCTGACCAGCTACGGAATCCACTGGatcAGACAGCCTCCAGGCAAAGGCCTG

GAATGGATCGGAGTGATTTGGAGAGGCGGCAGCACCGACTACAACcccAGCCTGAAGTCCAGAGTGA

CCATCAGCAAGGACAACAGCAAGAGCCAGGTGTCCCTGAAGCTGAGCAGCGTGACAGCCGCTGATA

CCGCCGTGTACTACTGCGCCAAGGGCAAAGTGACCACCGGCTTCTACTTCGACTTTTGGGGCCAGGG

CACCCTGGTCACAGTCTCTTCT

**VH v8:**

<-----------FR1---------> CDR1 <----FR2-----> CDR2 <-----

QVQLQESGPGLVKPSETLSLTCTVS**GFSLTSYGIH**WLRQPPGKGLEWIG**VIWRGGSTDYNPSLKS**RVTIS

K

--------FR3--------------> CDR3 <---FR4--->

DTSKSQVSLKLSSVTAADTAVYYCAK**GKVTTGFYFDF**WGQGTLVTVSS

Nucleotide sequence

CAGGTTCAGCTGCAAGAGTCTGGACCTGGCCTGGTCAAGCCTAGCGAGACACTGAGCCTGACCTGTA
CCGTGTCTGGCTTCAGCCTGACCAGCTACGGAATCCACTGGCTGAGACAGCCTCCAGGCAAAGGCCT
GGAATGGATCGGAGTGATTTGGAGAGGCGGCAGCACCGACTACAACcccAGCCTGAAGTCCAGAGTG
ACCATCAGCAAGGACaccAGCAAGAGCCAGGTGTCCCTGAAGCTGAGCAGCGTGACAGCCGCTGATA
CCGCCGTGTACTACTGCGCCAAGGGCAAAGTGACCACCGGCTTCTACTTCGACTTTTGGGGCCAGGG
CACCCTGGTCACAGTCTCTTCT

**VH v9:**

<-----------FR1--------->   CDR1   <----FR2----->   CDR2   <-----

QVQLQESGPGLVKPSETLSLTCTVS**GFSLTSYGIH**WIRQPPGKGLEWIG**VIWRGGSTDYNPSLKS**RVTIS
K

--------FR3-------------->   CDR3   <---FR4--->

DTSKSQVSLKLSSVTAADTAVYYCAR**GKVTTGFYFDF**WGQGTLVTVSS

Nucleotide sequence

CAGGTTCAGCTGCAAGAGTCTGGACCTGGCCTGGTCAAGCCTAGCGAGACACTGAGCCTGACCTGTA
CCGTGTCTGGCTTCAGCCTGACCAGCTACGGAATCCACTGGatcAGACAGCCTCCAGGCAAAGGCCTG
GAATGGATCGGAGTGATTTGGAGAGGCGGCAGCACCGACTACAACcccAGCCTGAAGTCCAGAGTGA
CCATCAGCAAGGACaccAGCAAGAGCCAGGTGTCCCTGAAGCTGAGCAGCGTGACAGCCGCTGATAC
CGCCGTGTACTACTGCGCCagaGGCAAAGTGACCACCGGCTTCTACTTCGACTTTTGGGGCCAGGGCA
CCCTGGTCACAGTCTCTTCT

**VH v10:**

<-----------FR1--------->   CDR1   <-----FR2---->   CDR2   <-----

QVQLQESGPGLVKPSETLSLTCTVS<u>GFSLTSYGIH</u>WIRQPPGKGLEWIG**VIWAGGSTDYNPSLKS**RVTISK

--------FR3--------------->   CDR3   <----FR4---->

DTSKSQVSLKLSSVTAADTAVYYCARGKVTTGFYFDFWGQGTLVTVSS

Nucleotide sequence

CAGGTTCAGCTGCAAGAGTCTGGACCTGGCCTGGTCAAGCCTAGCGAGACACTGAGCCTGACCTGTA

CCGTGTCTGGCTTCAGCCTGACCAGCTACGGAATCCACTGGatcAGACAGCCTCCAGGCAAAGGCCTG

GAATGGATCGGAGTGATTTGGgctGGCGGCAGCACCGACTACAACcccAGCCTGAAGTCCAGAGTGACC

ATCAGCAAGGACaccAGCAAGAGCCAGGTGTCCCTGAAGCTGAGCAGCGTGACAGCCGCTGATACCG

CCGTGTACTACTGCGCCagaGGCAAAGTGACCACCGGCTTCTACTTCGACTTTTGGGGCCAGGGCACC

CTGGTCACAGTCTCTTCT

**VH v11:**

<-----------FR1---------> CDR1 <-----FR2----> CDR2 <---

QVQLQESGPGLVKPSETLSLTCTVS**GFSLTSYGIH**WIRQPPGKGLEWIG**VIWRGGSTDYNPSLQS**RVTIS

K

--------FR3---------------> CDR3 <---FR4--->

DTSKSQVSLKLSSVTAADTAVYYCAR**GKVTTGFYFDF**WGQGTLVTVSS

Nucleotide sequence

CAGGTTCAGCTGCAAGAGTCTGGACCTGGCCTGGTCAAGCCTAGCGAGACACTGAGCCTGACCTGTA

CCGTGTCTGGCTTCAGCCTGACCAGCTACGGAATCCACTGGatcAGACAGCCTCCAGGCAAAGGCCTG

GAATGGATCGGAGTGATTTGGAGAGGCGGCAGCACCGACTACAACcccAGCCTGcAGTCCAGAGTGAC

CATCAGCAAGGACaccAGCAAGAGCCAGGTGTCCCTGAAGCTGAGCAGCGTGACAGCCGCTGATACC

GCCGTGTACTACTGCGCCagaGGCAAAGTGACCACCGGCTTCTACTTCGACTTTTGGGGCCAGGGCAC

CCTGGTCACAGTCTCTTCT

**VK v1:**

<---------FR1---------> CDR1 <-----FR2-----> CDR2 <--------------

DIQMTQSPSSLSASVGDRVTITC**RASEDIYNRLV**WYQQKPGKAPKLLIS**GVTSLET**GVPSRFSGSGSGKD

Y

FR3-------------> CDR3 <---FR4-->

TLTISSLQPEDFATYYC**QQYWSTPYT**FGQGTKVEIK

Nucleotide sequence

GACATCCAGATGACACAGAGCCCTAGCAGCCTGTCTGCCAGCGTGGGAGACAGAGTGACCATCACCT
GTAGAGCCAGCGAGGACATCTACAACAGACTCGTGTGGTATCAGCAGAAGCCCGGCAAGGCTCCCA
AGCTGCTGATTAGCGGAGTGACCAGCCTGGAAACAGGCGTGCCAAGCAGATTCAGCGGCTCTGGCTC
TGGCAAGGACTACACCCTGACCATCTCTAGCCTGCAGCCTGAGGACTTCGCCACCTACTACTGCCAG
CAGTACTGGTCTACCCCTTACACCTTCGGCCAGGGCACCAAGGTGGAAATCAAG

**VK v2:**

<---------FR1---------> CDR1 <-----FR2-----> CDR2 <--------------

DIVMTQSPLSLPVTPGEPASISC**RASEDIYNRLV**WYLQKPGQSPQLLIS**GVTSLET**GVPDRFSGSGSGKDY

FR3-------------> CDR3 <--FR4--->

TLKISRVEAEDVGVYYC**QQYWSTPYT**FGQGTKVEIK

Nucleotide sequence

GACATCGTGATGACACAGAGCCCTCTGAGCCTGCCTGTGACACCTGGCGAACCTGCCAGCATCAGCT
GTAGAGCCAGCGAGGACATCTACAACAGACTCGTGTGGTATCTGCAGAAGCCCGGCCAGTCTCCTCA
GCTGCTGATTAGCGGAGTGACCAGCCTGGAAACAGGCGTGCCAGACAGATTCAGCGGCTCTGGCTCT
GGCAAGGACTACACCCTGAAGATCAGCAGAGTGGAAGCCGAGGACGTGGGCGTGTACTACTGCCAG
CAGTACTGGTCTACCCCTTACACCTTCGGCCAGGGCACCAAGGTGGAAATCAAG

VK v3:

<---------FR1---------> CDR1 <-----FR2-----> CDR2 <--------------

EIVMTQSPPTLSLSPGERVTLSC**RASEDIYNRLV**WYQQKPGQAPRLLIS**GVTSLET**SIPARFSGSGSGKDY

FR3-------------> CDR3 <--FR4--->

TLTISSLQPEDFAVYYC**QQYWSTPYT**FGQGTKVEIK

Nucleotide sequence

GAGATCGTGATGACACAGAGCCCTCCAACACTGTCTCTGAGCCCTGGCGAGAGAGTGACCCTGAGCT
GTAGAGCCAGCGAGGACATCTACAACAGACTCGTGTGGTATCAGCAGAAGCCCGGCCAGGCTCCTA
GACTGCTGATTAGCGGAGTGACCAGCCTGGAAACTAGCATCCCCGCCAGATTCAGCGGCTCTGGCTC
TGGCAAGGACTACACCCTGACAATCAGCAGCCTGCAGCCTGAGGACTTCGCCGTGTACTACTGCCAG
CAGTACTGGTCTACCCCTTACACCTTCGGCCAGGGCACCAAGGTGGAAATCAAG

VK v4:

<---------FR1---------> CDR1 <-----FR2-----> CDR2 <--------------

EIVMTQSPGTLSLSPGERATLSC**RASEDIYNRLV**WYQQKPGQAPRLLIS**GVTSLET**GIPDRFSGSGSGKDY

FR3-------------> CDR3 <--FR4--->

TLTISRLEPEDFAVYYC**QQYWSTPYT**FGQGTKVEIK

Nucleotide sequence

GAGATCGTGATGACACAGAGCCCCGGCACACTGTCACTGTCTCCAGGCGAAAGAGCCACACTGAGCT
GTAGAGCCAGCGAGGACATCTACAACAGACTCGTGTGGTATCAGCAGAAGCCCGGCCAGGCTCCTA
GACTGCTGATTAGCGGAGTGACCAGCCTGGAAACAGGCATCCCCGACAGATTCAGCGGCTCTGGCTC
TGGCAAGGACTACACCCTGACCATCAGCAGACTGGAACCCGAGGACTTCGCCGTGTACTACTGCCAG
CAGTACTGGTCTACCCCTTACACCTTCGGCCAGGGCACCAAGGTGGAAATCAAG

VK v5:

<----------FR1--------> CDR1 <-----FR2-----> CDR2 <--------------

DIVMTQSPDSLAVSLGERATINC**KASEDIYNRLV**WYQQKPGQPPKLLIS**GVTSLET**GVPDRFSGSGSGKD
Y

FR3-------------> CDR3 <--FR4--->

TLTISSLQAEDVAVYYC**QQYWSTPYT**FGQGTKVEIK

Nucleotide sequence

GACATCGTGATGACACAGAGCCCTGATAGCCTGGCCGTGTCTCTGGGAGAGAGAGCCACCATCAACT
GCAAGGCCAGCGAGGACATCTACAACAGACTCGTGTGGTATCAGCAGAAGCCCGGCCAGCCTCCTA
AGCTGCTGATTAGCGGAGTGACCAGCCTGGAAACAGGCGTGCCAGACAGATTCAGCGGCTCTGGCTC
TGGCAAGGACTACACCCTGACAATCAGCTCCCTGCAGGCCGAGGATGTGGCCGTGTACTACTGCCAG
CAGTACTGGTCTACCCCTTACACCTTCGGCCAGGGCACCAAGGTGGAAATCAAG

VK v7:

<---------FR1---------> CDR1 <-----FR2-----> CDR2 <-------------

EIVMTQSPPTLSLSPGERVTLSC**RASEDIYNRLV**WYQQKPGQAPRLLIS**GVTSLET**SIPARFSGSGSGTDY

FR3-------------> CDR3 <--FR4--->

TLTISSLQPEDFAVYYC**QQYWSTPYT**FGQGTKVEIK

Nucleotide sequence

GAGATCGTGATGACACAGAGCCCTCCAACACTGTCTCTGAGCCCTGGCGAGAGAGTGACCCTGAGCT

GTAGAGCCAGCGAGGACATCTACAACAGACTCGTGTGGTATCAGCAGAAGCCCGGCCAGGCTCCTA

GACTGCTGATTAGCGGAGTGACCAGCCTGGAAACTAGCATCCCCGCCAGATTCAGCGGCTCTGGCTC

TGGCacaGACTACACCCTGACAATCAGCAGCCTGCAGCCTGAGGACTTCGCCGTGTACTACTGCCAGC

AGTACTGGTCTACCCCTTACACCTTCGGCCAGGGCACCAAGGTGGAAATCAAG

VK v8:

<---------FR1---------> CDR1 <-----FR2-----> CDR2 <--------------

EIVMTQSPPTLSLSPGERVTLSC**RASEDIYNYLV**WYQQKPGQAPRLLIS**GVTSLET**SIPARFSGSGSGTDY

FR3-------------> CDR3 <--FR4--->

TLTISSLQPEDFAVYYC**QQYWSTPYT**FGQGTKVEIK

Nucleotide sequence

GAGATCGTGATGACACAGAGCCCTCCAACACTGTCTCTGAGCCCTGGCGAGAGAGTGACCCTGAGCT

GTAGAGCCAGCGAGGACATCTACAACtacCTCGTGTGGTATCAGCAGAAGCCCGGCCAGGCTCCTAGA

CTGCTGATTAGCGGAGTGACCAGCCTGGAAACTAGCATCCCCGCCAGATTCAGCGGCTCTGGCTCTG

GCacaGACTACACCCTGACAATCAGCAGCCTGCAGCCTGAGGACTTCGCCGTGTACTACTGCCAGCAG

TACTGGTCTACCCCTTACACCTTCGGCCAGGGCACCAAGGTGGAAATCAAG

**Example 6. CDC killing of different humanized CD38 antibodies on tumor cells Daudi**

Different cells were seeded into a U-shaped 96-well plate, antibodies of different concentrations were added into each well, and the plate was placed in a $CO_2$ incubator for half an hour; 50 μl of complement mix was then added to each well and incubated in a $CO_2$ incubator for 2 hours. Then, 50 μl CCK-8 solution was added into each well, followed by shaking and blending, and culturing for another 3 hours; the light absorption OD450 was selected for plate reading. The results were shown in FIG.s 7-1 and 7-2. Among different humanized CD38 antibodies, humanized variants Ab031 and Ab033 have the highest maximum killing rate on Dauai cells.

Table 2: CDC killing of different humanized CD38 antibodies on tumor cells Daudi

| Humanized variants | VH | VK | CDC |
|---|---|---|---|
| Ab001 | h48H1-VHv1 | h48H1-VKv1 | + |
| Ab002 | h48H1-VHv1 | h48H1-VKv2 | + |
| Ab003 | h48H1-VHv1 | h48H1-VKv3 | + |
| Ab004 | h48H1-VHv1 | h48H1-VKv4 | + |
| Ab005 | h48H1-VHv1 | h48H1-VKv5 | + |
| Ab006 | h48H1-VHv2 | h48H1-VKv1 | + |
| Ab007 | h48H1-VHv2 | h48H1-VKv2 | + |
| Ab008 | h48H1-VHv2 | h48H1-VKv3 | ++ |
| Ab009 | h48H1-VHv2 | h48H1-VKv4 | + |
| Ab010 | h48H1-VHv2 | h48H1-VKv5 | + |
| Ab011 | h48H1-VHv3 | h48H1-VKv1 | + |
| Ab012 | h48H1-VHv3 | h48H1-VKv2 | + |

(continued)

| Humanized variants | VH | VK | CDC |
|---|---|---|---|
| Ab013 | h48H1-VHv3 | h48H1-VKv3 | + |
| Ab014 | h48H1-VHv3 | h48H1-VKv4 | + |
| Ab015 | h48H1-VHv3 | h48H1-VKv5 | + |
| Ab016 | h48H1-VHv4 | h48H1-VKv1 | ++ |
| Ab017 | h48H1-VHv4 | h48H1-VKv2 | + |
| Ab018 | h48H1-VHv4 | h48H1-VKv3 | ++ |
| Ab019 | h48H1-VHv4 | h48H1-VKv4 | ++ |
| Ab020 | h48H1-VHv4 | h48H1-VKv5 | ++ |
| Ab021 | h48H1-VHv5 | h48H1-VKv1 | + |
| Ab022 | h48H1-VHv5 | h48H1-VKv2 | + |
| Ab023 | h48H1-VHv5 | h48H1-VKv3 | + |
| Ab024 | h48H1-VHv5 | h48H1-VKv4 | + |
| Ab025 | h48H1-VHv5 | h48H1-VKv5 | + |
| Ab026 | h48H1-VHv6 | h48H1-VKv1 | + |
| Ab027 | h48H1-VHv6 | h48H1-VKv2 | + |
| Ab028 | h48H1-VHv6 | h48H1-VKv3 | + |
| Ab029 | h48H1-VHv6 | h48H1-VKv4 | + |
| Ab030 | h48H1-VHv6 | h48H1-VKv5 | + |
| Ab031 | h48H1-VHv7 | h48H1-VKv7 | ++ |
| Ab032 | h48H1-VHv7 | h48H1-VKv8 | - |
| Ab033 | h48H1-VHv8 | h48H1-VKv7 | ++ |
| Ab034 | h48H1-VHv8 | h48H1-VKv8 | - |
| Ab035 | h48H1-VHv9 | h48H1-VKv7 | + |
| Ab036 | h48H1-VHv9 | h48H1-VKv8 | - |
| Ab037 | h48H1-VHv10 | h48H1-VKv7 | - |
| Ab038 | h48H1-VHv10 | h48H1-VKv8 | - |
| Ab039 | h48H1-VHv11 | h48H1-VKv7 | + |
| Ab040 | h48H1-VHv11 | h48H1-VKv8 | - |

**Example 7 Determination of affinity for humanized CD38 antibodies**

1) Affinity Assay by ELISA

A 96-well plate was coated with human CD38 protein and incubated overnight at 4°C. The solution in wells were then discarded, washed 3 times with wash buffer, and blocked by the addition of 2% milk in PBS for 60 minutes. After washing 3 times with washing buffer, 100 μL of antibodies at different dilutions were added to each well, and the mixture was incubated at 37°C for 1 hour; after washing 3 times with washing buffer, HPR-labeled goat anti-human IgG Fc was diluted at 1:5000 with 0.5% BSA, and the mixture was incubated at room temperature for 1 hour; after washing 3 times with washing buffer, 100 μL of TMB substrate solution was added for color development; after reaction at 37°C for 10 minutes, the reaction was terminated with 50 μL of 2M sulfuric acid solution and the absorbance was read at 450 nm. The results were shown in FIG. 8-1 and Table 3, humanized CD38 antibodies Ab031 (EC50=0.51 nM) and Ab033 (EC50=4.24 nM) bound to CD38 with high affinity according to ELISA.

Table 3: Affinity Assay by ELISA

| Antibody | EC50 (nm) |
|---|---|
| Ab031 | 0.51 |
| Ab033 | 4.24 |

2) Affinity Assay by Biacore

The humanized variant of 0.5 μg/ml was captured on the surface of protein A chip with a 1:1 monovalent binding mode, the signal was captured at about 200 RU, human CD38 recombinant protein at different concentrations flowed through the chip surface as an analyte, and the change of SPR signal during the process of antibody binding and dissociation was recorded and fitted with Langmuir 1:1 kinetics theoretical model to analyze the goodness of fit with the model and provide affinity data. As shown in Table 4, the humanized antibody Ab031 has an affinity of 0.28 nM and Ab033 has an affinity of 1.43 nM according to Biacore assay.

Table 4: Affinity Assay by Biacore

| Antibody | Ka(1/Ms) | Kd(1/s) | KD(M) |
|---|---|---|---|
| Ab031 | 2.00E+06 | 5.56E-04 | 2.78E-10 |
| Ab033 | 3.71E+06 | 5.32E-03 | 1.43E-09 |

3) Cell Affinity Assay by FACS

Different cells in logarithmic phase were blocked with 3% BSA for 30 minutes, and then plated into a U-shaped 96-well plate at 5×10$^4$ cells/100 μL; centrifugation was conducted at 1100 rpm for 3 minutes, the supernatant was discarded, the cells were patted and loosed gently, 50 μL of serially diluted antibody (diluted 3-fold in 5 series, with an initial concentration of 30 μg/mL) was added to each well, and the mixture was incubated at 4°C for 1 hour. After the incubation, 180 μL 0.5% BSA was added to each well, wash 3 times, 30 μL/well of secondary antibody Alexa Fluro 647 anti-human IgG (Jackson ImmunoResearch, Cat: 109-606-170) was added, and the mixture was incubated at 4°C for 45 minutes. After the incubation, each well was washed 3 times with 180 μL 0.5% BSA, and finally resuspended in 50 μL PBS each well for iQue (Intellicyt, USA) assay. As shown in FIG. 8-2 and Table 5, according to FACS, the humanized antibodies Ab031 and Ab033 bind to CD38 molecules expressed on Daudi with a high affinity; meanwhile, the humanized variants Ab031 and Ab033 also recognize CD38 molecules expressed on other tumor cells NALM-6, Ramos.2G6.4C10, and TALL-1 (FIGs. 8-3, 8-4, and 8-5).

Table 5: Cell Affinity Assay by FACS

| Cell | Antibody $EC_{50}$ (nM) | |
|---|---|---|
| | Ab031 | Ab033 |
| Daudi | 0.44 | 0.54 |
| NALM-6 | 0.37 | 0.49 |
| Ramos.2G6.4C10 | 0.42 | 0.58 |
| TALL-1 | 0.41 | 0.34 |

**Example 8. Expression of CD38 on different tumor cells**

The expression of CD38 on different tumor cells was detected by FACS analysis. 1*10$^6$ different tumor cells were centrifugated and collected, then washed once with 3 mL PBS; after blocking for 20 minutes, 50 μl of diluted primary antibody solution anti-human CD38 hIgG1-biotin was added and incubated on ice for 40 minutes; after washing twice, and 100 μl diluted secondary antibody solution SA-PE (Jackson, Cat. No: 016110084) was added, the mixture was incubated on ice for 30 minutes in the dark, washed twice, and centrifuged to collect cells; 200 μl of PI diluent was added to each well, the mixture was pipetted gently and mixed well, 500 μl of 0.5% BSA/PBS solution was added into a standard BD Qusntibrite PE tube (BD, Cat. No: 340495) in the dark, followed by incubation on ice and performing flow cytometry. As shown in FIG. 9, high expression of CD38 was detected on Daudi and Ramos.2G6.4C10 (ATCC CRL-1923) cells; medium expression of CD38 was detected on RPMI-8226 (ATCC CRM-CCL-155), TALL-1 (Shanghai Sixin Biotechnology Co., Ltd.), and Raji (National Institutes for Food and Drug Control) cells; low expression of CD38 was detected on NCI-H929 (Nanjing Cobioer Biosciences), CCRF-CEM, and NALM-6 (Shanghai Zishi Biotechnology Co., Ltd.) cells; and weak expression of CD38 was detected on MM.1S (ATCC CRL-2974), MOLP-8, and WSU-DLCL-2 (Shanghai Sixin Biotechnology Co., Ltd.) cells.

**Example 9. CDC killing of humanized CD38 antibodies on different tumor cells**

Different cells were seeded into a U-shaped 96-well plate, antibodies of different concentrations were added into each well, and the plate was placed in a $CO_2$ incubator for half an hour; 50 $\mu$l of complement mix was then added to each well and incubated in a $CO_2$ incubator for 2 hours. Then, 50 $\mu$l CCK-8 solution was added into each well, followed by shaking and blending, and culturing for another 3 hours; the light absorption OD450 was selected for plate reading. As shown in FIGs. 10-1, 10-2, and 10-3, the humanized antibodies Ab031 and Ab033 had a maximum killing rate of 98% for Ramos.2G6.4C10 cells, 65% for Daudi cells, and 40% for TALL-1 cells.

**Example 10. ADCC killing of humanized CD38 antibodies on different tumor cells**

Different cells were seeded into a U-shaped 96-well plate, antibodies of different concentrations were added into each well, and the plate was placed in a $CO_2$ incubator for half an hour; $5\times10^5$ PBMC cells were then added to each well and incubated in a $CO_2$ incubator for 4 hours. 30 minutes before detection, cell lysate was added and the mixture was cultured, followed by centrifuging to collect the supernatant into a black ELISA plate, a LDH detection substrate was added, followed by shaking and evenly blending. After 10 minutes, the stop solution was added to terminate the reaction, followed by shaking and evenly blending; fluorescence was selected to read the plate, and killing rate was calculated. The calculation equation for killing rate was as follows:

$$\text{Killing rate }\% = \frac{\text{Experimental wells} - \text{Control wells}}{\text{Maximum release wells} - \text{Control wells}} \times 100\%$$

As shown in FIGs. 11-1, 11-2, 11-3, 11-4, 11-5, 11-6, 11-7, and 11-8, the humanized antibodies Ab031 and Ab033 had a killing rate of 90% for Ramos.2G6.4C10 cells, 65% for NALM-6 cells, 67% for RPMI-8226 cells, and 48% for CRFF-CEM cells; furthermore, 62% for Daudi cells, 40% for TALL-1 cells, 20% for MM.1S cells, 48% for NCI-H929 cells, and 75% for Raji cells.

**Example 11. ADCP killing of humanized CD38 antibodies on different tumor cells**

1 $\mu$M CFSE labeled Daudi cells as $CD38^+$ target cells were seeded into a 96-well plate, the serially diluted antibody was added, the mixture was incubated in $CO_2$ incubator, and finally GM-CSF1 was added to induce mature macrophages in a proportion of macrophages : Daudi = 1:4, and the mixture was incubated in a $CO_2$ incubator for 4 hour; the diluted solution of anti-human CD14 APC antibody was added, followed by standing on ice in the dark for 20 minutes; the collect cells were detected with a flow cytometer, single positive cell population of CD 14 was gated in macrophage alone wells as control, single positive cell population of CD14 and double positive cell population of CD14 and CSFE were gated in experimental wells, and the number of cells in two groups was counted. As shown in FIGs. 12-1, 12-2, 12-3, 12-4s and 12-5, the humanized CD38 antibodies Ab031 and Ab033 had varying degrees of ADCP phagocytosis on CD38-expressing cells Daudi, TALL-1, Ramos.2G6.4C10, Raji, and NALM-6.

**Example 12. Inhibition of humanized CD38 antibodies on CD38 enzymatic activity**

CD38 recombinant protein was mixed with the serially diluted humanized CD38 antibodies Ab031 and Ab0337, and the mixture was incubated at room temperature for 15 minutes; NGD substrate was added at different time points, and the mixture was evenly blended for detection; as shown in FIG. 13, both of the chimeric antibody 48H1-B11 and humanized CD38 antibodies Ab031 and Ab033 had significant inhibitory effect on CD38 enzyme activity.

**Example 13. Apoptosis induced by humanized CD38 antibodies**

$2*10^5$ Daudi cells were seeded in a 24-well plate and cultured at 37°C overnight; 100 $\mu$l of the diluted and mixed humanized antibodies were added into each well, and the plate was placed in a $CO_2$ incubator to culture; 24 hours or 48 hours after cells were induced and stimulated, the cells were collected, 100 $\mu$l Annexin V-FITC incubation buffer was added, the mixture was blended gently, then propidium iodide staining solution was added, and the mixture was blended gently. The mixture was incubated in the dark for 15 minutes at room temperature, and flow cytometry analysis indicated that early and late apoptotic cells stained with Annexin V-FITC showed green fluorescence (positive); late apoptotic or dead cells stained with PI showed red fluorescence. As shown in FIG. 14, both of the humanized CD38 antibodies Ab031 and Ab033 could detect the apoptosis of Daudi cells.

**Example 14. Lysis of human erythrocytes induced by humanized CD38 antibodies**

Human red blood cells were plated in a 96-well plate, 50 μl of serially diluted antibodies with different concentrations (10 μg/mL and 1 μg/mL) were added, then 50 μl of serially diluted complement was added, the mixture was placed in a $CO_2$ incubator for cultivation for 2 hours with inactivated complement as control, then the state of cells under microscope was observed. As shown in FIG. 15, neither the humanized CD38 antibodies Ab031 nor Ab033 caused lysis of erythrocytes in control and experimental groups.

**Example 15. Anti-tumor effect of humanized CD38 antibodies in Daudi cell line subcutaneous xenograft NOD/SCID mouse model**

Raji cells were cultured in a RPMI 1640 medium containing 10% fetal bovine serum. Raji cells in logarithmic phase were collected, resuspended in PBS to the appropriate concentration and mixed with matrigel at 1: 1 for subcutaneous tumor inoculation in NOD/SCID mice. Female mice were inoculated subcutaneously on the right side with $1\times10^7$ Raji cells, randomly grouped according to tumor size when the mean tumor volume was 100 $mm^3$. Intravenous administration in mice were performed via the tail vein twice weekly at a dose of 10 mg/kg for two weeks in each group of 6 mice, and tumor volume was monitored in the treated groups. Tumor volume was calculated as: tumor volume ($mm^3$) = $1/2\times(a\times b^2)$ (where a represents long diameter and b represents short diameter).

As shown in FIG. 16, all of the CD38 antibodies (3/10 mg/kg, IP, DO, 3) significantly inhibited the production of subcutaneous xenograft tumors in mice with human B-cell lymphoma Daudi, resulting in partial regression of tumor, wherein CM313-1 was humanized antibody Ab031, CM313-2 was humanized antibody Ab033, CM313-3 was antibody -005 (US 20150231235A1), and CM313-4 was 38SB19 (US 20110293606A1).

## Claims

1. An antibody or antigen-binding portion thereof that binds to CD38, comprising heavy chain CDRs selected from the amino acid sequences of SEQ ID NOs: 2-4, 12-14, 22-24, 32-34, 42-44, 52-54, 62-64, 72-74, 82-84, 92-94, 102-144, 112-114, 122-124, 132-34, 142-144, 152-154, 162-164, 172-174, 182-184, 187-189, 192-194, 197-199, 202-204, 207-209, 212-214, 217-219, 222-224, 227-229, 232-234, or any variant thereof, and/or light chain CDRs selected from the amino acid sequences of SEQ ID NOs: 7-9, 17-19, 27-29, 37-39, 47-49, 57-59, 67-69, 77-79, 87-89, 97-99, 107-109, 117-119, 127-129, 137-139, 147-149, 157-159, 167-169, 177-179, 237-239, 242-244, 247-249, 252-254, 257-259, 262-264, 267-269, or any variant thereof.

2. The antibody or antigen-binding portion thereof according to claim 1, comprising a heavy chain CDR1 selected from the amino acid sequences of SEQ ID NOs: 2, 12, 22, 32, 42, 52, 62, 72, 82, 92, 102, 112, 122, 132, 142, 152, 162, 172, 182, 187, 192, 197, 202, 207, 212, 217, 222, 227, 232, or any variant thereof, a heavy chain CDR2 selected from the amino acid sequences of SEQ ID NOs: 3, 13, 23, 33, 43, 53, 63, 73, 83, 93, 103, 113, 123, 133, 143, 153, 163, 173, 183, 188, 193, 198, 203, 208, 213, 218, 223, 228, 233, or any variant thereof, and a heavy chain CDR3 selected from the amino acid sequences of SEQ ID NOs: 4, 14, 24, 34, 44, 54, 64, 74, 84, 94, 104, 114, 124, 134, 144, 154, 164, 174, 184, 189, 194, 199, 204, 209, 214, 219, 224, 229, 234, or any variant thereof; and/or a light chain CDR1 selected from the amino acid sequences of SEQ ID NOs: 7, 17, 27, 37, 47, 57, 67, 77, 87, 97, 107, 117, 127, 137, 147, 157, 167, 177, 237, 242, 247, 252, 257, 262, 267, or any variant thereof, a light chain CDR2 selected from the amino acid sequences of SEQ ID NOs: 8, 18, 28, 38, 48, 58, 68, 78, 88, 98, 108, 118, 128, 138, 148, 158, 168, 178, 238, 243, 248, 253, 258, 263, 268, or any variant thereof, and a light chain CDR3 selected from the amino acid sequences of SEQ ID NOs: 9, 19, 29, 39, 49, 59, 69, 79, 89, 99, 109, 119, 129, 139, 149, 159, 169, 179, 239, 244, 249, 254, 259, 264, 269, or any variant thereof.

**3.** The antibody or antigen-binding portion thereof according to any one of preceding claims, comprising a CDR combination of heavy and light chains selected from the group consisting of:

(1) the heavy chain CDR1, CDR2, and CDR3 sequences including SEQ ID NOs: 2-4, respectively, and/or the light chain CDR1, CDR2, and CDR3 sequences including SEQ ID NOs: 7-9, respectively;
(2) the heavy chain CDR1, CDR2, and CDR3 sequences including SEQ ID NOs: 12-14, respectively, and/or the light chain CDR1, CDR2, and CDR3 sequences including SEQ ID NOs: 17-19, respectively;
(3) the heavy chain CDR1, CDR2, and CDR3 sequences including SEQ ID NOs: 22-24, respectively, and/or the light chain CDR1, CDR2, and CDR3 sequences including SEQ ID NOs: 27-29, respectively;
(4) the heavy chain CDR1, CDR2, and CDR3 sequences including SEQ ID NOs: 32-34, respectively, and/or the light chain CDR1, CDR2, and CDR3 sequences including SEQ ID NOs: 37-39, respectively;
(5) the heavy chain CDR1, CDR2, and CDR3 sequences including SEQ ID NOs: 42-44, respectively, and/or the light chain CDR1, CDR2, and CDR3 sequences including SEQ ID NOs: 47-49, respectively;
(6) the heavy chain CDR1, CDR2, and CDR3 sequences including SEQ ID NOs: 52-54, respectively, and/or the light chain CDR1, CDR2, and CDR3 sequences including SEQ ID NOs: 57-59, respectively;
(7) the heavy chain CDR1, CDR2, and CDR3 sequences including SEQ ID NOs: 62-64, respectively, and/or the light chain CDR1, CDR2, and CDR3 sequences including SEQ ID NOs: 67-69, respectively;
(8) the heavy chain CDR1, CDR2, and CDR3 sequences including SEQ ID NOs: 72-74, respectively, and/or the light chain CDR1, CDR2, and CDR3 sequences including SEQ ID NOs: 77-79, respectively;
(9) the heavy chain CDR1, CDR2, and CDR3 sequences including SEQ ID NOs: 82-84, respectively, and/or the light chain CDR1, CDR2, and CDR3 sequences including SEQ ID NOs: 87-89, respectively;
(10) the heavy chain CDR1, CDR2, and CDR3 sequences including SEQ ID NOs: 92-94, respectively, and/or the light chain CDR1, CDR2, and CDR3 sequences including SEQ ID NOs: 97-99, respectively;
(11) the heavy chain CDR1, CDR2, and CDR3 sequences including SEQ ID NOs: 102-104, respectively, and/or the light chain CDR1, CDR2, and CDR3 sequences including SEQ ID NOs: 107-109, respectively;
(12) the heavy chain CDR1, CDR2, and CDR3 sequences including SEQ ID NOs: 112-114, respectively, and/or the light chain CDR1, CDR2, and CDR3 sequences including SEQ ID NOs: 117-119, respectively;
(13) the heavy chain CDR1, CDR2, and CDR3 sequences including SEQ ID NOs: 122-124, respectively, and/or the light chain CDR1, CDR2, and CDR3 sequences including SEQ ID NOs: 127-129, respectively;
(14) the heavy chain CDR1, CDR2, and CDR3 sequences including SEQ ID NOs: 132-134, respectively, and/or the light chain CDR1, CDR2, and CDR3 sequences including SEQ ID NOs: 137-139, respectively;
(15) the heavy chain CDR1, CDR2, and CDR3 sequences including SEQ ID NOs: 142-144, respectively, and/or the light chain CDR1, CDR2, and CDR3 sequences including SEQ ID NOs: 147-149, respectively;
(16) the heavy chain CDR1, CDR2, and CDR3 sequences including SEQ ID NOs: 152-154, respectively, and/or the light chain CDR1, CDR2, and CDR3 sequences including SEQ ID NOs: 157-159, respectively;
(17) the heavy chain CDR1, CDR2, and CDR3 sequences including SEQ ID NOs: 162-164, respectively, and/or the light chain CDR1, CDR2, and CDR3 sequences including SEQ ID NOs: 167-169, respectively;
(18) the heavy chain CDR1, CDR2, and CDR3 sequences including SEQ ID NOs: 172-174, respectively, and/or the light chain CDR1, CDR2, and CDR3 sequences including SEQ ID NOs: 177-179, respectively;
(19) the heavy chain CDR1, CDR2, and CDR3 sequences including SEQ ID NOs: 182-184, respectively, and/or the light chain CDR1, CDR2, and CDR3 sequences including SEQ ID NOs: 237-239, respectively;
(20) the heavy chain CDR1, CDR2, and CDR3 sequences including SEQ ID NOs: 182-184, respectively, and/or the light chain CDR1, CDR2, and CDR3 sequences including SEQ ID NOs: 242-244, respectively;
(21) the heavy chain CDR1, CDR2, and CDR3 sequences including SEQ ID NOs: 182-184, respectively, and/or the light chain CDR1, CDR2, and CDR3 sequences including SEQ ID NOs: 247-249, respectively;
(22) the heavy chain CDR1, CDR2, and CDR3 sequences including SEQ ID NOs: 182-184, respectively, and/or the light chain CDR1, CDR2, and CDR3 sequences including SEQ ID NOs: 252-254, respectively;
(23) the heavy chain CDR1, CDR2, and CDR3 sequences including SEQ ID NOs: 182-184, respectively, and/or the light chain CDR1, CDR2, and CDR3 sequences including SEQ ID NOs: 257-259, respectively;
(24) the heavy chain CDR1, CDR2, and CDR3 sequences including SEQ ID NOs: 187-189, respectively, and/or the light chain CDR1, CDR2, and CDR3 sequences including SEQ ID NOs: 237-239, respectively;
(25) the heavy chain CDR1, CDR2, and CDR3 sequences including SEQ ID NOs: 187-189, respectively, and/or the light chain CDR1, CDR2, and CDR3 sequences including SEQ ID NOs: 242-244, respectively;
(26) the heavy chain CDR1, CDR2, and CDR3 sequences including SEQ ID NOs: 187-189, respectively, and/or the light chain CDR1, CDR2, and CDR3 sequences including SEQ ID NOs: 247-249, respectively;
(27) the heavy chain CDR1, CDR2, and CDR3 sequences including SEQ ID NOs: 187-189, respectively, and/or the light chain CDR1, CDR2, and CDR3 sequences including SEQ ID NOs: 252-254, respectively;
(28) the heavy chain CDR1, CDR2, and CDR3 sequences including SEQ ID NOs: 187-189, respectively, and/or the light chain CDR1, CDR2, and CDR3 sequences including SEQ ID NOs: 257-259, respectively;
(29) the heavy chain CDR1, CDR2, and CDR3 sequences including SEQ ID NOs: 192-194, respectively, and/or the light chain CDR1, CDR2, and CDR3 sequences including SEQ ID NOs: 237-239, respectively;
(30) the heavy chain CDR1, CDR2, and CDR3 sequences including SEQ ID NOs: 192-194, respectively, and/or the light chain CDR1, CDR2, and CDR3 sequences including SEQ ID NOs: 242-244, respectively;
(31) the heavy chain CDR1, CDR2, and CDR3 sequences including SEQ ID NOs: 192-194, respectively, and/or the light chain CDR1, CDR2, and CDR3 sequences including SEQ ID NOs: 247-249, respectively;
(32) the heavy chain CDR1, CDR2, and CDR3 sequences including SEQ ID NOs: 192-194, respectively, and/or the light chain CDR1, CDR2, and CDR3 sequences including SEQ ID NOs: 252-254, respectively;
(33) the heavy chain CDR1, CDR2, and CDR3 sequences including SEQ ID NOs: 192-194, respectively, and/or the light chain CDR1, CDR2, and CDR3 sequences including SEQ ID NOs: 257-259, respectively;
(34) the heavy chain CDR1, CDR2, and CDR3 sequences including SEQ ID NOs: 197-199, respectively, and/or the light chain CDR1, CDR2, and CDR3 sequences including SEQ ID NOs: 237-239, respectively;
(35) the heavy chain CDR1, CDR2, and CDR3 sequences including SEQ ID NOs: 197-199, respectively, and/or the light chain CDR1, CDR2, and CDR3 sequences including SEQ ID NOs: 242-244, respectively;
(36) the heavy chain CDR1, CDR2, and CDR3 sequences including SEQ ID NOs: 197-199, respectively, and/or the light chain CDR1, CDR2, and CDR3 sequences including SEQ ID NOs: 247-249, respectively;
(37) the heavy chain CDR1, CDR2, and CDR3 sequences including SEQ ID NOs: 197-199, respectively, and/or the light chain CDR1, CDR2, and CDR3 sequences including SEQ ID NOs: 252-254, respectively;
(38) the heavy chain CDR1, CDR2, and CDR3 sequences including SEQ ID NOs: 197-199, respectively, and/or the light chain CDR1, CDR2, and CDR3 sequences including SEQ ID NOs: 257-259, respectively;
(39) the heavy chain CDR1, CDR2, and CDR3 sequences including SEQ ID NOs: 202-204, respectively, and/or the light chain CDR1, CDR2, and CDR3 sequences including SEQ ID NOs: 237-239, respectively;
(40) the heavy chain CDR1, CDR2, and CDR3 sequences including SEQ ID NOs: 202-204, respectively, and/or the light chain CDR1, CDR2, and CDR3 sequences including SEQ ID NOs: 242-244, respectively;
(41) the heavy chain CDR1, CDR2, and CDR3 sequences including SEQ ID NOs: 202-204, respectively, and/or the light chain CDR1, CDR2, and CDR3 sequences including SEQ ID NOs: 247-249, respectively;
(42) the heavy chain CDR1, CDR2, and CDR3 sequences including SEQ ID NOs: 202-204, respectively, and/or the light chain CDR1, CDR2, and CDR3 sequences including SEQ ID NOs: 252-254, respectively;
(43) the heavy chain CDR1, CDR2, and CDR3 sequences including SEQ ID NOs: 202-204, respectively, and/or the light chain CDR1, CDR2, and CDR3 sequences including SEQ ID NOs: 257-259, respectively;
(44) the heavy chain CDR1, CDR2, and CDR3 sequences including SEQ ID NOs: 207-209, respectively, and/or the light chain CDR1, CDR2, and CDR3 sequences including SEQ ID NOs: 237-239, respectively;
(45) the heavy chain CDR1, CDR2, and CDR3 sequences including SEQ ID NOs: 207-209, respectively, and/or the light chain CDR1, CDR2, and CDR3 sequences including SEQ ID NOs: 242-244, respectively;
(46) the heavy chain CDR1, CDR2, and CDR3 sequences including SEQ ID NOs: 207-209, respectively, and/or the light chain CDR1, CDR2, and CDR3 sequences including SEQ ID NOs: 247-249, respectively;
(47) the heavy chain CDR1, CDR2, and CDR3 sequences including SEQ ID NOs: 207-209, respectively, and/or the light chain CDR1, CDR2, and CDR3 sequences including SEQ ID NOs: 252-254, respectively;
(48) the heavy chain CDR1, CDR2, and CDR3 sequences including SEQ ID NOs: 207-209, respectively, and/or the light chain CDR1, CDR2, and CDR3 sequences including SEQ ID NOs: 257-259, respectively;
(49) the heavy chain CDR1, CDR2, and CDR3 sequences including SEQ ID NOs: 212-214, respectively, and/or the light chain CDR1, CDR2, and CDR3 sequences including SEQ ID NOs: 262-264, respectively;
(50) the heavy chain CDR1, CDR2, and CDR3 sequences including SEQ ID NOs: 212-214, respectively, and/or the light chain CDR1, CDR2, and CDR3 sequences including SEQ ID NOs: 267-269, respectively;
(51) the heavy chain CDR1, CDR2, and CDR3 sequences including SEQ ID NOs: 217-219, respectively, and/or the light chain CDR1, CDR2, and CDR3 sequences including SEQ ID NOs: 262-264, respectively;
(52) the heavy chain CDR1, CDR2, and CDR3 sequences including SEQ ID NOs: 217-219, respectively, and/or the light chain CDR1, CDR2, and CDR3 sequences including SEQ ID NOs: 267-269, respectively;
(53) the heavy chain CDR1, CDR2, and CDR3 sequences including SEQ ID NOs: 222-224, respectively, and/or the light chain CDR1, CDR2, and CDR3 sequences including SEQ ID NOs: 262-264, respectively;
(54) the heavy chain CDR1, CDR2, and CDR3 sequences including SEQ ID NOs: 222-224, respectively, and/or the light chain CDR1, CDR2, and CDR3 sequences including SEQ ID NOs: 267-269, respectively;
(55) the heavy chain CDR1, CDR2, and CDR3 sequences including SEQ ID NOs: 227-229, respectively, and/or the light chain CDR1, CDR2, and CDR3 sequences including SEQ ID NOs: 262-264, respectively;
(56) the heavy chain CDR1, CDR2, and CDR3 sequences including SEQ ID NOs: 227-229, respectively, and/or the light chain CDR1, CDR2, and CDR3 sequences including SEQ ID NOs: 267-269, respectively;
(57) the heavy chain CDR1, CDR2, and CDR3 sequences including SEQ ID NOs: 232-234, respectively, and/or the light chain CDR1, CDR2, and CDR3 sequences including SEQ ID NOs: 262-264, respectively;
(58) the heavy chain CDR1, CDR2, and CDR3 sequences including SEQ ID NOs: 232-234, respectively, and/or the light chain CDR1, CDR2, and CDR3 sequences including SEQ ID NOs: 267-269, respectively.

**4.** The antibody or antigen-binding portion thereof according to claim 1 or 2, comprising a heavy chain variable region selected from the amino acid sequences of SEQ ID NOs: 1, 11, 21, 31, 41, 51, 61, 71, 81, 91, 101, 111, 121, 131, 141, 151, 161, 171, 181, 186, 191, 196, 201, 206, 211, 216, 221, 226, 231, or any variant thereof, and/or a light chain variable region selected from the amino acid sequence of SEQ ID NOs: 6, 16, 26, 36, 46, 56, 66, 76, 86, 96, 106, 116, 126, 136, 146, 156, 166, 176, 236, 241, 246, 251, 256, 261, 266, or any variant thereof;

preferably, comprising a heavy chain variable region of the amino acid sequence of SEQ ID NO: 1 or any variant thereof, and a light chain variable region of the amino acid sequence of SEQ ID NO: 6 or any variant thereof;
preferably, comprising a heavy chain variable region of the amino acid sequence of SEQ ID NO: 11 or any variant thereof, and a light chain variable region of the amino acid sequence of SEQ ID NO: 16 or any variant thereof;
preferably, comprising a heavy chain variable region of the amino acid sequence of SEQ ID NO: 21 or any variant thereof, and a light chain variable region of the amino acid sequence of SEQ ID NO: 26 or any variant thereof;
preferably, comprising a heavy chain variable region of the amino acid sequence of SEQ ID NO: 31 or any variant thereof, and a light chain variable region of the amino acid sequence of SEQ ID NO: 36 or any variant thereof;
preferably, comprising a heavy chain variable region of the amino acid sequence of SEQ ID NO: 41 or any variant thereof, and a light chain variable region of the amino acid sequence of SEQ ID NO: 46 or any variant thereof;
preferably, comprising a heavy chain variable region of the amino acid sequence of SEQ ID NO: 51 or any variant thereof, and a light chain variable region of the amino acid sequence of SEQ ID NO: 56 or any variant thereof;
preferably, comprising a heavy chain variable region of the amino acid sequence of SEQ ID NO: 61 or any variant thereof, and a light chain variable region of the amino acid sequence of SEQ ID NO: 66 or any variant thereof;
preferably, comprising a heavy chain variable region of the amino acid sequence of SEQ ID NO: 71 or any variant thereof, and a light chain variable region of the amino acid sequence of SEQ ID NO: 76 or any variant thereof;
preferably, comprising a heavy chain variable region of the amino acid sequence of SEQ ID NO: 81 or any variant thereof, and a light chain variable region of the amino acid sequence of SEQ ID NO: 86 or any variant thereof;
preferably, comprising a heavy chain variable region of the amino acid sequence of SEQ ID NO: 91 or any variant thereof, and a light chain variable region of the amino acid sequence of SEQ ID NO: 96 or any variant thereof;
preferably, comprising a heavy chain variable region of the amino acid sequence of SEQ ID NO: 101 or any variant thereof, and a light chain variable region of the amino acid sequence of SEQ ID NO: 106 or any variant thereof;
preferably, comprising a heavy chain variable region of the amino acid sequence of SEQ ID NO: 111 or any variant thereof, and a light chain variable region of the amino acid sequence of SEQ ID NO: 116 or any variant thereof;
preferably, comprising a heavy chain variable region of the amino acid sequence of SEQ ID NO: 121 or any variant thereof, and a light chain variable region of the amino acid sequence of SEQ ID NO: 126 or any variant thereof;

preferably, comprising a heavy chain variable region of the amino acid sequence of SEQ ID NO: 131 or any variant thereof, and a light chain variable region of the amino acid sequence of SEQ ID NO: 136 or any variant thereof;

preferably, comprising a heavy chain variable region of the amino acid sequence of SEQ ID NO: 141 or any variant thereof, and a light chain variable region of the amino acid sequence of SEQ ID NO: 146 or any variant thereof;

preferably, comprising a heavy chain variable region of the amino acid sequence of SEQ ID NO: 151 or any variant thereof, and a light chain variable region of the amino acid sequence of SEQ ID NO: 156 or any variant thereof;

preferably, comprising a heavy chain variable region of the amino acid sequence of SEQ ID NO: 161 or any variant thereof, and a light chain variable region of the amino acid sequence of SEQ ID NO: 166 or any variant thereof;

preferably, comprising a heavy chain variable region of the amino acid sequence of SEQ ID NO: 171 or any variant thereof, and a light chain variable region of the amino acid sequence of SEQ ID NO: 176 or any variant thereof;

preferably, comprising a heavy chain variable region of the amino acid sequence of SEQ ID NO: 181 or any variant thereof, and a light chain variable region of the amino acid sequence of SEQ ID NO: 236 or any variant thereof;

preferably, comprising a heavy chain variable region of the amino acid sequence of SEQ ID NO: 181 or any variant thereof, and a light chain variable region of the amino acid sequence of SEQ ID NO: 241 or any variant thereof;

preferably, comprising a heavy chain variable region of the amino acid sequence of SEQ ID NO: 181 or any variant thereof, and a light chain variable region of the amino acid sequence of SEQ ID NO: 246 or any variant thereof;

preferably, comprising a heavy chain variable region of the amino acid sequence of SEQ ID NO: 181 or any variant thereof, and a light chain variable region of the amino acid sequence of SEQ ID NO: 251 or any variant thereof;

preferably, comprising a heavy chain variable region of the amino acid sequence of SEQ ID NO: 181 or any variant thereof, and a light chain variable region of the amino acid sequence of SEQ ID NO: 256 or any variant thereof;

preferably, comprising a heavy chain variable region of the amino acid sequence of SEQ ID NO: 186 or any variant thereof, and a light chain variable region of the amino acid sequence of SEQ ID NO: 236 or any variant thereof;

preferably, comprising a heavy chain variable region of the amino acid sequence of SEQ ID NO: 186 or any variant thereof, and a light chain variable region of the amino acid sequence of SEQ ID NO: 241 or any variant thereof;

preferably, comprising a heavy chain variable region of the amino acid sequence of SEQ ID NO: 186 or any variant thereof, and a light chain variable region of the amino acid sequence of SEQ ID NO: 246 or any variant thereof;

preferably, comprising a heavy chain variable region of the amino acid sequence of SEQ ID NO: 186 or any variant thereof, and a light chain variable region of the amino acid sequence of SEQ ID NO: 251 or any variant thereof;

preferably, comprising a heavy chain variable region of the amino acid sequence of SEQ ID NO: 186 or any variant thereof, and a light chain variable region of the amino acid sequence of SEQ ID NO: 256 or any variant thereof;

preferably, comprising a heavy chain variable region of the amino acid sequence of SEQ ID NO: 191 or any variant thereof, and a light chain variable region of the amino acid sequence of SEQ ID NO: 236 or any variant thereof;

preferably, comprising a heavy chain variable region of the amino acid sequence of SEQ ID NO: 191 or any variant thereof, and a light chain variable region of the amino acid sequence of SEQ ID NO: 241 or any variant thereof;

preferably, comprising a heavy chain variable region of the amino acid sequence of SEQ ID NO: 191 or any variant thereof, and a light chain variable region of the amino acid sequence of SEQ ID NO: 246 or any variant thereof;

preferably, comprising a heavy chain variable region of the amino acid sequence of SEQ ID NO: 191 or any variant thereof, and a light chain variable region of the amino acid sequence of SEQ ID NO: 251 or any variant thereof;

preferably, comprising a heavy chain variable region of the amino acid sequence of SEQ ID NO: 191 or any variant thereof, and a light chain variable region of the amino acid sequence of SEQ ID NO: 256 or any variant thereof;
preferably, comprising a heavy chain variable region of the amino acid sequence of SEQ ID NO: 196 or any variant thereof, and a light chain variable region of the amino acid sequence of SEQ ID NO: 236 or any variant thereof;
preferably, comprising a heavy chain variable region of the amino acid sequence of SEQ ID NO: 196 or any variant thereof, and a light chain variable region of the amino acid sequence of SEQ ID NO: 241 or any variant thereof;
preferably, comprising a heavy chain variable region of the amino acid sequence of SEQ ID NO: 196 or any variant thereof, and a light chain variable region of the amino acid sequence of SEQ ID NO: 246 or any variant thereof;
preferably, comprising a heavy chain variable region of the amino acid sequence of SEQ ID NO: 196 or any variant thereof, and a light chain variable region of the amino acid sequence of SEQ ID NO: 251 or any variant thereof;
preferably, comprising a heavy chain variable region of the amino acid sequence of SEQ ID NO: 196 or any variant thereof, and a light chain variable region of the amino acid sequence of SEQ ID NO: 256 or any variant thereof;
preferably, comprising a heavy chain variable region of the amino acid sequence of SEQ ID NO: 201 or any variant thereof, and a light chain variable region of the amino acid sequence of SEQ ID NO: 236 or any variant thereof;
preferably, comprising a heavy chain variable region of the amino acid sequence of SEQ ID NO: 201 or any variant thereof, and a light chain variable region of the amino acid sequence of SEQ ID NO: 241 or any variant thereof;
preferably, comprising a heavy chain variable region of the amino acid sequence of SEQ ID NO: 201 or any variant thereof, and a light chain variable region of the amino acid sequence of SEQ ID NO: 246 or any variant thereof;
preferably, comprising a heavy chain variable region of the amino acid sequence of SEQ ID NO: 201 or any variant thereof, and a light chain variable region of the amino acid sequence of SEQ ID NO: 251 or any variant thereof;
preferably, comprising a heavy chain variable region of the amino acid sequence of SEQ ID NO: 201 or any variant thereof, and a light chain variable region of the amino acid sequence of SEQ ID NO: 256 or any variant thereof;
preferably, comprising a heavy chain variable region of the amino acid sequence of SEQ ID NO: 206 or any variant thereof, and a light chain variable region of the amino acid sequence of SEQ ID NO: 236 or any variant thereof;
preferably, comprising a heavy chain variable region of the amino acid sequence of SEQ ID NO: 206 or any variant thereof, and a light chain variable region of the amino acid sequence of SEQ ID NO: 241 or any variant thereof;
preferably, comprising a heavy chain variable region of the amino acid sequence of SEQ ID NO: 206 or any variant thereof, and a light chain variable region of the amino acid sequence of SEQ ID NO: 246 or any variant thereof;
preferably, comprising a heavy chain variable region of the amino acid sequence of SEQ ID NO: 206 or any variant thereof, and a light chain variable region of the amino acid sequence of SEQ ID NO: 251 or any variant thereof;
preferably, comprising a heavy chain variable region of the amino acid sequence of SEQ ID NO: 206 or any variant thereof, and a light chain variable region of the amino acid sequence of SEQ ID NO: 256 or any variant thereof;
preferably, comprising a heavy chain variable region of the amino acid sequence of SEQ ID NO: 211 or any variant thereof, and a light chain variable region of the amino acid sequence of SEQ ID NO: 261 or any variant thereof;
preferably, comprising a heavy chain variable region of the amino acid sequence of SEQ ID NO: 211 or any variant thereof, and a light chain variable region of the amino acid sequence of SEQ ID NO: 266 or any variant thereof;
preferably, comprising a heavy chain variable region of the amino acid sequence of SEQ ID NO: 216 or any variant thereof, and a light chain variable region of the amino acid sequence of SEQ ID NO: 261 or any variant thereof;
preferably, comprising a heavy chain variable region of the amino acid sequence of SEQ ID NO: 216 or any variant thereof, and a light chain variable region of the amino acid sequence of SEQ ID NO: 266 or any variant thereof;
preferably, comprising a heavy chain variable region of the amino acid sequence of SEQ ID NO: 221 or any variant thereof, and a light chain variable region of the amino acid sequence of SEQ ID NO: 261 or any variant thereof;
preferably, comprising a heavy chain variable region of the amino acid sequence of SEQ ID NO: 221 or any variant thereof, and a light chain variable region of the amino acid sequence of SEQ ID NO: 266 or any variant thereof;
preferably, comprising a heavy chain variable region of the amino acid sequence of SEQ ID NO: 226 or any variant thereof, and a light chain variable region of the amino acid sequence of SEQ ID NO: 261 or any variant thereof;
preferably, comprising a heavy chain variable region of the amino acid sequence of SEQ ID NO: 226 or any variant thereof, and a light chain variable region of the amino acid sequence of SEQ ID NO: 266 or any variant thereof;
preferably, comprising a heavy chain variable region of the amino acid sequence of SEQ ID NO: 231 or any variant thereof, and a light chain variable region of the amino acid sequence of SEQ ID NO: 261 or any variant thereof;
preferably, comprising a heavy chain variable region of the amino acid sequence of SEQ ID NO: 231 or any variant thereof, and a light chain variable region of the amino acid sequence of SEQ ID NO: 266 or any variant thereof.

**5.** A bispecific or multispecific molecule, comprising the antibody or antigen-binding portion thereof according to any one of claims 1 to 4.

**6.** The antibody or antigen-binding portion thereof according to any one of claims 1 to 4 or the bispecific or multispecific molecule according to claim 5, which is humanized.

**7.** A nucleic acid molecule encoding the antibody or antigen-binding portion thereof according to any one of claims 1 to 4 or the bispecific or multispecific molecule according to claim 5; preferably, the nucleic acid molecule comprises the nucleotide sequence of the antibody heavy chain selected from SEQ ID NOs: 5, 15, 25, 35, 45, 55, 65, 75, 85, 95, 105, 115, 125, 135, 145, 155, 165, 175, 185, 190, 195, 200, 205, 210, 215, 220, 225, 230, 235, or any variant thereof, and/or the nucleotide sequence of the antibody light chain selected from SEQ ID NOs: 10, 20, 30, 40, 50, 60, 70, 80, 90, 100, 110, 120, 130, 140, 150, 160, 170, 180, 240, 245, 250, 255, 260, 265, 270, or any variant thereof.

**8.** A vector comprising the nucleic acid according to claim 7.

**9.** A cell containing the nucleic acid according to claim 7 or the vector according to claim 8.

**10.** A composition comprising the antibody or antigen-binding portion thereof according to any one of claims 1 to 4, the bispecific or multispecific molecule according to claim 5, the antibody or antigen-binding portion thereof or the bispecific or multispecific molecule according to claim 6, the nucleic acid according to claim 7, the vector according to claim 8 and/or the cell according to claim 9.

**11.** A kit comprising the antibody or antigen-binding portion thereof according to any one of claims 1 to 4, the bispecific or multispecific molecule according to claim 5, the antibody or antigen-binding portion thereof or the bispecific or multispecific molecule according to claim 6, the nucleic acid according to claim 7, the vector according to claim 8, the cell according to claim 9 and/or the composition according to claim 10.

**12.** Use of the antibody or antigen-binding portion thereof according to any one of claims 1 to 4, the bispecific or multispecific molecule according to claim 5, the antibody or antigen-binding portion thereof or the bispecific or multispecific molecule according to claim 6, the nucleic acid molecule according to claim 7, the vector according to claim 8, the cell according to claim 9 and/or the composition according to claim 10 in the preparation of a medicament or kit for the treatment of a CD38-related disorder, preferably, the CD38-related disorder is selected from tumors and autoimmune diseases; preferably, the tumor is selected from the group consisting of multiple myeloma (MM), NK/T cell lymphoma, immunoglobulin light chain amyloidosis, chronic lymphocytic leukemia (CLL), chronic myelogenous leukemia (CML), T cell acute lymphocytic leukemia, acute myelogenous leukemia (AML) and acute lymphocytic leukemia (ALL); preferably, the autoimmune disease is selected from rheumatoid arthritis (RA) or systemic lupus erythematosus (SLE).

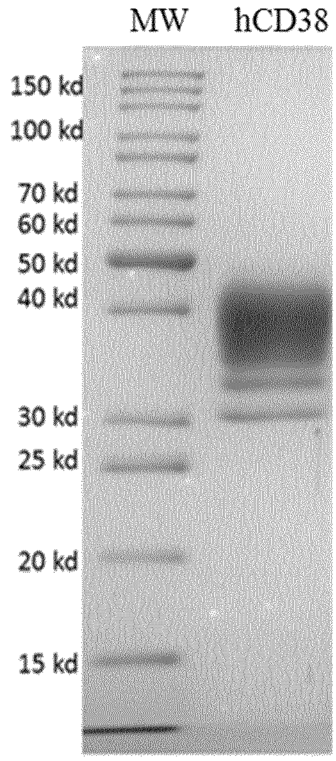
MW
hCD38
150 kd
100 kd
70 kd
60 kd
50 kd
40 kd
30 kd
25 kd
20 kd
15 kd

FIG. 1

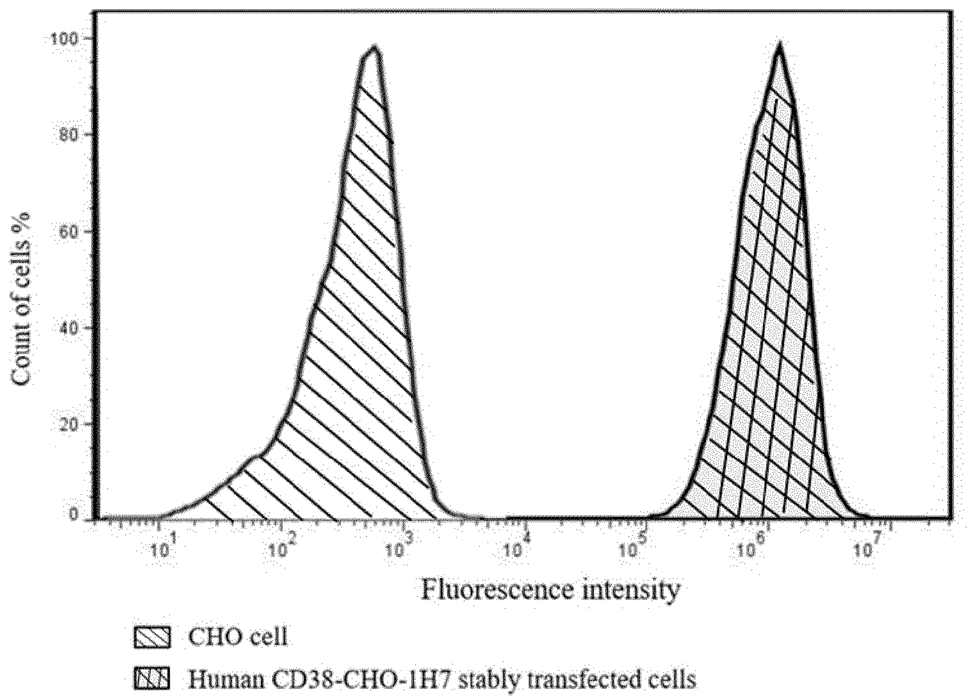
Count of cells %
100
80
60
40
20
0
$10^1$
$10^2$
$10^3$
$10^4$
$10^5$
$10^6$
$10^7$
Fluorescence intensity
CHO cell
Human CD38-CHO-1H7 stably transfected cells

FIG. 2

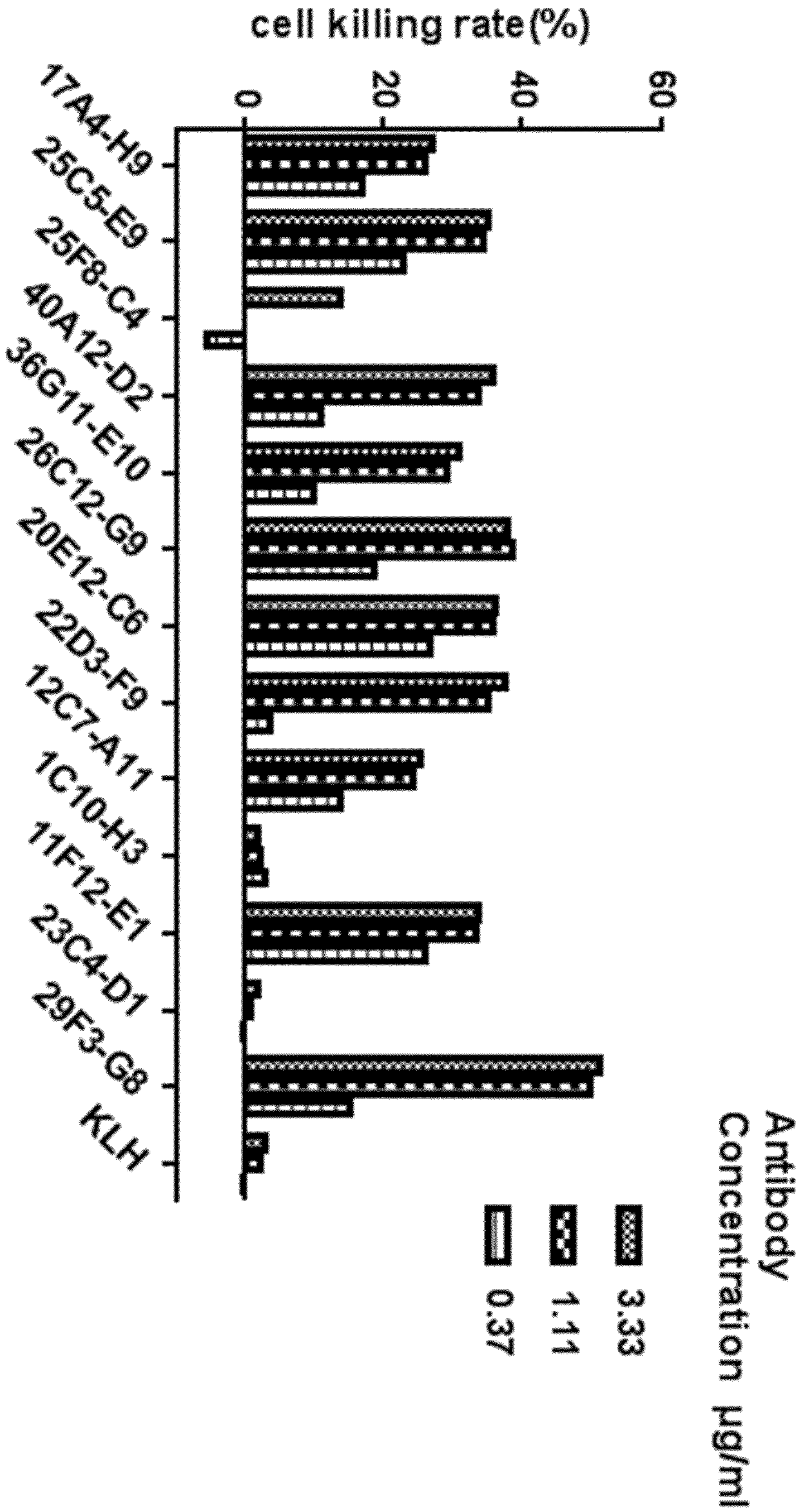
cell killing rate(%)
0
20
40
60
17A4-H9
25C5-E9
25F8-C4
40A12-D2
36G11-E10
26C12-G9
20E12-C6
22D3-F9
12C7-A11
1C10-H3
11F12-E1
23C4-D1
29F3-G8
KLH
Antibody Concentration μg/ml
3.33
1.11
0.37

FIG. 3-1

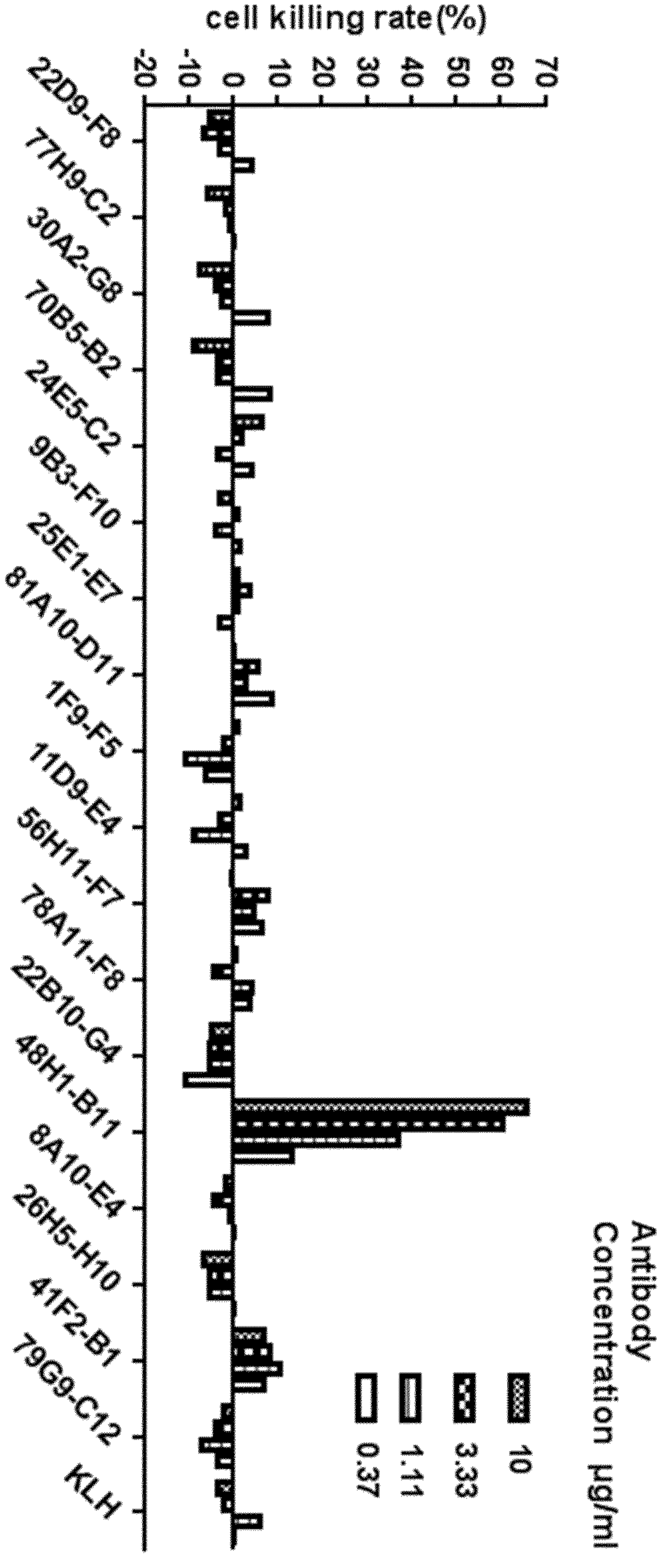
cell killing rate(%)
-20
-10
0
10
20
30
40
50
60
70
22D9-F8
77H9-C2
30A2-G8
70B5-B2
24E5-C2
9B3-F10
25E1-E7
81A10-D11
1F9-F5
11D9-E4
56H11-F7
78A11-F8
22B10-G4
48H1-B11
8A10-E4
26H5-H10
41F2-B1
79G9-C12
KLH
Antibody
Concentration μg/ml
10
3.33
1.11
0.37

FIG. 3-2

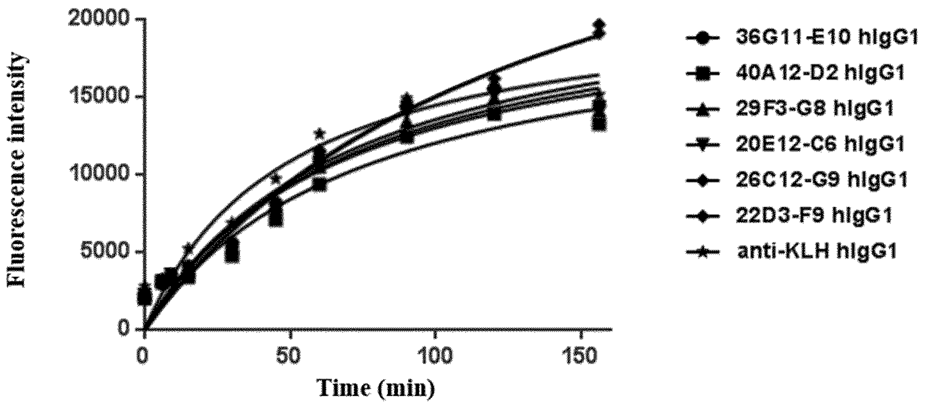


FIG. 4-1

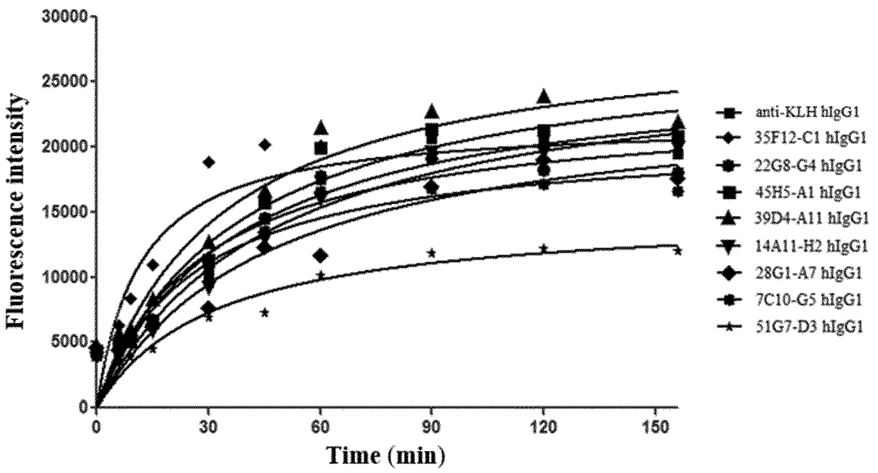


FIG. 4-2

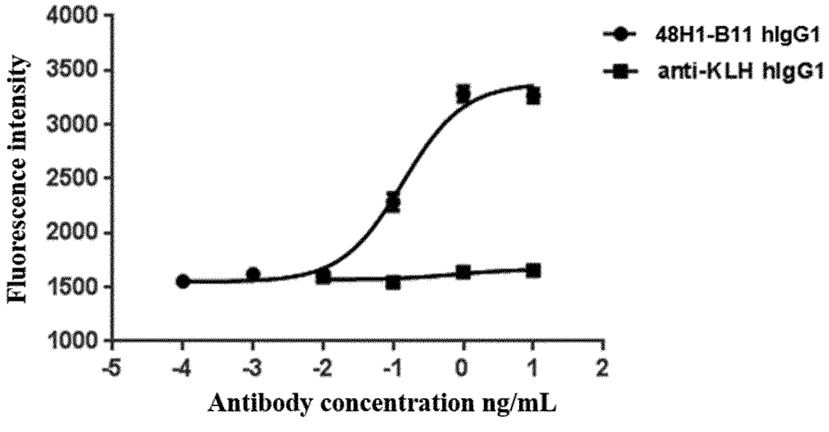


FIG. 5

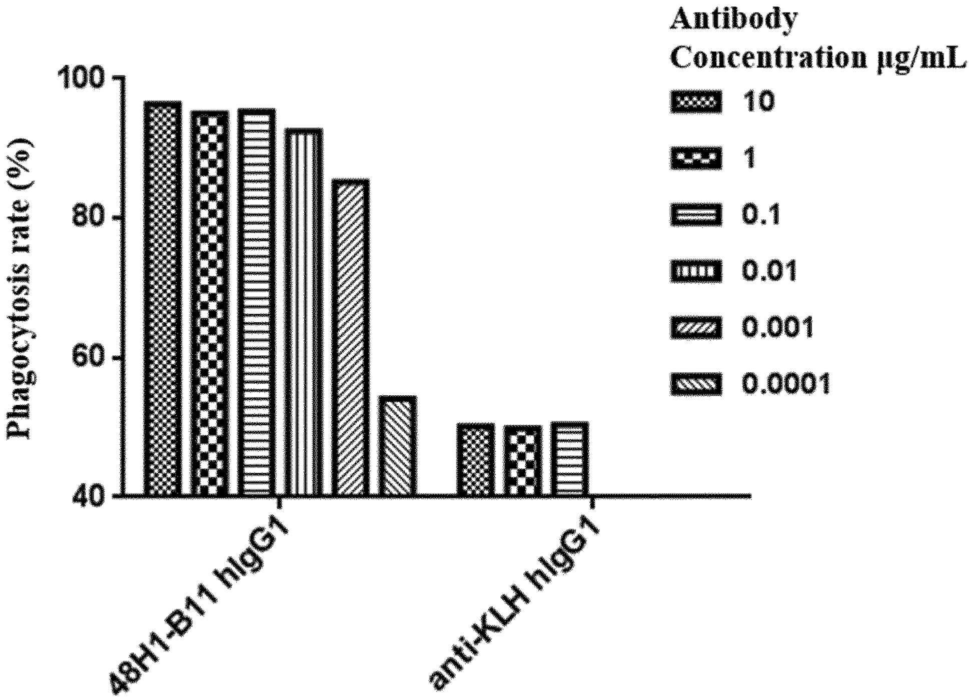
Antibody
Concentration µg/mL
10
1
0.1
0.01
0.001
0.0001
Phagocytosis rate (%)
100
80
60
40
48H1-B11 hIgG1
anti-KLH hIgG1

FIG. 6

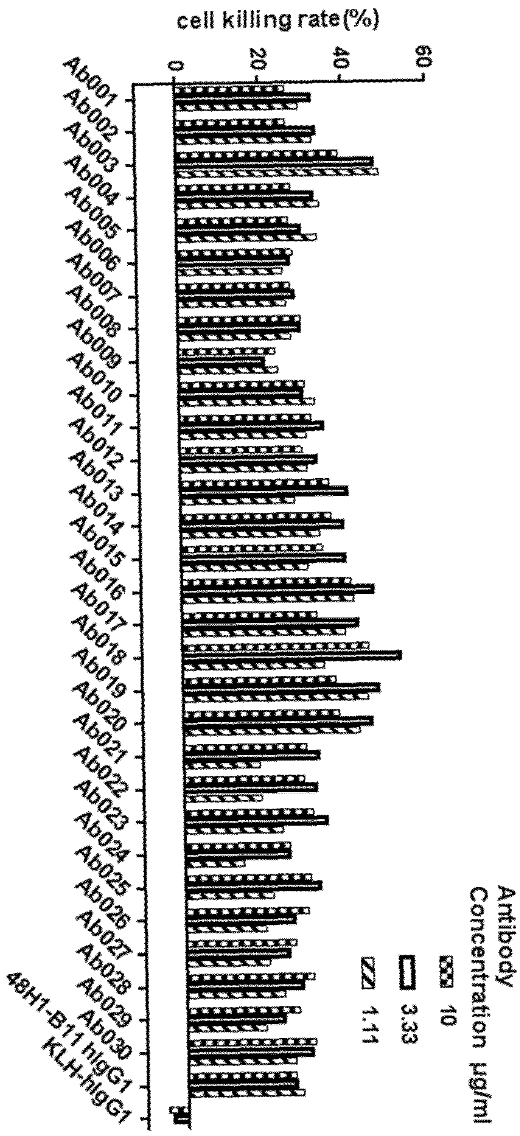
cell killing rate(%)
0
20
40
60
Ab001
Ab002
Ab003
Ab004
Ab005
Ab006
Ab007
Ab008
Ab009
Ab010
Ab011
Ab012
Ab013
Ab014
Ab015
Ab016
Ab017
Ab018
Ab019
Ab020
Ab021
Ab022
Ab023
Ab024
Ab025
Ab026
Ab027
Ab028
Ab029
Ab030
48H1-B11 hIgG1
KLH-hIgG1
Antibody
Concentration μg/ml
10
3.33
1.11

FIG. 7-1

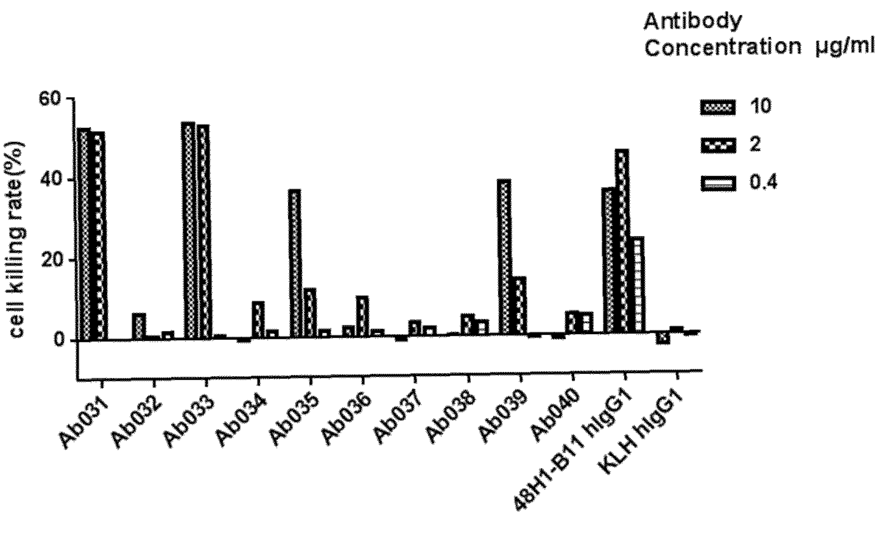
Antibody
Concentration μg/ml
10
2
0.4
60
40
20
0
cell killing rate(%)
Ab031
Ab032
Ab033
Ab034
Ab035
Ab036
Ab037
Ab038
Ab039
Ab040
48H1-B11 hIgG1
KLH hIgG1

FIG. 7-2

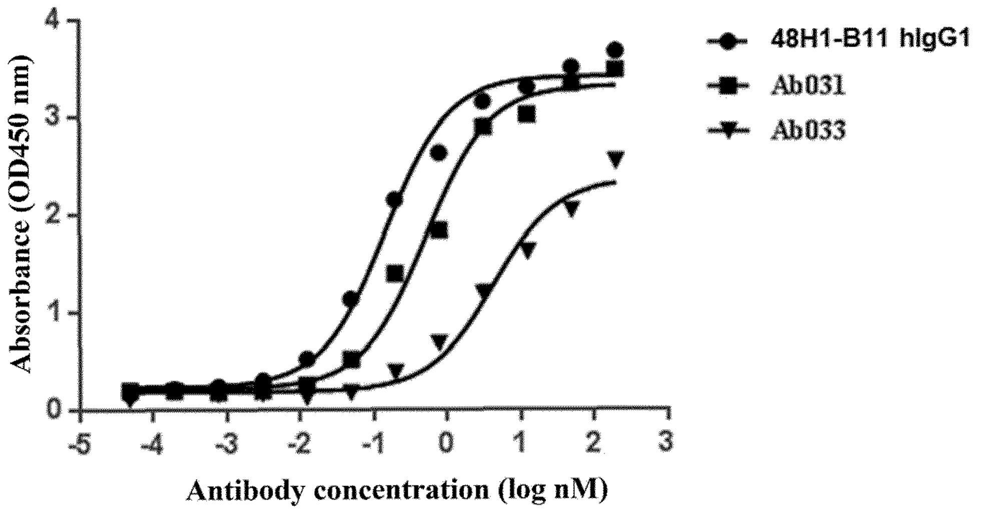


FIG. 8-1

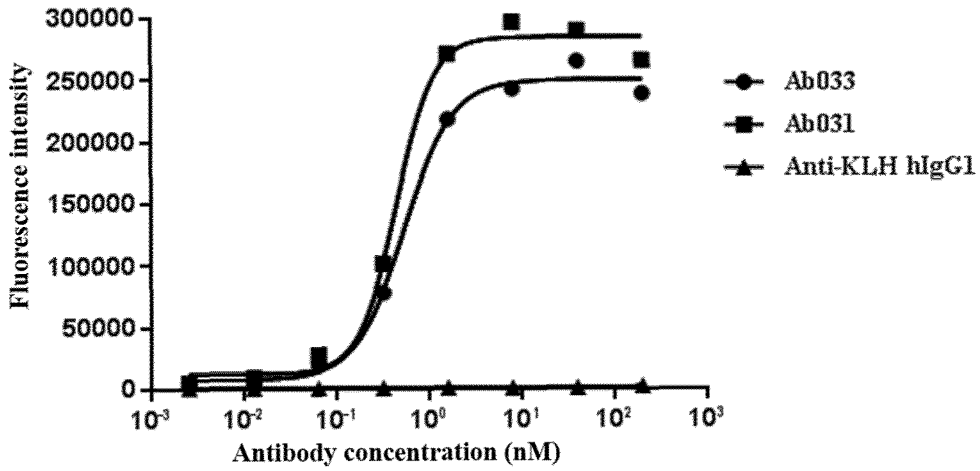


FIG. 8-2

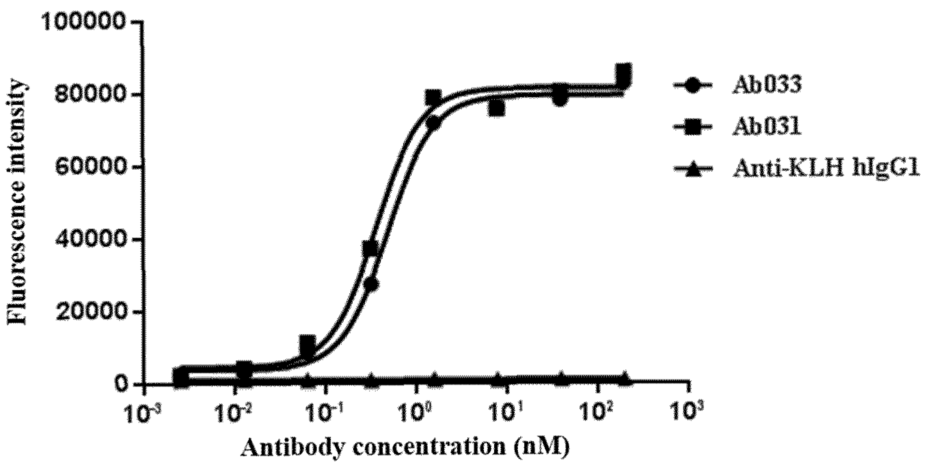


FIG. 8-3

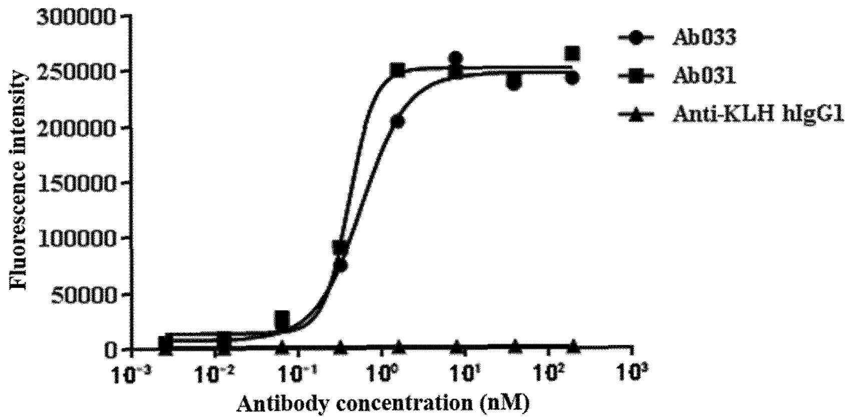


FIG. 8-4

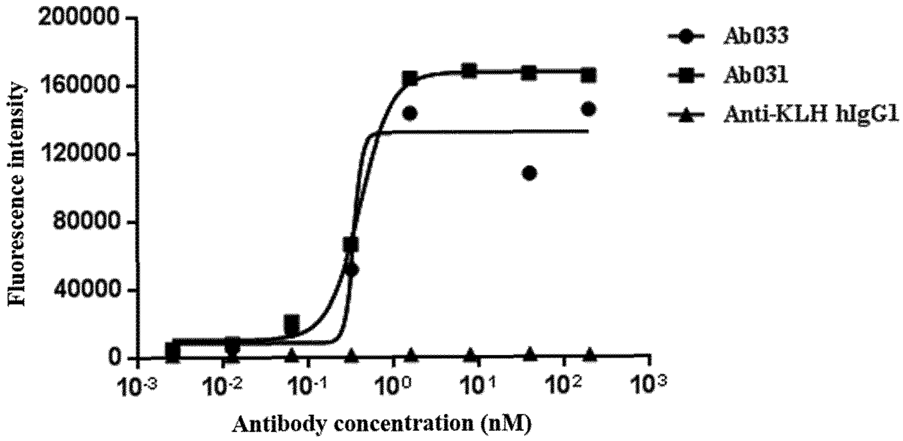


FIG. 8-5

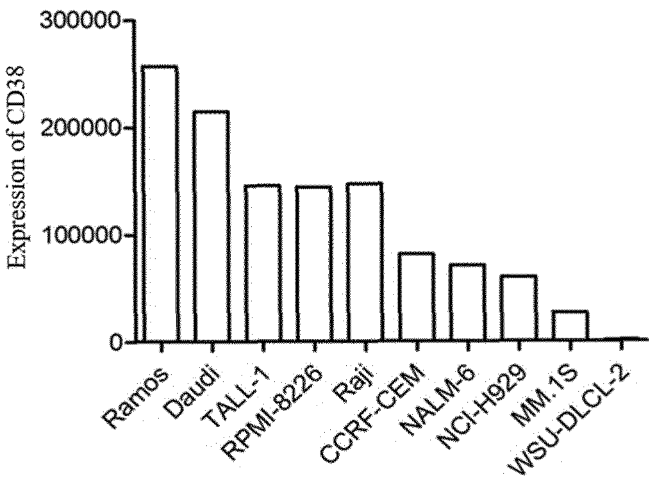


FIG. 9

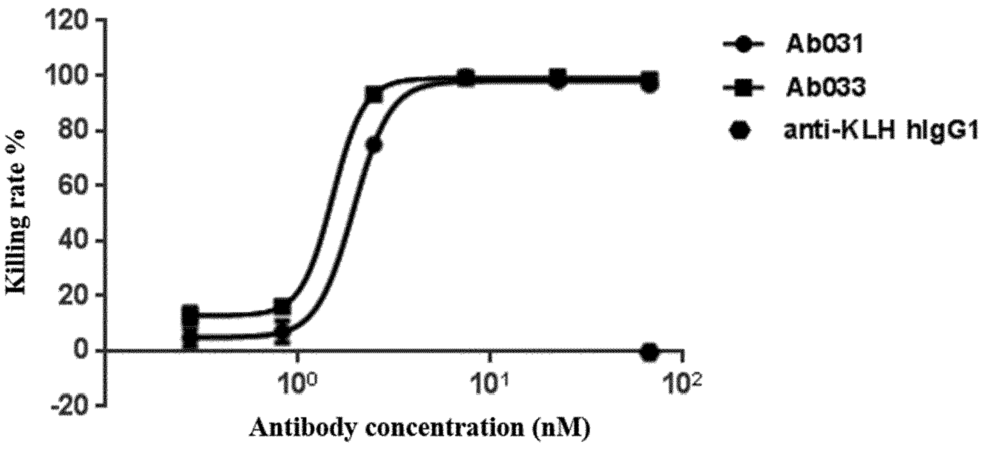


FIG. 10-1

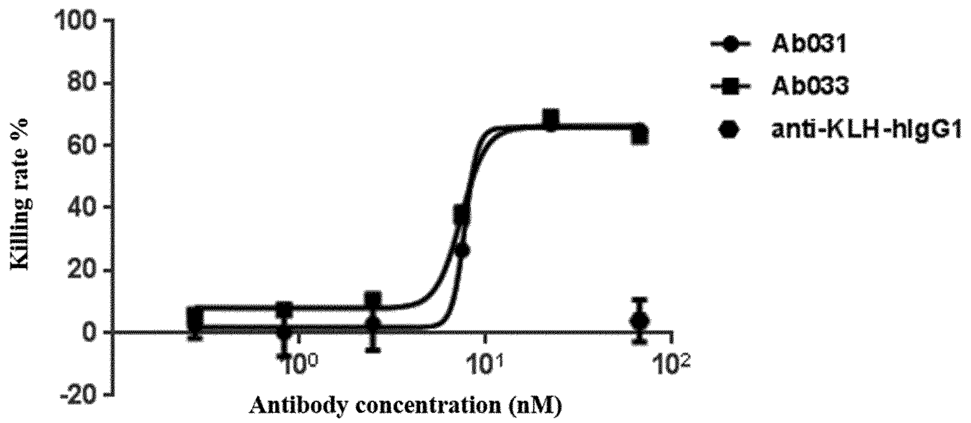


FIG. 10-2

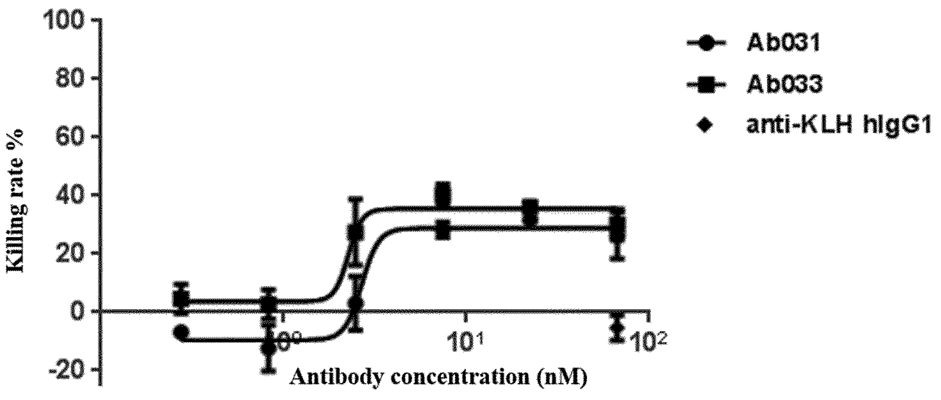


FIG.10-3

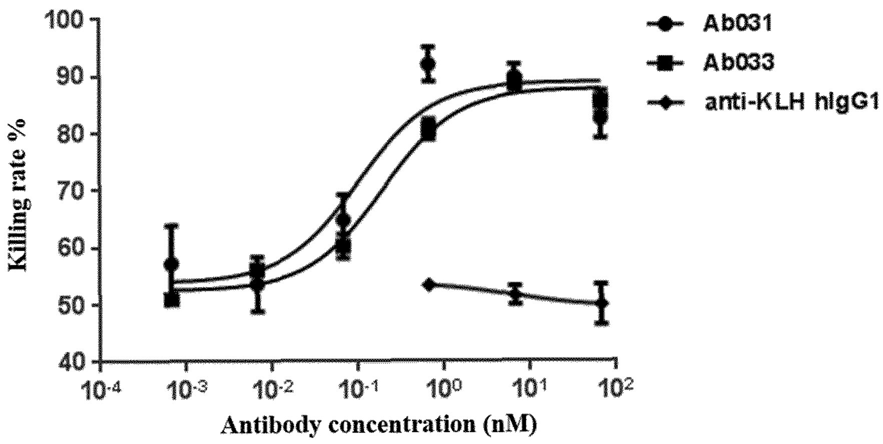


FIG. 11-1

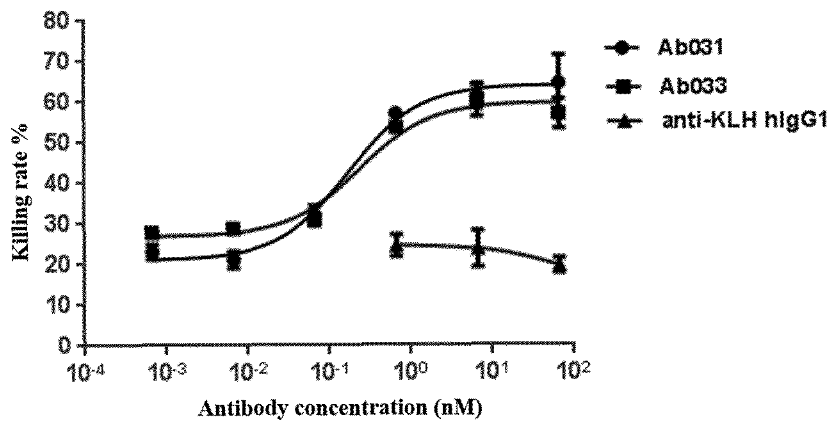


FIG. 11-2

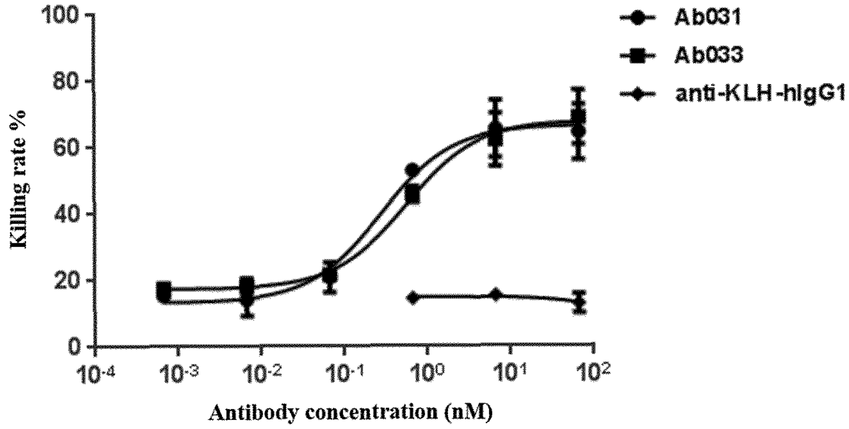


FIG. 11-3

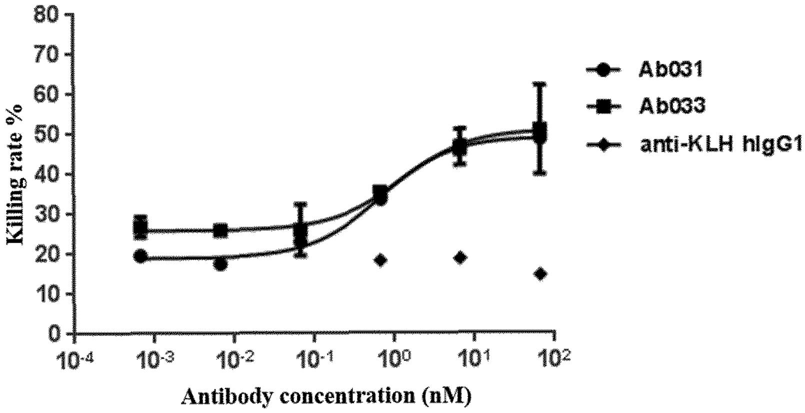


FIG. 11-4

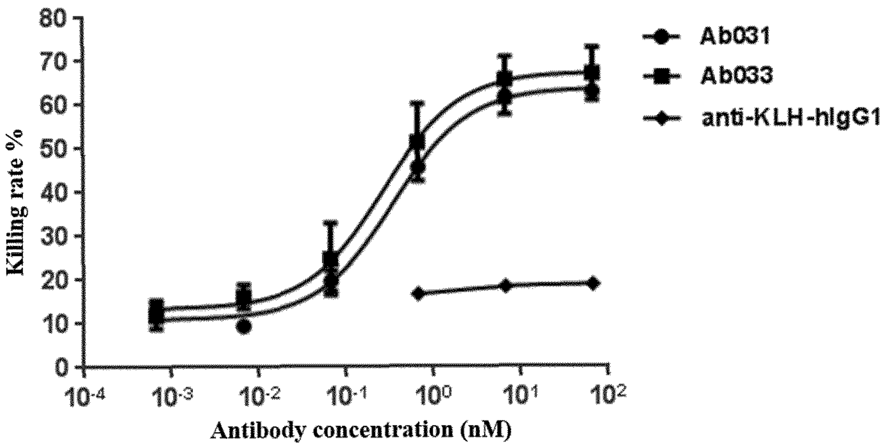


FIG. 11-5

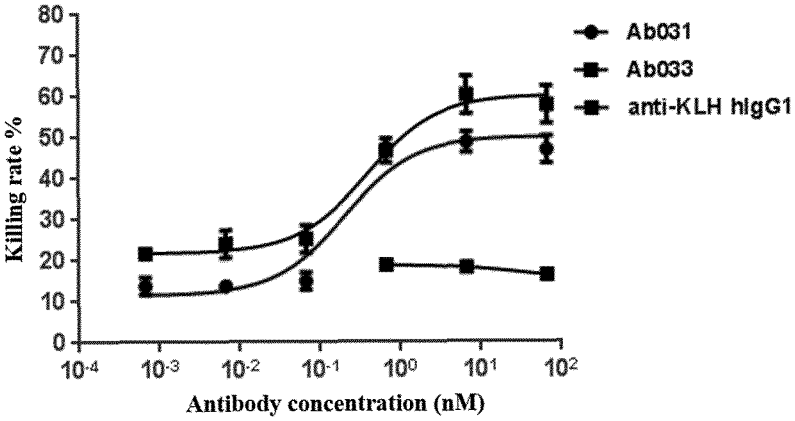


FIG. 11-6

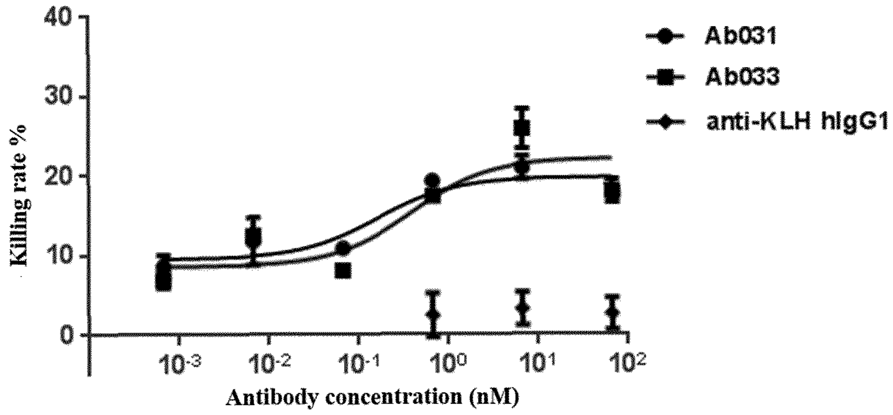


FIG. 11-7

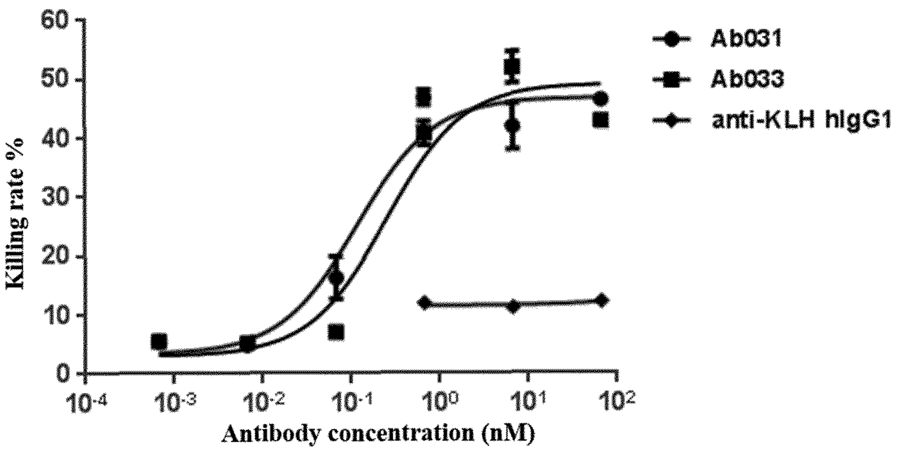


FIG. 11-8

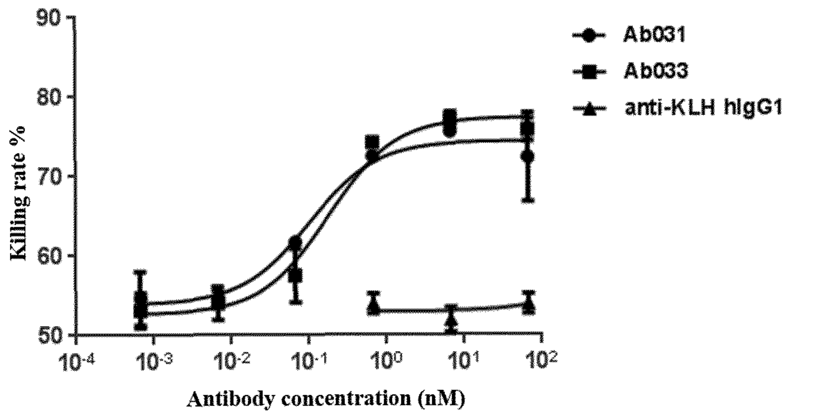


FIG. 11-9

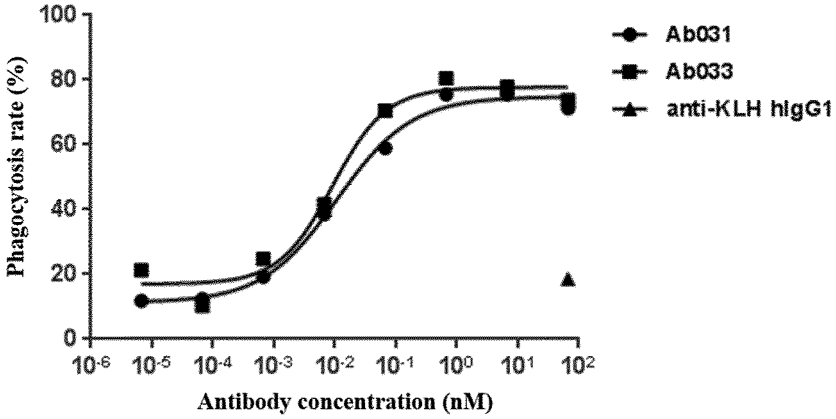


FIG. 12-1

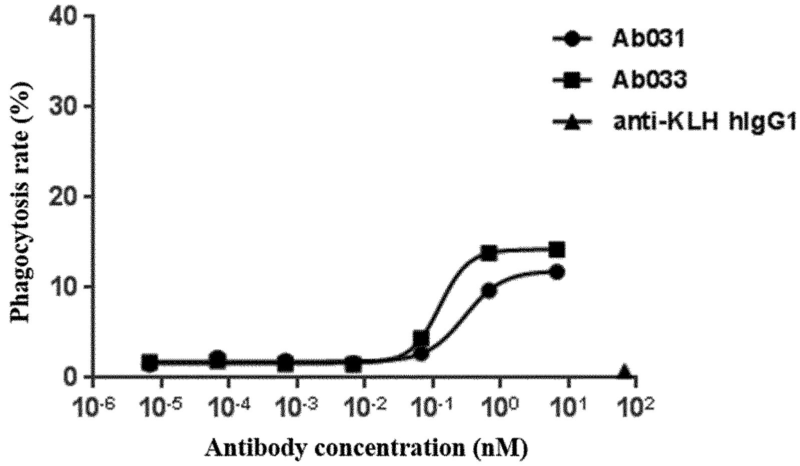


FIG. 12-2

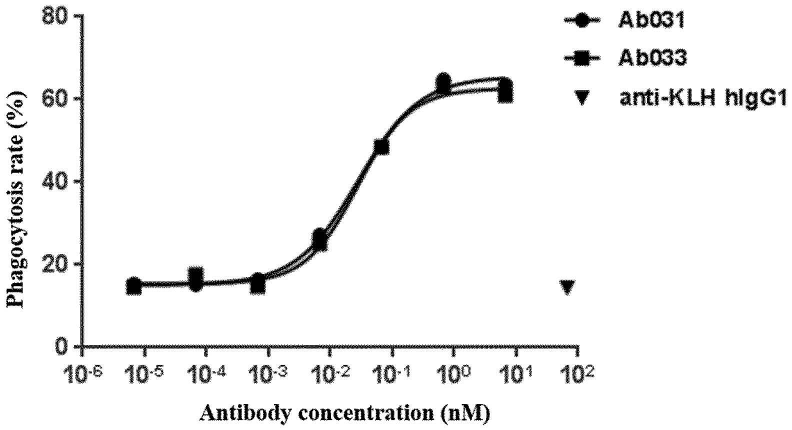


FIG. 12-3

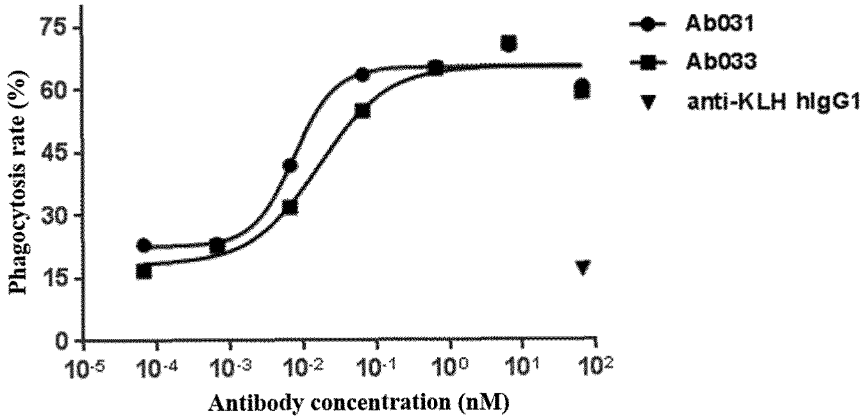


FIG. 12-4

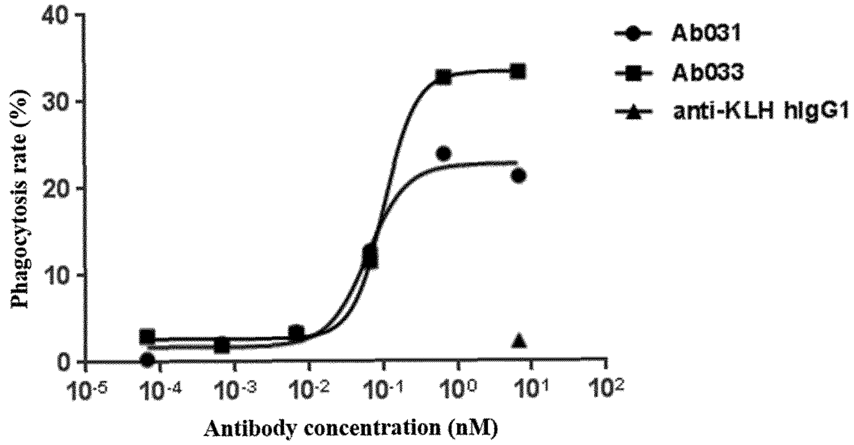


FIG. 12-5

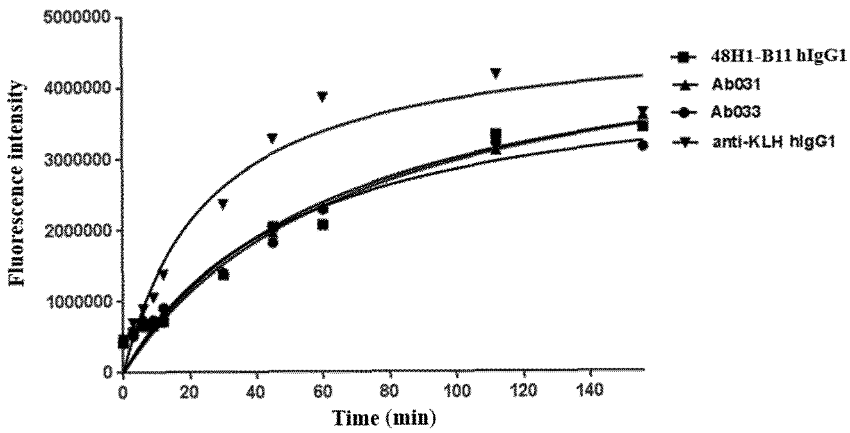


FIG. 13

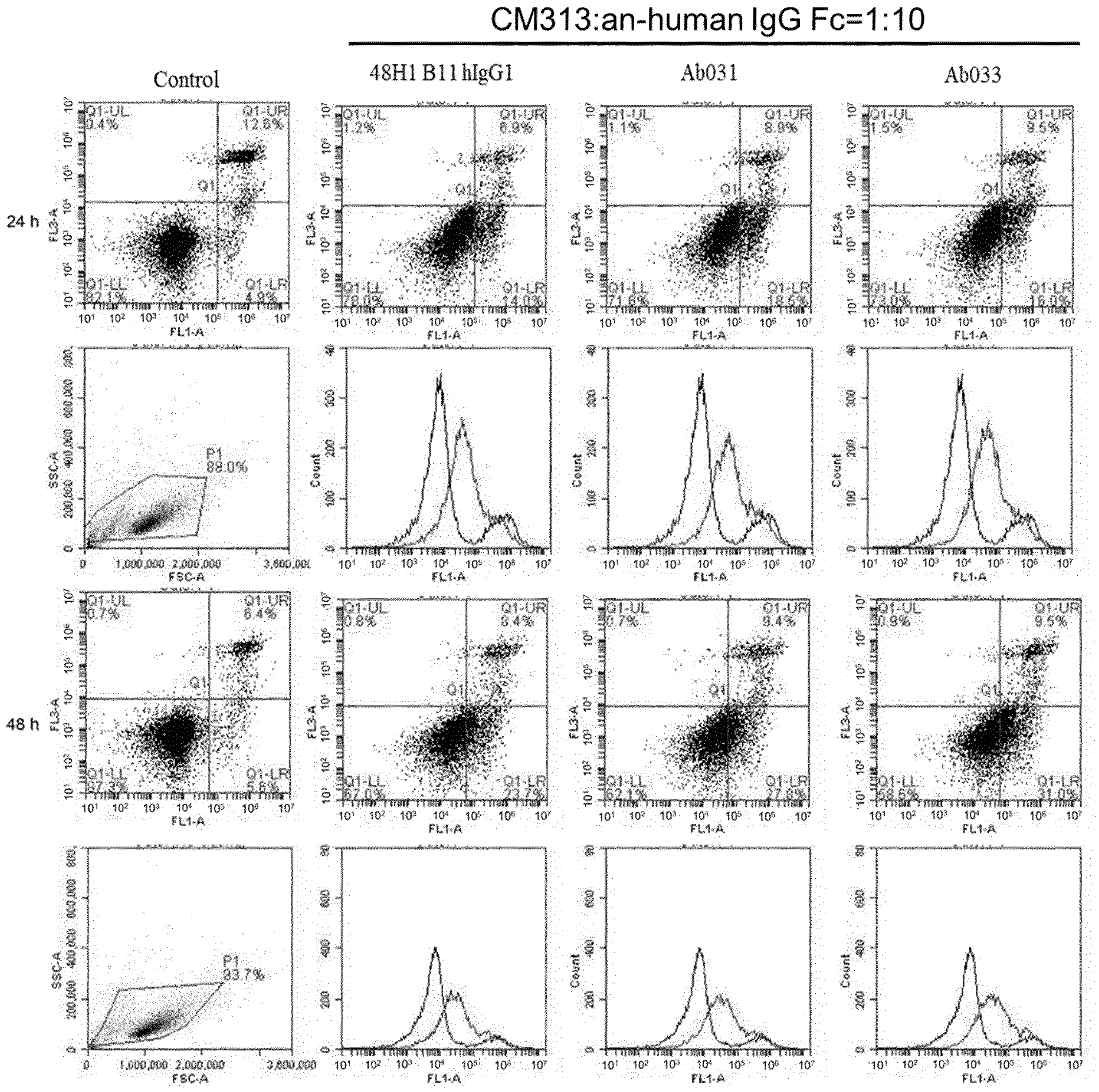
CM313:an-human IgG Fc=1:10
Control
48H1 B11 hIgG1
Ab031
Ab033
24 h
48 h

FIG. 14

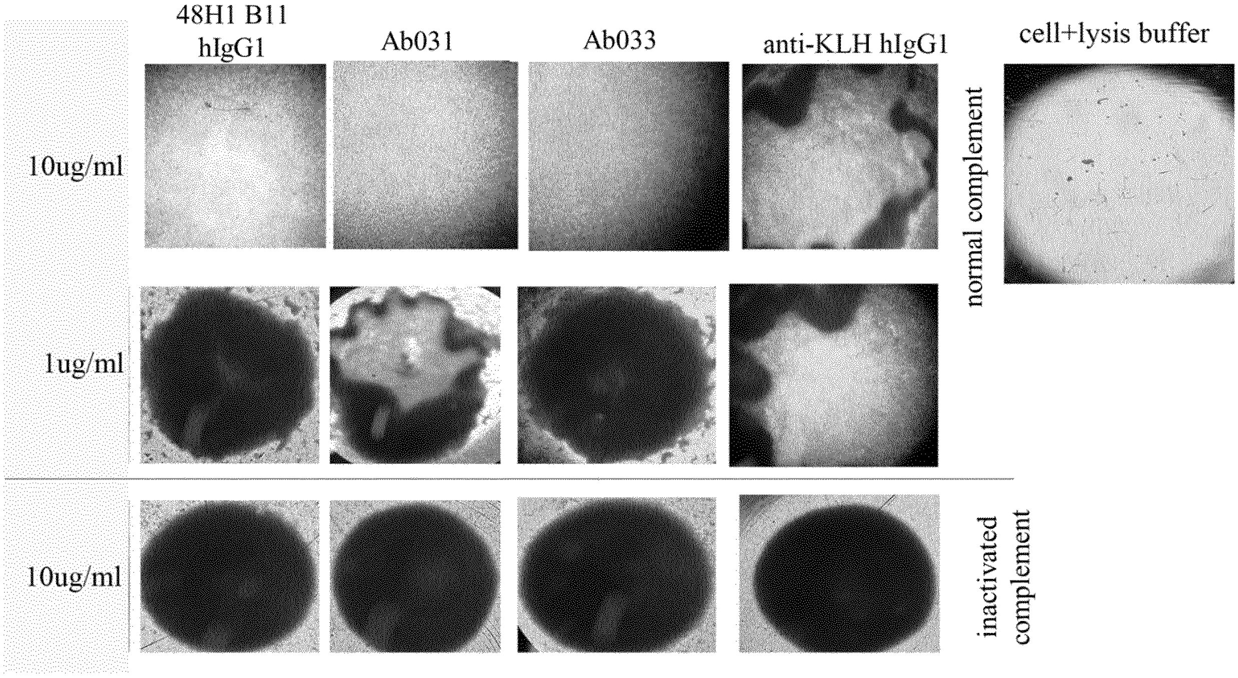
48H1 B11 hIgG1
Ab031
Ab033
anti-KLH hIgG1
cell+lysis buffer
10ug/ml
1ug/ml
10ug/ml
normal complement
inactivated complement

FIG. 15

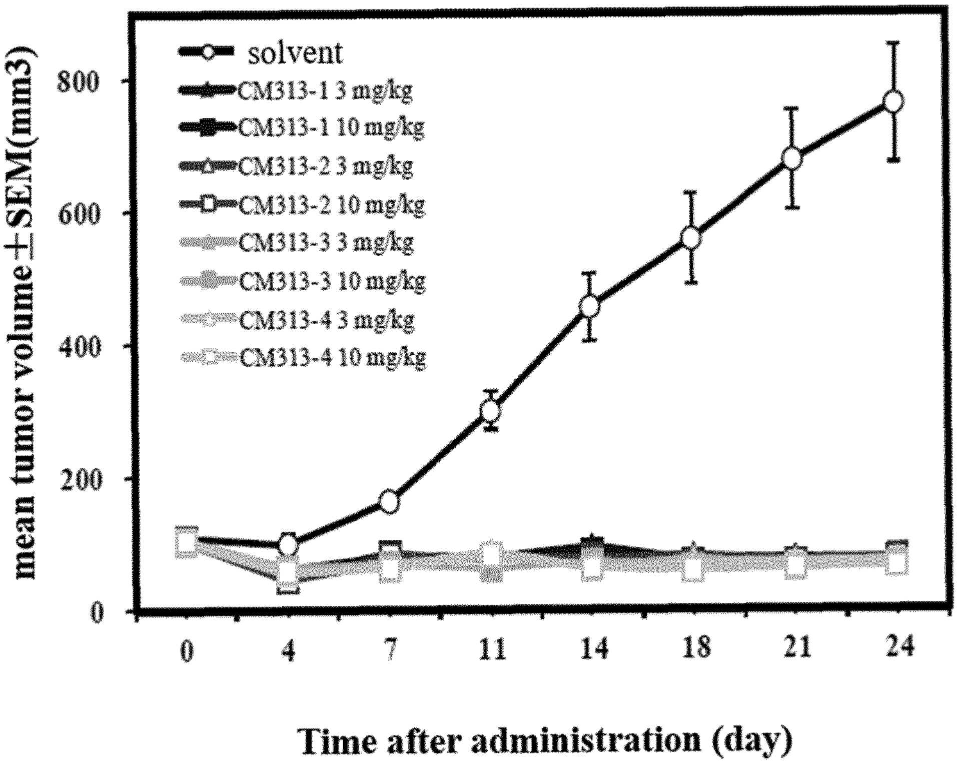
solvent
CM313-1 3 mg/kg
CM313-1 10 mg/kg
CM313-2 3 mg/kg
CM313-2 10 mg/kg
CM313-3 3 mg/kg
CM313-3 10 mg/kg
CM313-4 3 mg/kg
CM313-4 10 mg/kg
mean tumor volume ± SEM(mm3)
0
200
400
600
800
0
4
7
11
14
18
21
24
Time after administration (day)

FIG. 16

## INTERNATIONAL SEARCH REPORT

International application No.
**PCT/CN2020/128820**

**A. CLASSIFICATION OF SUBJECT MATTER**

C07K 16/28(2006.01)i; A61K 39/395(2006.01)i; C12N 15/13(2006.01)i; A61P 35/00(2006.01)i; A61P 19/02(2006.01)i; A61P 29/00(2006.01)i; A61P 37/02(2006.01)i

According to International Patent Classification (IPC) or to both national classification and IPC

**B. FIELDS SEARCHED**

Minimum documentation searched (classification system followed by classification symbols)

C07K; A61K; C12N; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , NCBI, baidu, blast, 中国专利生物序列检索系统, genbank, STNext, 康诺亚生物医药科技(成都)有限公司 , 于俊涛 , CD38, 抗体, antibody, 肿瘤, tumor, 红细胞裂解, RBC lysis, SEQ ID NOs: 1-266

**C. DOCUMENTS CONSIDERED TO BE RELEVANT**

| Category* | Citation of document, with indication, where appropriate, of the relevant passages | Relevant to claim No. |
|---|---|---|
| X | CN 102264386 A (SANOFI) 30 November 2011 (2011-11-30) description page 1 paragraphs 1, 4, 7-8, page 2 paragraphs 16-17, SEQ ID NOs: 1-12, 50-53, 38-41 | 1-12 |
| X | CN 105873953 A (GLENMARK PHARMACEUTICALS S.A.) 17 August 2016 (2016-08-17) description, page 43, paragraph 373 to page 44, paragraph 376 | 1-12 |
| A | WO 2019140410 A1 (TAKEDA PHARMACEUTICAL COMPANY LIMITED et al.) 18 July 2019 (2019-07-18) entire document | 1-12 |
| A | US 2019330363 A1 (JANSSEN BIOTECH, INC.) 31 October 2019 (2019-10-31) entire document | 1-12 |
| A | CN 110229230 A (SHANGHAI KEQI MEDICINE TECHNOLOGY CO., LTD.) 13 September 2019 (2019-09-13) entire document | 1-12 |
| A | CN 110144008 A (HANGZHOU SUMGEN BIOTECHNOLOGY CO., LTD. et al.) 20 August 2019 (2019-08-20) entire document | 1-12 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 January 2021** | **18 February 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/128820**

**C. DOCUMENTS CONSIDERED TO BE RELEVANT**

| Category* | Citation of document, with indication, where appropriate, of the relevant passages | Relevant to claim No. |
|---|---|---|
| A | CN 108495641 A (CELLECTIS) 04 September 2018 (2018-09-04)<br>entire document | 1-12 |

Form PCT/ISA/210 (second sheet) (January 2015)

**INTERNATIONAL SEARCH REPORT**

International application No.
**PCT/CN2020/128820**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2020/128820**

| Patent document cited in search report | Publication date (day/month/year) | Patent family member(s) | Publication date (day/month/year) |
|---|---|---|---|
| CN 102264386 A | 30 November 2011 | SI 2370096 T1 | 30 October 2017 |
| | | AU 2009321251 B2 | 09 June 2016 |
| | | CN 107096022 A | 29 August 2017 |
| | | DK 2370096 T3 | 17 July 2017 |
| | | NZ 593085 A | 21 December 2012 |
| | | JP 6072854 B2 | 01 February 2017 |
| | | TN 2011000242 A1 | 17 December 2012 |
| | | HU E035910 T2 | 28 May 2018 |
| | | PA 8849901 A1 | 27 July 2010 |
| | | CR 20110282 A | 20 September 2011 |
| | | EP 2370096 A1 | 05 October 2011 |
| | | EC SP11011071 A | 29 July 2011 |
| | | IL 213115 D0 | 31 July 2011 |
| | | SG 171821 A1 | 28 July 2011 |
| | | DK 2370096 T5 | 18 September 2017 |
| | | EA 027070 B1 | 30 June 2017 |
| | | US 2012093806 A1 | 19 April 2012 |
| | | MX 2011005666 A | 30 September 2011 |
| | | PT 2370096 T | 08 September 2017 |
| | | HR P20171301 T1 | 20 October 2017 |
| | | PL 2370096 T3 | 31 October 2017 |
| | | RS 56248 B1 | 30 November 2017 |
| | | HN 2011001423 A | 13 January 2014 |
| | | AU 2009321251 A1 | 03 June 2010 |
| | | IL 213118 D0 | 31 July 2011 |
| | | MA 32899 B1 | 01 December 2011 |
| | | AR 073425 A1 | 03 November 2010 |
| | | EP 2191842 A1 | 02 June 2010 |
| | | MX 344973 B | 12 January 2017 |
| | | EP 2370096 B1 | 31 May 2017 |
| | | BR PI0921858 A2 | 09 October 2018 |
| | | WO 2010061359 A1 | 03 June 2010 |
| | | CA 2745005 A1 | 03 June 2010 |
| | | KR 20110096551 A | 30 August 2011 |
| | | NI 201100107 A | 26 September 2011 |
| | | JP 2012510463 A | 10 May 2012 |
| | | CL 2011001232 A1 | 10 February 2012 |
| | | UA 104160 C2 | 10 January 2014 |
| | | CO 6440556 A2 | 15 May 2012 |
| | | TW 201023863 A | 01 July 2010 |
| | | TW I436770 B | 11 May 2014 |
| | | IL 213115 A | 31 August 2017 |
| | | EA 201100868 A1 | 31 October 2011 |
| | | LT 2370096 T | 25 September 2017 |
| | | KR 101715958 B1 | 14 March 2017 |
| | | PE 20120205 A1 | 03 March 2012 |
| | | ES 2638926 T3 | 24 October 2017 |
| | | ZA 201104064 B | 26 September 2012 |
| | | JP 2015205893 A | 19 November 2015 |
| CN 105873953 A | 17 August 2016 | BR 112017009264 A2 | 30 January 2018 |

Form PCT/ISA/210 (patent family annex) (January 2015)

**INTERNATIONAL SEARCH REPORT**
**Information on patent family members**

International application No.
**PCT/CN2020/128820**

| Patent document cited in search report | Publication date (day/month/year) | Patent family member(s) | Publication date (day/month/year) |
|---|---|---|---|
| | | PH 12016500823 A1 | 13 June 2016 |
| | | AU 2014343636 A1 | 02 June 2016 |
| | | IL 245381 D0 | 30 June 2016 |
| | | MX 2016005781 A | 18 July 2016 |
| | | KR 20160090308 A | 29 July 2016 |
| | | US 2017145115 A1 | 25 May 2017 |
| | | PE 20160724 A1 | 04 August 2016 |
| | | CL 2016001068 A1 | 18 November 2016 |
| | | AU 2015342169 A1 | 08 June 2017 |
| | | CA 2966124 A1 | 12 May 2016 |
| | | EP 3176185 A1 | 07 June 2017 |
| | | US 2020102403 A1 | 02 April 2020 |
| | | US 2015133640 A1 | 14 May 2015 |
| | | ZA 201603433 B | 27 March 2019 |
| | | EA 201690803 A1 | 30 September 2016 |
| | | CN 110627907 A | 31 December 2019 |
| | | AP 201609222 D0 | 31 May 2016 |
| | | KR 20170092562 A | 11 August 2017 |
| | | BR 112016009919 A2 | 05 December 2017 |
| | | IL 251850 D0 | 29 June 2017 |
| | | AU 2019257534 A1 | 28 November 2019 |
| | | EP 3066133 A1 | 14 September 2016 |
| | | CA 2929256 A1 | 07 May 2015 |
| | | AP 201609222 A0 | 31 May 2016 |
| | | AP 2016009222 A0 | 31 May 2016 |
| | | JP 2017536412 A | 07 December 2017 |
| | | US 2018355064 A1 | 13 December 2018 |
| | | SG 10201909806 S A | 28 November 2019 |
| | | SG 11201603244V A | 30 May 2016 |
| | | CN 107207595 A | 26 September 2017 |
| | | EP 3215540 A1 | 13 September 2017 |
| | | US 2018112011 A1 | 26 April 2018 |
| | | US 9493563 B2 | 15 November 2016 |
| | | HK 1244013 A1 | 27 July 2018 |
| | | WO 2015063339 A1 | 07 May 2015 |
| | | JP 2016538275 A | 08 December 2016 |
| | | JP 2020023516 A | 13 February 2020 |
| | | WO 2016071004 A1 | 12 May 2016 |
| WO 2019140410 A1 | 18 July 2019 | CA 3088199 A1 | 18 July 2019 |
| | | AU 2019208102 A1 | 02 July 2020 |
| US 2019330363 A1 | 31 October 2019 | US 10781261 B2 | 22 September 2020 |
| CN 110229230 A | 13 September 2019 | None | |
| CN 110144008 A | 20 August 2019 | CA 3091492 A1 | 15 August 2019 |
| | | TW 201934577 A | 01 September 2019 |
| | | AU 2019218518 A1 | 01 October 2020 |
| | | CN 111712521 A | 25 September 2020 |
| | | WO 2019154421 A1 | 15 August 2019 |
| | | SG 11202007443 X A | 29 September 2020 |
| CN 108495641 A | 04 September 2018 | IL 257009 D0 | 29 March 2018 |

Form PCT/ISA/210 (patent family annex) (January 2015)

**INTERNATIONAL SEARCH REPORT**
**Information on patent family members**

International application No.
**PCT/CN2020/128820**

| Patent document cited in search report | Publication date (day/month/year) | Patent family member(s) | | | Publication date (day/month/year) |
|---|---|---|---|---|---|
| | | US | 2018236053 | A1 | 23 August 2018 |
| | | RU | 2018107754 | A3 | 20 December 2019 |
| | | RU | 2018107754 | A | 12 September 2019 |
| | | KR | 20180041152 | A | 23 April 2018 |
| | | JP | 2018522907 | A | 16 August 2018 |
| | | BR | 112018002600 | A2 | 23 October 2018 |
| | | WO | 2017025323 | A1 | 16 February 2017 |
| | | MX | 2018001776 | A | 06 June 2018 |
| | | AU | 2016307050 | A1 | 15 February 2018 |
| | | CA | 2994746 | A1 | 16 February 2017 |
| | | EP | 3334442 | A1 | 20 June 2018 |
| | | US | 10709775 | B2 | 14 July 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7229619 B
- US 20150231235 A1
- US 20110293606 A1


### Non-patent literature cited in the description


- **YUE LONGTAO et al.** *Current Immunology,* 2014, 89-93
- Computational Molecular Biology. Oxford University Pres, 1988
- Biocomputing: Informatics and Genome Projects. Academic Press, 1993
- Computer Analysis of Sequence Data. Humana Press, 1994
- Sequence Analysis in Molecular Biology. Academic Press, 1987
- Sequence Analysis Prime. M Stockton Press, 1991
- **CARRILLO, H. ; LIPMAN, D.** *SIAM J Applied Math,* 1988, vol. 48, 1073
- **KABAT et al.** Sequences of Proteins of Immunological Interest. national Institute of Health, 1987
- *Methods in Molecular Biology,* vol. 207
- Recombinant Antibodies for Cancer Therapy Methods and Protocols. 2003, 3-25
- **SMITH et al.** *J.Clin.Pathol.,* 2004, vol. 57, 912-917
- **NELSON et al.** *J Clin Pathol,* 2000, vol. 53, 111-117
- **RICH ; MYSZKA.** *Curr. Opin. Biotechnol,* 2000, vol. 11, 54
- **ENGLEBIENNE.** *Analyst,* 1998, vol. 123, 1599
- Fundamental Immunology. Raven Press, 1989, 332-336
- BiaCore 2000, Biacore AB, Upsala, Sweden and GE Healthcare Life Sciences; Malmqvist. *Biochem.Soc.Trans.,* 2000, vol. 27, 335
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl. Acad. Sci. USA,* 1997, vol. 94, 412-417
- Remington's Pharmaceutical Sciences. Mack Publishing Company, 1975
- ELISA: Theory and Practice: Methods in Molecular Biology. Human Press, 1995, vol. 42
- **E. DIAMANDIS ; T. CHRISTOPOULUS.** Immunoassay. Academic Press, Inc, 1996
- **P. TIJSSEN.** Practice and Theory of Enzyme Immunoassays. Elsevier Science Publishers, 1985
- Remington's Pharmaceutical Sciences: The Science and Practice of Pharmacy. Mack Pub. Co, 1995
- Drug Absorption Enhancement: Concepts, Possibilities, Limitations, and Trends. Harwood Academic Publishers, 1994
- Peptide and Protein Drug Delivery (Advances In Parenteral Sciences. M. Dekker, 1991, vol. 4
- **MARASCO et al.** *Proc. Natl. Acad. Sci. USA,* 1993, vol. 90, 7889-7893

SEQUENCE LISTING

<110>  Keymed Biosciences Co.,Ltd
Shanghai Lingyue Biopharma Co.,Ltd

<120>  Pharmaceutical composition, preparation method therefor and use thereof

<130>  MTPIEP19042

<140>  PCT/CN2020/128820
<141>  2020-11-13

<150>  CN201911207085.X
<151>  2019-11-29

<160>  270

<170>  SIPOSequenceListing 1.0

<210>  1
<211>  114
<212>  PRT
<213>  Mus musculus

<400>  1
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Asn His
            20                  25                  30
Gly Met Asn Trp Val Lys Gln Ala Pro Gly Lys Gly Leu Lys Trp Met
        35                  40                  45
Gly Trp Ile Asn Thr Tyr Thr Gly Glu Pro Thr Tyr Gly Glu Asp Phe
    50                  55                  60
Lys Gly Arg Phe Ala Phe Ser Leu Glu Thr Ser Ala Ser Thr Ala Tyr
65                  70                  75                  80
Leu Gln Ile Asn Asn Leu Lys Asn Glu Asp Leu Ala Thr Tyr Phe Cys
                85                  90                  95
Ala Arg Lys Gly Phe Val Tyr Trp Gly Gln Gly Thr Leu Val Thr Val
            100                 105                 110
Ser Val

<210>  2
<211>  6
<212>  PRT
<213>  Mus musculus

<400>  2
Thr Asn His Gly Met Asn
1               5

<210>  3
<211>  17
<212>  PRT
<213>  Mus musculus

<400>  3
Trp Ile Asn Thr Tyr Thr Gly Glu Pro Thr Tyr Gly Glu Asp Phe Lys
1               5                   10                  15
Gly

```
<210>  4
<211>  5
<212>  PRT
<213>  Mus musculus

<400>  4
Lys Gly Phe Val Tyr
1               5

<210>  5
<211>  342
<212>  DNA
<213>  Mus musculus

<400>  5
cagatccagt tggtgcagtc tggacctgag ctgaagaagc ctggagagac agtcaagatc       60
tcctgcaagg cttctgggta tactttcaca aaccatggaa tgaactgggt gaagcaggct      120
ccaggaaagg gcttaaagtg gatgggctgg ataaacacct acactggaga gccaacatat      180
ggtgaagact tcaagggacg gtttgccttc tctttggaaa cctctgccag cactgcctat      240
ttgcagatca acaacctcaa aaatgaggac ctggctacat atttctgtgc aagaaagggg      300
tttgtttact ggggccaagg gactctggtc actgtctctg ta                         342

<210>  6
<211>  111
<212>  PRT
<213>  Mus musculus

<400>  6
Asp Ile Val Leu Thr Gln Ser Pro Ala Ser Leu Ala Val Ser Leu Gly
1               5                   10                  15
Gln Arg Ala Thr Met Ser Cys Arg Ala Ser Glu Ser Val Asp Ile Tyr
            20                  25                  30
Gly Asn Ser Phe Ile Tyr Trp Tyr Gln Gln Lys Pro Gly Gln Pro Pro
        35                  40                  45
Lys Leu Leu Ile Tyr Arg Ala Ser Ile Leu Glu Ser Gly Ile Pro Ala
    50                  55                  60
Arg Phe Ser Gly Ser Gly Ser Arg Thr Asp Phe Thr Leu Thr Ile Asn
65                  70                  75                  80
Pro Val Glu Ala Asp Asp Val Ala Thr Tyr Tyr Cys Gln Gln Ile Asn
                85                  90                  95
Glu Asp Pro Phe Thr Phe Gly Gly Gly Thr Lys Leu Glu Ile Lys
            100                 105                 110

<210>  7
<211>  15
<212>  PRT
<213>  Mus musculus

<400>  7
Arg Ala Ser Glu Ser Val Asp Ile Tyr Gly Asn Ser Phe Ile Tyr
1               5                   10                  15

<210>  8
<211>  7
<212>  PRT
<213>  Mus musculus

<400>  8
Arg Ala Ser Ile Leu Glu Ser
1               5

<210>  9
<211>  9
```

<212> PRT
<213> Mus musculus

<400> 9
Gln Gln Ile Asn Glu Asp Pro Phe Thr
1 5

<210> 10
<211> 332
<212> DNA
<213> Mus musculus

<400> 10
acattgtgct gacccaatct ccagcttctt tggctgtgtc tctagggcag agggccacca 60
tgtcctgcag agccagtgaa agtgttgata tttatggcaa tagttttata tactggtacc 120
aacagaaacc aggacagcca cccaaactcc tcatctatcg tgcatccatc ctagaatctg 180
ggatccctgc caggttcagt ggcagtgggt ctaggacaga cttcaccctc accattaatc 240
ctgtggaggc tgatgatgtt gcaacctatt actgtcagca aattaatgaa gatccattca 300
cgttcggagg ggggaccaag ctggaaataa aa 332

<210> 11
<211> 120
<212> PRT
<213> Mus musculus

<400> 11
Gln Ala Tyr Leu Gln Gln Ser Gly Ala Glu Leu Val Arg Ser Gly Ala
1 5 10 15
Ser Val Lys Met Ser Cys Lys Ala Ser Gly Tyr Thr Phe Thr Ser Tyr
20 25 30
Asn Ile His Trp Val Lys Gln Thr Pro Gly Gln Gly Leu Lys Trp Ile
35 40 45
Gly Tyr Ile Tyr Pro Gly Asn Gly Gly Thr Asn Tyr Asn Gln Lys Phe
50 55 60
Lys Val Lys Ala Thr Leu Thr Ala Asp Thr Ser Ser Ser Thr Ala Tyr
65 70 75 80
Met Gln Ile Ser Ser Leu Thr Ser Glu Asp Ser Ala Val Tyr Phe Cys
85 90 95
Ala Arg Arg Gly Ser Asp Tyr Asp Gly Gly Phe Ala Ser Trp Gly Gln
100 105 110
Gly Thr Leu Val Thr Val Ser Ala
115 120

<210> 12
<211> 6
<212> PRT
<213> Mus musculus

<400> 12
Thr Ser Tyr Asn Ile His
1 5

<210> 13
<211> 17
<212> PRT
<213> Mus musculus

<400> 13
Tyr Ile Tyr Pro Gly Asn Gly Gly Thr Asn Tyr Asn Gln Lys Phe Lys
1 5 10 15
Val

<210> 14
<211> 11
<212> PRT
<213> Mus musculus

<400> 14
Arg Gly Ser Asp Tyr Asp Gly Gly Phe Ala Ser
1 5 10

<210> 15
<211> 360
<212> DNA
<213> Mus musculus

<400> 15
caggcttatc tacagcagtc tggggctgag ctggtgaggt ctggggcctc agtgaagatg 60
tcctgcaagg cttctggcta cacatttacc agttacaata tacactgggt aaagcagaca 120
cctggacagg gcctgaaatg gattggatat atctatcctg gaaatggtgg tactaactac 180
aatcagaagt tcaaggtcaa ggccacattg actgcagaca catcctccag cacagcctac 240
atgcagatca gcagcctgac atctgaagac tctgcggtct atttctgtgc aagaaggggg 300
tctgattacg acggggggtt tgcttcctgg ggccaaggga ctctggtcac tgtctctgca 360

<210> 16
<211> 111
<212> PRT
<213> Mus musculus

<400> 16
Asp Ile Leu Leu Thr Gln Ser Pro Ala Ser Leu Ala Val Ser Leu Gly
1 5 10 15
Gln Arg Ala Thr Ile Ser Cys Arg Ala Ser Lys Ser Val Asp Ile Tyr
20 25 30
Gly Asn Ser Phe Ile His Trp Tyr Gln Gln Lys Pro Gly Gln Ser Pro
35 40 45
Lys Leu Leu Ile Tyr Leu Ala Ser Asn Leu Asp Ser Gly Val Pro Ala
50 55 60
Arg Phe Ser Gly Ser Gly Ser Arg Thr Asp Phe Thr Leu Thr Ile Asp
65 70 75 80
Pro Val Glu Ala Asp Asp Ala Ala Thr Tyr Tyr Cys Gln Gln Asn Asn
85 90 95
Glu Asp Pro Leu Thr Phe Gly Ala Gly Thr Lys Leu Glu Leu Lys
100 105 110

<210> 17
<211> 15
<212> PRT
<213> Mus musculus

<400> 17
Arg Ala Ser Lys Ser Val Asp Ile Tyr Gly Asn Ser Phe Ile His
1 5 10 15

<210> 18
<211> 7
<212> PRT
<213> Mus musculus

<400> 18
Leu Ala Ser Asn Leu Asp Ser
1 5

<210> 19
<211> 9

<212> PRT
<213> Mus musculus

<400> 19
Gln Gln Asn Asn Glu Asp Pro Leu Thr
1 5

<210> 20
<211> 333
<212> DNA
<213> Mus musculus

<400> 20
gacattttgc tgacccagtc tccagcttct ttggctgtgt ctctagggca gagggccacc 60
atatcctgca gagccagtaa aagtgttgat atttatggca atagttttat acactggtac 120
cagcagaaac caggacagtc acccaaactc ctcatctatc ttgcatccaa cctagattct 180
ggggtccctg ccaggttcag tggcagtggg tctaggacag acttcaccct caccattgat 240
cctgtggagg ctgatgatgc tgcaacctat tactgtcagc aaaataatga ggatccgctc 300
acgttcggtg ctgggaccaa gctggagctg aaa 333

<210> 21
<211> 119
<212> PRT
<213> Mus musculus

<400> 21
Gln Leu Gln Leu Lys Gln Ser Gly Pro Gly Leu Val Gln Pro Ser Gln
1 5 10 15
Ser Leu Ser Ile Thr Cys Thr Val Ser Gly Phe Ser Leu Thr Ser Phe
20 25 30
Gly Val His Trp Ile Arg Gln Ser Pro Gly Lys Gly Leu Glu Trp Leu
35 40 45
Gly Val Ile Trp Arg Gly Gly Ser Thr Asp Tyr Asn Ala Ala Phe Met
50 55 60
Ser Arg Leu Ser Ile Thr Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Lys Thr Met Ile Thr Arg Gly Tyr Thr Met Asp Tyr Trp Gly Gln Gly
100 105 110
Thr Ser Val Thr Val Ser Ser
115

<210> 22
<211> 6
<212> PRT
<213> Mus musculus

<400> 22
Thr Ser Phe Gly Val His
1 5

<210> 23
<211> 16
<212> PRT
<213> Mus musculus

<400> 23
Val Ile Trp Arg Gly Gly Ser Thr Asp Tyr Asn Ala Ala Phe Met Ser
1 5 10 15

<210> 24
<211> 11

<212> PRT
<213> Mus musculus

<400> 24
Thr Met Ile Thr Arg Gly Tyr Thr Met Asp Tyr
1 5 10

<210> 25
<211> 357
<212> DNA
<213> Mus musculus

<400> 25
cagttgcagc tgaagcagtc aggacctggc ctagtgcagc cctcacagag cctgtccata 60
acctgcacag tctctggttt ctcattaact agctttggtg tacactggat tcgccagtct 120
ccaggaaagg gtctggagtg gctgggagtg atttggagag gtggaagcac agactacaat 180
gcagctttca tgtccagact gagcatcacc aaggacaact ccaagagcca agttttcttt 240
aaaatgaaca gtctgcaagc tgatgacact gccatatatt actgtgccaa aactatgatt 300
acgagggggt atactatgga ctactggggt caaggaacct cagtcaccgt ctcctca 357

<210> 26
<211> 107
<212> PRT
<213> Mus musculus

<400> 26
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Lys Pro Gly Asn Ala Pro Arg Leu Leu Ile
35 40 45
Ser Gly Ala Thr Thr Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Phe Trp Ser Thr Pro Tyr
85 90 95
Thr Phe Gly Gly Gly Thr Lys Leu Glu Met Ile
100 105

<210> 27
<211> 11
<212> PRT
<213> Mus musculus

<400> 27
Lys Ala Ser Glu Asp Ile Tyr Asn Arg Leu Ala
1 5 10

<210> 28
<211> 7
<212> PRT
<213> Mus musculus

<400> 28
Gly Ala Thr Thr Leu Glu Thr
1 5

<210> 29
<211> 9
<212> PRT
<213> Mus musculus

<400> 29
Gln Gln Phe Trp Ser Thr Pro Tyr Thr
1 5

<210> 30
<211> 321
<212> DNA
<213> Mus musculus

<400> 30
gacatccaga tgacacaatc ctcatcctcc ttttctgtat ctctaggaga cagggtcacc 60
attacttgca aggcaagtga ggacatatat aatcggttag cctggtatca gcagaaacca 120
ggaaatgctc ctaggctctt aatatctggt gcaaccactt tggaaactgg ggttccttca 180
agattcagtg gcagtggatc tggaaaggat tacactctca gcattaccag tcttcagact 240
gaagatgttg ctacttatta ctgtcaacag ttttggagta ctccgtacac gttcggaggg 300
gggaccaagc tggaaatgat a 321

<210> 31
<211> 118
<212> PRT
<213> Mus musculus

<400> 31
Glu Val Gln Leu Val Glu Ser Gly Gly Gly Leu Val Lys Pro Gly Gly
1 5 10 15
Ser Leu Lys Leu Ser Cys Glu Gly Ser Gly Phe Thr Phe Asn Asn Tyr
20 25 30
Ala Met Ser Trp Val Arg Gln Ser Pro Glu Lys Arg Leu Glu Trp Val
35 40 45
Ala Glu Ile Ser Thr Gly Gly Thr Phe Thr Tyr Tyr Val Asp Thr Val
50 55 60
Thr Gly Arg Phe Thr Ile Ser Arg Asp Asn Ala Lys Asn Thr Leu Tyr
65 70 75 80
Leu Glu Met Ser Ser Leu Arg Ser Glu Asp Thr Ala Arg Tyr Tyr Cys
85 90 95
Ala Arg Gly Tyr Arg Tyr Asp Ala Ala Asp Tyr Trp Gly Gln Gly Ile
100 105 110
Ser Val Thr Val Ser Ser
115

<210> 32
<211> 6
<212> PRT
<213> Mus musculus

<400> 32
Asn Asn Tyr Ala Met Ser
1 5

<210> 33
<211> 17
<212> PRT
<213> Mus musculus

<400> 33
Glu Ile Ser Thr Gly Gly Thr Phe Thr Tyr Tyr Val Asp Thr Val Thr
1 5 10 15
Gly

<210> 34
<211> 9

<212> PRT
<213> Mus musculus

<400> 34
Gly Tyr Arg Tyr Asp Ala Ala Asp Tyr
1 5

<210> 35
<211> 354
<212> DNA
<213> Mus musculus

<400> 35
gaagtgcagc tggtggagtc tgggggaggc ttagtgaagc ctggagggtc cctgaaactc 60
tcctgtgaag gctctggatt cactttcaac aactatgcca tgtcttgggt tcgccagtct 120
ccagagaaga ggctggagtg ggtcgcagag atttctactg gtggtacttt cacctactat 180
gtagacactg tgacgggccg attcaccatc tccagagaca atgccaagaa caccctgtac 240
ctggaaatga gtagtctgag gtctgaggac acggccaggt attattgtgc aagaggctat 300
aggtacgacg cggcggacta ctggggtcaa ggaatctcag tcaccgtctc ctca 354

<210> 36
<211> 106
<212> PRT
<213> Mus musculus

<400> 36
Asp Ile Gln Met Thr Gln Ser Pro Ser Ser Leu Ser Ala Ser Leu Gly
1 5 10 15
Glu Arg Val Ser Leu Thr Cys Arg Ala Ser Gln Glu Ile Ser Gly Tyr
20 25 30
Leu Ile Trp Leu Gln Gln Lys Pro Asp Gly Thr Ile Lys Arg Leu Ile
35 40 45
Tyr Ala Thr Ser Thr Leu Asp Ser Gly Val Pro Lys Arg Phe Ser Gly
50 55 60
Ser Arg Ser Gly Ser Asp Tyr Thr Leu Thr Ile Ser Ser Leu Glu Ser
65 70 75 80
Glu Asp Phe Ala Asp Tyr Tyr Cys Leu Gln Tyr Ala Asn Tyr Phe Thr
85 90 95
Phe Gly Ala Gly Thr Lys Leu Glu Leu Lys
100 105

<210> 37
<211> 11
<212> PRT
<213> Mus musculus

<400> 37
Arg Ala Ser Gln Glu Ile Ser Gly Tyr Leu Ile
1 5 10

<210> 38
<211> 7
<212> PRT
<213> Mus musculus

<400> 38
Ala Thr Ser Thr Leu Asp Ser
1 5

<210> 39
<211> 8
<212> PRT
<213> Mus musculus

<400> 39
Leu Gln Tyr Ala Asn Tyr Phe Thr
1               5

<210> 40
<211> 318
<212> DNA
<213> Mus musculus

<400> 40
gacatccaga tgacccagtc cccctcctcc ctctccgcct ccctcggcga gcgcgtctcc 60
ctcacctgcc gcgcctccca ggagatctcc ggctacctca tctggctcca gcagaagccc 120
gacggcacca tcaagcgcct catctacgcc acctccaccc tcgactccgg cgtccccaag 180
cgcttctccg gctcccgctc cggctccgac tacaccctca ccatctcctc cctcgagtcc 240
gaggacttcg ccgactacta ctgcctccag tacgccaact acttcacctt cggcgccggc 300
accaagctcg agctcaag 318

<210> 41
<211> 119
<212> PRT
<213> Mus musculus

<400> 41
Gln Val Gln Leu Asn Gln Ser Gly Pro Gly Leu Val Gln Pro Ser Gln
1               5                   10                  15
Ser Leu Ser Ile Thr Cys Thr Val Ser Gly Phe Ser Leu Thr Ser Tyr
            20                  25                  30
Gly Val His Trp Leu Arg Gln Ser Ala Gly Lys Gly Leu Glu Trp Leu
        35                  40                  45
Gly Val Ile Trp Arg Gly Gly Ser Thr Asp Tyr Asn Val Gly Phe Met
    50                  55                  60
Ser Arg Leu Thr Ile Ser Lys Asp Asn Ser Lys Ser Gln Val Phe Phe
65                  70                  75                  80
Lys Met Asn Ser Leu Gln Gly Asp Asp Thr Ala Ile Tyr Phe Cys Ala
                85                  90                  95
Lys Ser Met Ile Thr Thr Gly Tyr Thr Met Asp Tyr Trp Gly Gln Gly
            100                 105                 110
Thr Ser Val Thr Val Ser Ser
        115

<210> 42
<211> 6
<212> PRT
<213> Mus musculus

<400> 42
Thr Ser Tyr Gly Val His
1               5

<210> 43
<211> 16
<212> PRT
<213> Mus musculus

<400> 43
Val Ile Trp Arg Gly Gly Ser Thr Asp Tyr Asn Val Gly Phe Met Ser
1               5                   10                  15

<210> 44
<211> 11
<212> PRT
<213> Mus musculus

<400> 44
Ser Met Ile Thr Thr Gly Tyr Thr Met Asp Tyr
1 5 10

<210> 45
<211> 357
<212> DNA
<213> Mus musculus

<400> 45
caggtgcagc tgaaccagtc aggacctggc ctagtgcagc cctcacagag cctgtccata 60
acctgcacag tctctggttt ctcattaact agctatggtg tacattggct tcgccagtct 120
gcaggaaagg gtctggagtg gctgggagtg atatggagag gtggaagcac agactacaat 180
gtaggtttca tgtccagact gaccattagt aaggacaatt ccaagagcca agttttcttt 240
aaaatgaaca gtctgcaagg tgatgacact gccatatact tctgtgccaa aagtatgatt 300
acgacgggct atactatgga ctactggggt caaggaacct cagtcaccgt ctcctca 357

<210> 46
<211> 107
<212> PRT
<213> Mus musculus

<400> 46
Ala Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Lys Ser Gly Asn Ala Pro Arg Leu Leu Ile
35 40 45
Ser Gly Ser Thr Ser Leu Glu Thr Gly Val Pro Ser Arg Phe Ser Gly
50 55 60
Ser Gly Ser Gly Lys Asp Tyr Thr Leu Ser Ile Ile Ser Leu Gln Thr
65 70 75 80
Glu Asp Val Ala Thr Tyr Tyr Cys Gln Gln Phe Trp Ser Thr Pro Trp
85 90 95
Thr Phe Gly Gly Gly Thr Lys Leu Glu Ile Lys
100 105

<210> 47
<211> 11
<212> PRT
<213> Mus musculus

<400> 47
Lys Ala Ser Glu Asp Ile Tyr Asn Arg Leu Ala
1 5 10

<210> 48
<211> 7
<212> PRT
<213> Mus musculus

<400> 48
Gly Ser Thr Ser Leu Glu Thr
1 5

<210> 49
<211> 9
<212> PRT
<213> Mus musculus

<400> 49

```
Gln Gln Phe Trp Ser Thr Pro Trp Thr
1               5

<210>  50
<211>  321
<212>  DNA
<213>  Mus musculus

<400>  50
gccatccaga tgacacaatc ttcatcctcc ttttctgtat ctctaggaga cagagtcacc        60
attacttgca aggcaagtga ggacatatat aatcggttag cttggtatca gcagaaatca       120
ggaaatgctc caaggctctt aatatctggt tcaaccagtt tggaaactgg ggttccttca       180
agattcagtg gcagtggatc tggaaaggat tacactctca gcattatcag tcttcagact       240
gaagatgttg ctacttatta ctgtcaacag ttttggagta ctccgtggac gttcggtgga       300
ggcaccaagc tggaaataaa a                                                 321

<210>  51
<211>  119
<212>  PRT
<213>  Mus musculus

<400>  51
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Ser Tyr
            20                  25                  30
Gly Val His Trp Val Arg Gln Ser Pro Gly Lys Gly Leu Glu Trp Leu
        35                  40                  45
Gly Val Met Trp Arg Gly Gly Ser Thr Asp Tyr Asn Ala Pro Phe Met
    50                  55                  60
Ser Arg Leu Ser Ile Thr Lys Asp Asn Ser Lys Ser Gln Val Phe Phe
65                  70                  75                  80
Lys Met Asn Ser Leu Gln Ala Asp Asp Thr Ala Ile Tyr Tyr Cys Ala
                85                  90                  95
Lys Ser Leu Ile Thr Thr Gly Tyr Ala Met Asp Tyr Trp Gly Gln Gly
            100                 105                 110
Thr Ser Val Thr Val Ser Ser
        115

<210>  52
<211>  6
<212>  PRT
<213>  Mus musculus

<400>  52
Thr Ser Tyr Gly Val His
1               5

<210>  53
<211>  16
<212>  PRT
<213>  Mus musculus

<400>  53
Val Met Trp Arg Gly Gly Ser Thr Asp Tyr Asn Ala Pro Phe Met Ser
1               5                   10                  15

<210>  54
<211>  11
<212>  PRT
<213>  Mus musculus

<400>  54
```

Ser Leu Ile Thr Thr Gly Tyr Ala Met Asp Tyr
1               5                   10

<210>  55
<211>  357
<212>  DNA
<213>  Mus musculus

<400>  55
caggtccagc tgaagcagtc aggacctggc ctagtgcagc cctcacagag cctgtccata     60
acctgcacag tctctggttt ctcattaact agctatggtg tacactgggt tcgccagtct    120
ccaggaaagg gtctggagtg gctgggagtg atgtggagag gtggaagcac agactacaat    180
gcacctttca tgtccagact gagcatcacc aaggacaact ccaagagcca agttttcttt    240
aaaatgaaca gtctgcaagc tgatgacact gccatatact actgtgccaa aagtctgatt    300
acgactgggt atgctatgga ctactggggt caaggaacct cagtcaccgt ctcctca       357

<210>  56
<211>  107
<212>  PRT
<213>  Mus musculus

<400>  56
Asp Ile Gln Met Thr Gln Ser Ser Ser Ser Phe Ser Val Ser Leu Gly
1               5                   10                  15
Asp Arg Val Thr Ile Thr Cys Lys Ala Ser Glu Asp Ile Tyr Asn Arg
            20                  25                  30
Leu Ala Trp Tyr Gln Gln Lys Pro Gly Asn Ala Pro Arg Leu Leu Ile
        35                  40                  45
Ser Gly Ala Thr Ser Leu Glu Thr Gly Val Pro Ser Arg Phe Ser Gly
    50                  55                  60
Ser Gly Ser Gly Lys Asp Tyr Thr Leu Ser Ile Thr Ser Leu Gln Thr
65                  70                  75                  80
Glu Asp Val Ala Thr Tyr Tyr Cys Gln Gln Tyr Trp Ser Asn Pro Tyr
                85                  90                  95
Thr Phe Gly Gly Gly Thr Lys Leu Glu Ile Lys
            100                 105

<210>  57
<211>  11
<212>  PRT
<213>  Mus musculus

<400>  57
Lys Ala Ser Glu Asp Ile Tyr Asn Arg Leu Ala
1               5                   10

<210>  58
<211>  7
<212>  PRT
<213>  Mus musculus

<400>  58
Gly Ala Thr Ser Leu Glu Thr
1               5

<210>  59
<211>  9
<212>  PRT
<213>  Mus musculus

<400>  59
Gln Gln Tyr Trp Ser Asn Pro Tyr Thr
1               5

<210> 60
<211> 321
<212> DNA
<213> Mus musculus

<400> 60
gacatccaga tgacacaatc ttcatcctcc ttttctgtat ctctaggaga cagagtcacc 60
attacttgca aggcaagtga ggacatatat aatcggttag cctggtatca gcagaaacca 120
ggaaatgctc ctaggctctt aatatctggt gcaaccagtt tggaaactgg ggttccttca 180
agattcagtg gcagtggatc tggaaaggat tacactctca gcattaccag tcttcagact 240
gaagatgttg ctacttatta ctgtcaacag tattggagta atccgtacac gttcggaggg 300
gggaccaagc tggaaataaa a 321

<210> 61
<211> 120
<212> PRT
<213> Mus musculus

<400> 61
Gln Ala Tyr Leu Gln Gln Ser Gly Ala Glu Leu Val Arg Ser Gly Ala
1 5 10 15
Ser Val Lys Met Ser Cys Lys Ala Ser Gly Tyr Thr Phe Ser Ser Tyr
20 25 30
Asn Met His Trp Val Lys Gln Thr Pro Gly Gln Gly Leu Glu Trp Ile
35 40 45
Gly Tyr Val Tyr Pro Gly Asn Gly Gly Thr Asn Tyr Asn Gln Lys Phe
50 55 60
Lys Gly Lys Ala Thr Leu Thr Ala Asp Thr Ser Ser Ser Thr Ala Tyr
65 70 75 80
Met Gln Ile Ser Ser Leu Thr Ser Glu Glu Ser Ala Val Tyr Phe Cys
85 90 95
Ala Arg Pro Glu Ser Ser Phe His Pro Trp Phe Ala Tyr Trp Gly Gln
100 105 110
Gly Thr Leu Val Thr Val Ala Pro
115 120

<210> 62
<211> 6
<212> PRT
<213> Mus musculus

<400> 62
Ser Ser Tyr Asn Met His
1 5

<210> 63
<211> 17
<212> PRT
<213> Mus musculus

<400> 63
Tyr Val Tyr Pro Gly Asn Gly Gly Thr Asn Tyr Asn Gln Lys Phe Lys
1 5 10 15
Gly

<210> 64
<211> 11
<212> PRT
<213> Mus musculus

<400> 64

Pro Glu Ser Ser Phe His Pro Trp Phe Ala Tyr
1 5 10

<210> 65
<211> 360
<212> DNA
<213> Mus musculus

<400> 65
caggcttatc tacagcagtc tggggctgag ctggtgaggt ctggggcctc agtgaagatg 60
tcctgcaagg cttctggcta cacattttcc agttacaata tgcactgggt aaagcagaca 120
cctggacagg gcctggaatg gattggatat gtttatcctg gaaatggtgg tacgaactac 180
aatcagaagt tcaagggcaa ggccacattg actgcagaca catcctccag cacagcctac 240
atgcagatca gcagcctgac atctgaagag tctgcggtct atttctgtgc aagaccagag 300
agtagtttcc acccctggtt tgcttactgg ggccaaggga ctctggtcac tgtcgcgcca 360

<210> 66
<211> 111
<212> PRT
<213> Mus musculus

<400> 66
Asn Ile Val Leu Thr Gln Ser Pro Ala Ser Leu Ala Val Ser Leu Gly
1 5 10 15
Gln Arg Ala Thr Ile Ser Cys Arg Ala Ser Glu Ser Val Asn Ile Tyr
20 25 30
Gly Asn Ile Phe Met Tyr Trp Tyr Gln Gln Lys Pro Gly Gln Pro Pro
35 40 45
Lys Leu Leu Ile Tyr Leu Ala Ser Asn Leu Ala Ser Gly Val Pro Ala
50 55 60
Arg Phe Ser Gly Ser Gly Ser Arg Thr Gly Phe Thr Leu Thr Ile Asp
65 70 75 80
Pro Val Glu Ala Asp Asp Ala Ala Thr Tyr Tyr Cys Gln Gln Asn Ala
85 90 95
Lys Asp Pro Trp Thr Phe Gly Gly Gly Thr Lys Leu Glu Ile Lys
100 105 110

<210> 67
<211> 15
<212> PRT
<213> Mus musculus

<400> 67
Arg Ala Ser Glu Ser Val Asn Ile Tyr Gly Asn Ile Phe Met Tyr
1 5 10 15

<210> 68
<211> 7
<212> PRT
<213> Mus musculus

<400> 68
Leu Ala Ser Asn Leu Ala Ser
1 5

<210> 69
<211> 9
<212> PRT
<213> Mus musculus

<400> 69
Gln Gln Asn Ala Lys Asp Pro Trp Thr
1 5

<210> 70
<211> 333
<212> DNA
<213> Mus musculus

<400> 70
aacattgtgc tgacccaatc tccagcttct ttggctgtgt ctctagggca gagggccacc 60
atatcctgca gagccagtga aagtgttaat atttatggca atatttttat gtactggtac 120
cagcagaaac caggacagcc acccaaactc ctcatctatc ttgcatccaa cctagcatct 180
ggggtccctg ccaggttcag tggcagtggg tctaggacag gcttcaccct caccattgat 240
cctgtggagg ctgatgatgc tgcaacctat tactgtcagc aaaatgctaa ggatccgtgg 300
acgttcggtg gaggcaccaa gctggaaatc aaa 333

<210> 71
<211> 119
<212> PRT
<213> Mus musculus

<400> 71
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Ser Tyr
            20                  25                  30
Gly Ile His Trp Val Arg Gln Ser Pro Gly Lys Gly Leu Glu Trp Leu
        35                  40                  45
Gly Val Met Trp Arg Gly Gly Ser Thr Asp Tyr Asn Ala Pro Phe Met
    50                  55                  60
Ser Arg Leu Ser Ile Thr Lys Asp Asn Ser Lys Ser Gln Val Phe Phe
65                  70                  75                  80
Lys Met Asn Ser Leu Gln Ala Asp Asp Thr Ala Ile Tyr Tyr Cys Ala
                85                  90                  95
Lys Ser Leu Ile Thr Thr Gly Tyr Ala Met Asp Phe Trp Gly Gln Gly
            100                 105                 110
Thr Ser Val Thr Val Ser Ser
        115

<210> 72
<211> 6
<212> PRT
<213> Mus musculus

<400> 72
Thr Ser Tyr Gly Ile His
1               5

<210> 73
<211> 16
<212> PRT
<213> Mus musculus

<400> 73
Val Met Trp Arg Gly Gly Ser Thr Asp Tyr Asn Ala Pro Phe Met Ser
1               5                   10                  15

<210> 74
<211> 11
<212> PRT
<213> Mus musculus

<400> 74
Ser Leu Ile Thr Thr Gly Tyr Ala Met Asp Phe
1               5                   10

<210> 75
<211> 357
<212> DNA
<213> Mus musculus

<400> 75
caggtccagc tgaagcagtc aggacctggc ctagtgcagc cctcacagag cctgtccata 60
acctgcacag tctctggttt ctcattaact agctatggta tacactgggt tcgccagtct 120
ccaggaaagg gtctggagtg gctgggagtg atgtggagag gtggaagcac agactacaat 180
gcacctttta tgtccagact gagcatcacc aaggacaact ccaagagcca agttttcttt 240
aaaatgaaca gtctgcaagc tgatgacact gccatatatt actgtgccaa aagtctgatt 300
acgactgggt atgctatgga cttctggggt caaggaacct cagtcaccgt ctcctca 357

<210> 76
<211> 107
<212> PRT
<213> Mus musculus

<400> 76
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Lys Pro Gly Asn Ala Pro Arg Leu Leu Ile
35 40 45
Ser Gly Ala Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Tyr Trp Ser Asn Pro Tyr
85 90 95
Thr Phe Gly Gly Gly Thr Lys Leu Glu Ile Lys
100 105

<210> 77
<211> 11
<212> PRT
<213> Mus musculus

<400> 77
Lys Ala Ser Glu Asp Ile Tyr Asn Arg Leu Ala
1 5 10

<210> 78
<211> 7
<212> PRT
<213> Mus musculus

<400> 78
Gly Ala Thr Ser Leu Glu Thr
1 5

<210> 79
<211> 9
<212> PRT
<213> Mus musculus

<400> 79
Gln Gln Tyr Trp Ser Asn Pro Tyr Thr
1 5

<210> 80

<211> 321
<212> DNA
<213> Mus musculus

<400> 80
gacatccaga tgacacaatc ttcatcctcc ttttctgtat ctctaggaga cagagtcacc 60
attacttgca aggcaagtga ggacatatat aatcggttag cctggtatca gcagaaacca 120
ggaaatgctc ctaggctctt aatatctggt gcaaccagtt tggaaactgg ggttccttca 180
agattcagtg gcagtggatc tggaaaggat tacactctca gcattaccag tcttcagact 240
gaagatgttg ctacttatta ctgtcaacag tattggagta atccgtacac gttcggaggg 300
gggaccaagc tggaaataaa a 321

<210> 81
<211> 119
<212> PRT
<213> Mus musculus

<400> 81
Gln Val Gln Leu Lys Gln Ser Gly Pro Ser Pro Val Gln Pro Ser Gln
1 5 10 15
Ser Leu Ser Ile Thr Cys Thr Val Ser Gly Phe Ser Leu Thr Ser Tyr
20 25 30
Gly Val His Trp Val Arg Gln Ser Pro Gly Lys Gly Leu Glu Trp Leu
35 40 45
Gly Val Ile Trp Arg Gly Gly Ser Thr Asp Tyr Asn Ala Val Phe Met
50 55 60
Ser Arg Leu Ser Ile Thr Lys Asp Asn Ser Lys Ser Gln Val Phe Phe
65 70 75 80
Lys Met Asn Ser Leu Gln Thr Asp Asp Thr Ala Ile Tyr Tyr Cys Ala
85 90 95
Lys Ser Leu Val Thr Thr Gly Tyr Thr Met Asp Tyr Trp Gly Gln Gly
100 105 110
Thr Ser Val Thr Val Ser Ser
115

<210> 82
<211> 6
<212> PRT
<213> Mus musculus

<400> 82
Thr Ser Tyr Gly Val His
1 5

<210> 83
<211> 16
<212> PRT
<213> Mus musculus

<400> 83
Val Ile Trp Arg Gly Gly Ser Thr Asp Tyr Asn Ala Val Phe Met Ser
1 5 10 15

<210> 84
<211> 11
<212> PRT
<213> Mus musculus

<400> 84
Ser Leu Val Thr Thr Gly Tyr Thr Met Asp Tyr
1 5 10

<210> 85

<211> 357
<212> DNA
<213> Mus musculus

<400> 85
caggtccagc tgaagcagtc aggacctagc ccagtgcagc cctcacagag cctgtccata 60
acctgcacag tctctggttt ctcattaacg agctatgggg tacactgggt tcgccagtct 120
ccaggaaagg gtctggagtg gctgggagtg atatggagag gtggaagtac agactacaat 180
gcagttttca tgtccagact gagcatcacc aaggacaact ccaagagcca agttttcttt 240
aaaatgaaca gtctgcaaac tgatgacact gccatatact actgtgccaa aagtcttgtt 300
actacgggct atactatgga ctactggggt caaggaacct cagtcaccgt ctcctca 357

<210> 86
<211> 107
<212> PRT
<213> Mus musculus

<400> 86
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Arg Pro Gly Asn Ala Pro Arg Leu Leu Ile
35 40 45
Ser Gly Thr Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Tyr Trp Ser Ile Pro Trp
85 90 95
Thr Phe Gly Gly Gly Thr Lys Leu Glu Ile Lys
100 105

<210> 87
<211> 11
<212> PRT
<213> Mus musculus

<400> 87
Lys Ala Ser Glu Asp Ile Tyr Asn Arg Leu Ala
1 5 10

<210> 88
<211> 7
<212> PRT
<213> Mus musculus

<400> 88
Gly Thr Thr Ser Leu Glu Thr
1 5

<210> 89
<211> 9
<212> PRT
<213> Mus musculus

<400> 89
Gln Gln Tyr Trp Ser Ile Pro Trp Thr
1 5

<210> 90
<211> 321
<212> DNA

<213> Mus musculus

<400> 90
gacatccaga tgacacagtc ttcatcctcc ttttctgtat ctctaggaga cagagtcacc 60
attacttgca aggcaagtga ggacatatat aatcggttag cctggtatca gcagagacca 120
ggaaatgctc ctaggctctt aatatctggt acaaccagtt tggaaactgg ggttccttca 180
agattcagtg gcagtggatc tggaaaggat tacactctca gcattaccag tcttcagact 240
gaagatgttg ctacttatta ctgtcaacag tattggagta ttccgtggac gttcggtgga 300
ggcaccaagc tggaaataaa a 321

<210> 91
<211> 119
<212> PRT
<213> Mus musculus

<400> 91
Gln Val Gln Leu Lys Gln Ser Gly Pro Gly Leu Val Gln Pro Ser Gln
1 5 10 15
Ser Leu Ser Ile Ile Cys Thr Val Ser Gly Phe Ser Leu Ser Ser Tyr
20 25 30
Gly Ile His Trp Val Arg Gln Ser Pro Gly Lys Gly Leu Glu Trp Leu
35 40 45
Gly Val Ile Trp Arg Gly Gly Asn Thr Asp Tyr Ser Val Thr Phe Met
50 55 60
Ser Arg Leu Ser Ile Thr Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Lys Ser Leu Ile Thr Thr Gly Tyr Ala Met Asp Tyr Trp Gly Gln Gly
100 105 110
Thr Ser Val Thr Val Ser Ser
115

<210> 92
<211> 6
<212> PRT
<213> Mus musculus

<400> 92
Ser Ser Tyr Gly Ile His
1 5

<210> 93
<211> 16
<212> PRT
<213> Mus musculus

<400> 93
Val Ile Trp Arg Gly Gly Asn Thr Asp Tyr Ser Val Thr Phe Met Ser
1 5 10 15

<210> 94
<211> 11
<212> PRT
<213> Mus musculus

<400> 94
Ser Leu Ile Thr Thr Gly Tyr Ala Met Asp Tyr
1 5 10

<210> 95
<211> 357
<212> DNA

<213> Mus musculus

<400> 95
caggtccagc tgaagcagtc aggacctggc ctagtgcagc cctcacagag cctgtccata 60
atctgcacag tctctggttt ctccttaagt agttatggta ttcactgggt tcgccagtct 120
ccaggaaagg gtctggagtg gctgggagtg atatggagag gtggaaacac agactacagt 180
gtaactttca tgtccagact gagcatcacc aaggacaact ccaagagcca agttttcttt 240
aaaatgaaca gtctgcaagc tgatgacact gccatatatt attgtgccaa aagtttgatt 300
acgacagggt atgctatgga ctactggggt caaggaacct cagtcaccgt ctcctca 357

<210> 96
<211> 107
<212> PRT
<213> Mus musculus

<400> 96
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Lys Pro Gly Thr Thr Pro Arg Leu Leu Ile
35 40 45
Ser Gly Ala Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Gly Thr Tyr Tyr Cys Gln Gln Tyr Trp Ser Asn Pro Arg
85 90 95
Thr Phe Gly Gly Gly Thr Lys Leu Glu Ile Lys
100 105

<210> 97
<211> 11
<212> PRT
<213> Mus musculus

<400> 97
Lys Ala Ser Glu Asp Ile Tyr Asn Arg Leu Ala
1 5 10

<210> 98
<211> 7
<212> PRT
<213> Mus musculus

<400> 98
Gly Ala Thr Ser Leu Glu Thr
1 5

<210> 99
<211> 9
<212> PRT
<213> Mus musculus

<400> 99
Gln Gln Tyr Trp Ser Asn Pro Arg Thr
1 5

<210> 100
<211> 321
<212> DNA
<213> Mus musculus

<400> 100
gacatccaga tgacacaatc ttcatcctcc ttttctgttt ctctaggaga cagagtcacc 60
attacttgca aggcaagtga agacatatat aatcggttag cctggtatca gcagaaacca 120
ggaactactc ctcggctctt aatatctggt gcaaccagtt tggaaactgg ggttccttca 180
agattcagtg gcagtggatc tggaaaagat tacactctca gcattaccag tcttcagact 240
gaagatgttg gtacttatta ctgtcaacag tattggagta atccgcggac gttcggtgga 300
ggcaccaagc tggaaataaa a 321

<210> 101
<211> 119
<212> PRT
<213> Mus musculus

<400> 101
Gln Val Gln Leu Lys Gln Ser Gly Pro Gly Leu Val Gln Pro Ser Gln
1 5 10 15
Thr Leu Ser Ile Thr Cys Thr Val Ser Gly Phe Ser Leu Thr Ser Tyr
20 25 30
Gly Val His Trp Val Arg Gln Ser Pro Gly Lys Gly Leu Glu Trp Leu
35 40 45
Gly Val Ile Trp Arg Gly Gly Gly Thr Glu Tyr Asn Ala Ala Phe Met
50 55 60
Ser Arg Leu Ser Ile Thr Lys Asp Asn Ser Lys Ser Gln Val Phe Leu
65 70 75 80
Lys Met Asn Ser Leu Gln Ala Asp Asp Thr Ala Ile Tyr Tyr Cys Ala
85 90 95
Lys Ser Met Ile Thr Thr Gly Tyr Ala Met Asp Phe Trp Gly Gln Gly
100 105 110
Thr Ser Val Thr Val Ser Ser
115

<210> 102
<211> 6
<212> PRT
<213> Mus musculus

<400> 102
Thr Ser Tyr Gly Val His
1 5

<210> 103
<211> 16
<212> PRT
<213> Mus musculus

<400> 103
Val Ile Trp Arg Gly Gly Gly Thr Glu Tyr Asn Ala Ala Phe Met Ser
1 5 10 15

<210> 104
<211> 11
<212> PRT
<213> Mus musculus

<400> 104
Ser Met Ile Thr Thr Gly Tyr Ala Met Asp Phe
1 5 10

<210> 105
<211> 357
<212> DNA
<213> Mus musculus

<400> 105
caggtccagc tgaagcagtc aggacctggc ctagtgcagc cctcacagac cctgtccata 60
acctgcacag tctctggttt ctcattaact agctatggtg tacactgggt tcgccagtct 120
ccaggaaagg gtctggagtg gctgggagtg atatggagag gtggaggcac agagtacaat 180
gcagctttca tgtccagact gagcatcacc aaggacaact ccaagagcca agttttctta 240
aagatgaaca gtctgcaagc tgatgacact gccatatact actgtgccaa gtcgatgatt 300
acgacaggtt atgctatgga cttctggggt caaggaacct cagtcaccgt ctcctca 357

<210> 106
<211> 107
<212> PRT
<213> Mus musculus

<400> 106
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Lys Arg
20 25 30
Leu Ala Trp Tyr Gln Gln Lys Pro Gly Asn Ala Pro Arg Leu Leu Ile
35 40 45
Ser Gly Ala Thr Ser Leu Glu Thr Gly Ile Pro Ser Arg Phe Ser Gly
50 55 60
Ser Gly Ser Gly Lys Asp Tyr Thr Leu Ser Leu Thr Ser Leu Gln Thr
65 70 75 80
Glu Asp Val Ala Thr Tyr Tyr Cys Gln Gln Tyr Trp Ser Ser Pro Arg
85 90 95
Thr Phe Gly Gly Gly Ala Lys Leu Glu Ile Lys
100 105

<210> 107
<211> 11
<212> PRT
<213> Mus musculus

<400> 107
Lys Ala Ser Glu Asp Ile Tyr Lys Arg Leu Ala
1 5 10

<210> 108
<211> 7
<212> PRT
<213> Mus musculus

<400> 108
Gly Ala Thr Ser Leu Glu Thr
1 5

<210> 109
<211> 9
<212> PRT
<213> Mus musculus

<400> 109
Gln Gln Tyr Trp Ser Ser Pro Arg Thr
1 5

<210> 110
<211> 321
<212> DNA
<213> Mus musculus

<400> 110
gacatccaga tgacacaatc ttcatcctcc ttttctgtat ctctaggaga cagagtcacc 60

```
atcacttgca aggcaagtga ggacatatat aagcggttag cctggtatca gcagaaacca      120
ggaaatgctc ctaggctctt aatatctggt gcaaccagtt tggaaactgg gattccttca      180
agattcagtg gcagtggatc tggaaaggat tacactctca gccttaccag tcttcagact      240
gaagatgttg ctacttatta ctgtcaacag tattggagtt ctcctcggac gttcggtgga      300
ggcgccaagc tggaaataaa a                                                321

<210>  111
<211>  119
<212>  PRT
<213>  Mus musculus

<400>  111
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Thr Tyr
            20                  25                  30
Gly Ile His Trp Ile Arg Gln Ser Pro Gly Arg Gly Leu Glu Trp Leu
        35                  40                  45
Gly Val Ile Trp Arg Gly Gly Ser Thr Asp Tyr Asn Ala Ala Phe Met
    50                  55                  60
Ser Arg Leu Ser Phe Thr Lys Asp Asn Ser Lys Ser Gln Val Phe Phe
65                  70                  75                  80
Lys Met Asn Ser Leu Gln Ala Asp Asp Thr Ala Ile Tyr Tyr Cys Ala
                85                  90                  95
Lys Ser Met Ile Thr Thr Gly Phe Thr Met Asp Tyr Trp Gly Gln Gly
            100                 105                 110
Thr Ser Val Thr Val Ser Ser
        115

<210>  112
<211>  6
<212>  PRT
<213>  Mus musculus

<400>  112
Thr Thr Tyr Gly Ile His
1               5

<210>  113
<211>  16
<212>  PRT
<213>  Mus musculus

<400>  113
Val Ile Trp Arg Gly Gly Ser Thr Asp Tyr Asn Ala Ala Phe Met Ser
1               5                   10                  15

<210>  114
<211>  11
<212>  PRT
<213>  Mus musculus

<400>  114
Ser Met Ile Thr Thr Gly Phe Thr Met Asp Tyr
1               5                   10

<210>  115
<211>  358
<212>  DNA
<213>  Mus musculus

<400>  115
caggtccagc tgaagcagtc aggacctggc ctagtgcagc cctcacagag cctgtccata       60
```

acctgcacag tctctggttt ctcattgact acctatggta tacactggat tcgccagtct 120
cctggaaggg gtctggagtg gctgggagtg atttggagag gtggaagcac agactacaat 180
gcagctttca tgtccagact gagcttcacc aaggacaact ccaagagcca agttttcttt 240
aaaatgaaca gtctgcaagc tgatgacact gccatatact actgtgccaa aagtatgatt 300
acgactggat ttactatgga ctactggggt caaggaacct cagtcaccgt ctcctcag 358

<210> 116
<211> 107
<212> PRT
<213> Mus musculus

<400> 116
Asp Ile Gln Met Thr Gln Ser Ser Ser Ser Phe Ser Val Ser Leu Gly
1 5 10 15
Asp Arg Val Thr Ile Thr Cys Arg Ala Ser Glu Asp Ile Tyr Asn Arg
20 25 30
Leu Ala Trp Tyr Gln Gln Lys Pro Gly Asn Ala Pro Arg Leu Leu Ile
35 40 45
Ser Gly Ala Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Phe Trp Ser Thr Pro Tyr
85 90 95
Thr Phe Gly Gly Gly Thr Lys Leu Glu Ile Lys
100 105

<210> 117
<211> 11
<212> PRT
<213> Mus musculus

<400> 117
Arg Ala Ser Glu Asp Ile Tyr Asn Arg Leu Ala
1 5 10

<210> 118
<211> 7
<212> PRT
<213> Mus musculus

<400> 118
Gly Ala Thr Ser Leu Glu Thr
1 5

<210> 119
<211> 9
<212> PRT
<213> Mus musculus

<400> 119
Gln Gln Phe Trp Ser Thr Pro Tyr Thr
1 5

<210> 120
<211> 321
<212> DNA
<213> Mus musculus

<400> 120
gacatccaga tgacacaatc ttcatcctcc ttttctgtat ctctaggaga cagagtcacc 60
attacttgca gggcaagtga ggacatatat aaccggttag cctggtatca gcagaaacca 120
ggaaatgctc ctaggctctt aatatctggt gcaaccagtt tggaaactgg ggttccttca 180

agattcagtg gcagtggatc tggaaaggat tacactctca gcattaccag tcttcagact 240
gaagatgttg ctacttatta ttgtcaacag ttttggagta ctccgtacac gttcggaggg 300
gggaccaagc tggaaataaa a 321

<210> 121
<211> 119
<212> PRT
<213> Mus musculus

<400> 121
Gln Val Gln Leu Lys Gln Ser Gly Pro Gly Leu Val Gln Pro Ser Gln
1 5 10 15
Ser Leu Ser Ile Ser Cys Ser Val Ser Gly Phe Ser Leu Thr Ser Tyr
20 25 30
Gly Val His Trp Val Arg Gln Ser Pro Gly Lys Gly Leu Glu Trp Leu
35 40 45
Gly Val Ile Trp Arg Gly Gly Ser Thr Asp Tyr Asn Ala Ala Phe Ser
50 55 60
Ser Arg Leu Ser Ile Thr Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Lys Ser Met Ile Thr Thr Gly Arg Tyr Phe Asp Val Trp Gly Ala Gly
100 105 110
Thr Thr Val Thr Val Ser Ser
115

<210> 122
<211> 6
<212> PRT
<213> Mus musculus

<400> 122
Thr Ser Tyr Gly Val His
1 5

<210> 123
<211> 16
<212> PRT
<213> Mus musculus

<400> 123
Val Ile Trp Arg Gly Gly Ser Thr Asp Tyr Asn Ala Ala Phe Ser Ser
1 5 10 15

<210> 124
<211> 11
<212> PRT
<213> Mus musculus

<400> 124
Ser Met Ile Thr Thr Gly Arg Tyr Phe Asp Val
1 5 10

<210> 125
<211> 357
<212> DNA
<213> Mus musculus

<400> 125
caggtccagc tgaagcagtc aggacctggc ctagtgcagc cctcacagag cctgtccata 60
tcctgctcag tctctggttt ctcattaact agttatggtg tacactgggt tcgccagtct 120
ccaggaaagg gtctggagtg gctgggagtg atatggagag gaggaagcac agactacaat 180

```
gcagctttct cgtccagact gagcatcacc aaggacaact ccaagagcca agttttcttt      240
aaaatgaaca gtctgcaagc tgatgacact gccatatatt actgtgccaa atctatgatt      300
acgacgggga ggtacttcga tgtctggggc gcagggacca cggtcaccgt ctcctca         357

<210>  126
<211>  107
<212>  PRT
<213>  Mus musculus

<400>  126
Asp Ile Gln Met Thr Gln Ser Ser Ser Ser Phe Ser Val Ser Leu Gly
1               5                   10                  15
Asp Arg Val Thr Ile Thr Cys Lys Ala Ser Glu Asp Ile Tyr Asn Arg
            20                  25                  30
Leu Ala Trp Tyr Gln Gln Lys Pro Gly Asn Ala Pro Arg Leu Leu Ile
        35                  40                  45
Ser Gly Ala Ala Ser Leu Glu Thr Gly Ile Pro Ser Arg Phe Ser Gly
    50                  55                  60
Ser Gly Ser Gly Lys Asn Tyr Thr Leu Ser Ile Thr Ser Leu Gln Thr
65                  70                  75                  80
Glu Asp Val Ala Thr Tyr Tyr Cys Gln Gln Tyr Trp Ser Thr Pro Trp
                85                  90                  95
Thr Phe Gly Gly Gly Thr Lys Leu Glu Ile Lys
            100                 105

<210>  127
<211>  11
<212>  PRT
<213>  Mus musculus

<400>  127
Lys Ala Ser Glu Asp Ile Tyr Asn Arg Leu Ala
1               5                   10

<210>  128
<211>  7
<212>  PRT
<213>  Mus musculus

<400>  128
Gly Ala Ala Ser Leu Glu Thr
1               5

<210>  129
<211>  9
<212>  PRT
<213>  Mus musculus

<400>  129
Gln Gln Tyr Trp Ser Thr Pro Trp Thr
1               5

<210>  130
<211>  321
<212>  DNA
<213>  Mus musculus

<400>  130
gacatccaga tgacacaatc ttcatcctcc ttttctgtat ctctcggaga cagagtcacc       60
attacttgca aggcaagtga ggacatatat aatcggttag cctggtatca gcagaaacca      120
ggaaatgctc ctaggctctt aatatctggt gcagccagtt tggaaactgg gattccttca      180
agattcagtg gcagtggatc tggaaagaat tacactctca gcattaccag tcttcagact      240
gaagatgttg ctacttatta ctgtcaacag tattggagta ctccgtggac gttcggtgga      300
```

ggcaccaagc tggaaataaa a 321

<210> 131
<211> 119
<212> PRT
<213> Mus musculus

<400> 131
Gln Val Gln Leu Lys Gln Ser Gly Pro Gly Leu Val Gln Pro Ser Gln
1 5 10 15
Ser Leu Ser Ile Thr Cys Ser Val Ser Gly Phe Ser Leu Thr Ser Tyr
20 25 30
Gly Val His Trp Val Arg Gln Ser Pro Gly Lys Gly Leu Glu Trp Leu
35 40 45
Gly Val Ile Trp Arg Gly Gly Ser Thr Asp Tyr Asn Ala Ala Phe Met
50 55 60
Ser Arg Leu Ser Ile Thr Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Lys Ser Met Ile Thr Thr Gly Arg Tyr Phe Asp Val Trp Gly Ala Gly
100 105 110
Thr Thr Val Thr Val Ser Ser
115

<210> 132
<211> 6
<212> PRT
<213> Mus musculus

<400> 132
Thr Ser Tyr Gly Val His
1 5

<210> 133
<211> 16
<212> PRT
<213> Mus musculus

<400> 133
Val Ile Trp Arg Gly Gly Ser Thr Asp Tyr Asn Ala Ala Phe Met Ser
1 5 10 15

<210> 134
<211> 11
<212> PRT
<213> Mus musculus

<400> 134
Ser Met Ile Thr Thr Gly Arg Tyr Phe Asp Val
1 5 10

<210> 135
<211> 357
<212> DNA
<213> Mus musculus

<400> 135
caggtccagc tgaagcagtc aggacctggc ctagtgcagc cctcacagag cctgtccata 60
acctgctcag tctctggttt ctcattaact agctatggtg tacactgggt tcgccagtct 120
ccaggaaagg gtctggagtg gctgggagtg atatggagag gaggaagcac agactacaat 180
gcagctttca tgtccagact gagcatcacc aaggacaact ccaagagcca agttttcttt 240
aaaatgaaca gtctgcaagc tgatgacact gccatatact actgtgccaa atctatgatt 300

acgacgggga ggtacttcga tgtctggggc gcagggacca cggtcaccgt ctcctca 357

<210> 136
<211> 107
<212> PRT
<213> Mus musculus

<400> 136
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Arg Pro Gly Asn Ala Pro Arg Leu Leu Ile
35 40 45
Ser Gly Ala Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Tyr Trp Ser Ala Pro Trp
85 90 95
Thr Phe Gly Gly Gly Thr Lys Leu Glu Ile Lys
100 105

<210> 137
<211> 11
<212> PRT
<213> Mus musculus

<400> 137
Lys Ala Ser Glu Asp Ile Tyr Asn Arg Leu Ala
1 5 10

<210> 138
<211> 7
<212> PRT
<213> Mus musculus

<400> 138
Gly Ala Thr Ser Leu Glu Thr
1 5

<210> 139
<211> 9
<212> PRT
<213> Mus musculus

<400> 139
Gln Gln Tyr Trp Ser Ala Pro Trp Thr
1 5

<210> 140
<211> 321
<212> DNA
<213> Mus musculus

<400> 140
gacatccaga tgacacaatc ttcatcctcc ttttctgtat ctctcggaga cagagtcacc 60
attacttgca aggcaagtga ggacatatat aatcggttag cctggtatca gcagagacca 120
ggaaatgctc ctaggctctt aatatctggt gcaaccagtt tggaaactgg ggttccttca 180
agattcagtg gcagtggctc tggaaaggat tacactctca gcattaccag tcttcagact 240
gaagatgttg ctacttatta ctgtcaacag tattggagtg ctccgtggac gttcggtgga 300
ggcaccaagc tggaaataaa a 321

<210> 141
<211> 117
<212> PRT
<213> Mus musculus

<400> 141
Asp Val His Leu Val Glu Ser Gly Gly Gly Leu Val Gln Pro Gly Gly
1 5 10 15
Ser Arg Lys Leu Ser Cys Ala Ala Ser Gly Phe Thr Phe Ser Ser Phe
20 25 30
Gly Met His Trp Val Arg Gln Ala Pro Glu Arg Gly Leu Glu Trp Val
35 40 45
Ala Tyr Ile Ser Gly Asp Ser Asn Thr Ile Tyr Tyr Thr Asp Thr Val
50 55 60
Lys Gly Arg Phe Thr Ile Ser Arg Asp Asn Pro Lys Asn Thr Leu Phe
65 70 75 80
Leu Gln Met Thr Ser Leu Arg Ser Glu Asp Thr Ala Met Tyr Tyr Cys
85 90 95
Ala Lys Ser Phe Asp Tyr Ala Met Asp Tyr Trp Gly Gln Gly Thr Ser
100 105 110
Val Thr Val Ser Ser
115

<210> 142
<211> 6
<212> PRT
<213> Mus musculus

<400> 142
Ser Ser Phe Gly Met His
1 5

<210> 143
<211> 16
<212> PRT
<213> Mus musculus

<400> 143
Tyr Ile Ser Gly Asp Ser Asn Thr Ile Tyr Tyr Thr Asp Thr Val Lys
1 5 10 15

<210> 144
<211> 8
<212> PRT
<213> Mus musculus

<400> 144
Ser Phe Asp Tyr Ala Met Asp Tyr
1 5

<210> 145
<211> 351
<212> DNA
<213> Mus musculus

<400> 145
gatgtgcatc tggtggagtc tgggggaggc ttagtgcagc ctggagggtc ccggaaactc 60
tcctgtgcag cctctggatt cactttcagt agctttggaa tgcactgggt tcgtcaggct 120
ccagagaggg ggctggagtg ggtcgcatat atcagtggtg acagtaatac catctactat 180
acagacacag tgaagggccg gttcaccatc tccagagaca atcccaagaa caccctgttc 240
ctgcaaatga ccagtctaag gtctgaggac acggccatgt attactgtgc aaaatcgttc 300
gactatgcta tggactactg gggtcaggga acctcagtca ccgtctcctc a 351

<210> 146
<211> 113
<212> PRT
<213> Mus musculus

<400> 146
Asp Ile Val Met Ser Gln Ser Pro Ser Ser Leu Ala Val Ser Val Gly
1 5 10 15
Glu Lys Val Thr Leu Ser Cys Lys Ser Ser Gln Ser Leu Leu Tyr Ser
20 25 30
Thr Asn Gln Lys Asn Tyr Leu Ala Trp Tyr Leu Gln Lys Pro Gly Gln
35 40 45
Ser Pro Lys Leu Leu Ile Tyr Trp Ala Ser Thr Arg Glu Ser Gly Val
50 55 60
Pro Asp Arg Phe Ser Gly Ser Gly Ser Gly Thr Asp Phe Ala Leu Thr
65 70 75 80
Ile Asn Thr Val Lys Ala Glu Asp Leu Ala Val Tyr Tyr Cys Gln Gln
85 90 95
Tyr Tyr Thr Tyr Pro Tyr Thr Phe Gly Gly Gly Thr Lys Leu Glu Ile
100 105 110
Lys

<210> 147
<211> 17
<212> PRT
<213> Mus musculus

<400> 147
Lys Ser Ser Gln Ser Leu Leu Tyr Ser Thr Asn Gln Lys Asn Tyr Leu
1 5 10 15
Ala

<210> 148
<211> 7
<212> PRT
<213> Mus musculus

<400> 148
Trp Ala Ser Thr Arg Glu Ser
1 5

<210> 149
<211> 12
<212> PRT
<213> Mus musculus

<400> 149
Tyr Tyr Cys Gln Gln Tyr Tyr Thr Tyr Pro Tyr Thr
1 5 10

<210> 150
<211> 339
<212> DNA
<213> Mus musculus

<400> 150
gacattgtga tgtcacagtc tccatcctcc ctagctgtgt cagttggaga gaaggttact 60
ttgagctgca agtccagtca gagcctttta tatagtacca atcaaaagaa ctacttggcc 120
tggtacctgc agaaaccagg gcagtctcct aaactgctga tttactgggc atccactagg 180
gaatctgggg tccctgatcg cttctcaggc agtggatctg ggacagattt cgctctcacc 240
atcaatactg tgaaggctga agacctggca gtttattact gtcagcaata ttatacctat 300

ccgtacacgt tcggaggggg gaccaagctg gaaataaaa 339

<210> 151
<211> 119
<212> PRT
<213> Mus musculus

<400> 151
Gln Val Gln Leu Lys Gln Ser Gly Pro Gly Leu Val Gln Pro Ser Gln
1 5 10 15
Ser Leu Ser Ile Thr Cys Thr Val Ser Gly Phe Ser Leu Thr Ser Tyr
20 25 30
Gly Ile His Trp Leu Arg Gln Ser Pro Gly Lys Gly Leu Glu Trp Leu
35 40 45
Gly Val Ile Trp Arg Gly Gly Ser Thr Asp Tyr Asn Ala Ala Phe Met
50 55 60
Ser Arg Leu Ser Ile Thr Lys Asp Asn Ser Lys Ser Gln Val Phe Phe
65 70 75 80
Lys Met Asn Ser Leu Gln Ala Asp Asp Thr Ala Ile Tyr Tyr Cys Ala
85 90 95
Lys Met Arg Val Thr Thr Gly Phe Thr Met Asp Tyr Trp Gly Gln Gly
100 105 110
Thr Ser Val Thr Val Ser Ser
115

<210> 152
<211> 6
<212> PRT
<213> Mus musculus

<400> 152
Thr Ser Tyr Gly Ile His
1 5

<210> 153
<211> 16
<212> PRT
<213> Mus musculus

<400> 153
Val Ile Trp Arg Gly Gly Ser Thr Asp Tyr Asn Ala Ala Phe Met Ser
1 5 10 15

<210> 154
<211> 11
<212> PRT
<213> Mus musculus

<400> 154
Met Arg Val Thr Thr Gly Phe Thr Met Asp Tyr
1 5 10

<210> 155
<211> 357
<212> DNA
<213> Mus musculus

<400> 155
caggtccagc tgaagcagtc aggacctggc ctagtgcagc cctcacagag cctgtccata 60
acctgcacag tctctggttt ctcattaact agctacggta tacactggct tcgccagtct 120
ccaggaaagg gtctggagtg gctgggagtg atatggagag gtggaagcac agactacaat 180
gcagctttca tgtccagact gagcatcacc aaggacaact ccaagagcca agttttcttt 240
aaaatgaaca gtctgcaagc tgatgacact gccatatact actgtgccaa aatgagagtt 300

acgacggggt ttactatgga ctactggggt caaggaacct cagtcaccgt ctcctca 357

<210> 156
<211> 107
<212> PRT
<213> Mus musculus

<400> 156
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Ala Trp Tyr Gln Gln Lys Pro Gly Asn Ala Pro Arg Leu Leu Ile
35 40 45
Ser Gly Ala Thr Ser Leu Glu Thr Gly Val Pro Ser Arg Phe Ser Gly
50 55 60
Ser Gly Ser Gly Lys Asp Tyr Thr Leu Ser Ile Thr Ser Leu Gln Thr
65 70 75 80
Glu Asp Val Ala Thr Tyr Tyr Cys Gln Gln Tyr Trp Ser Asn Pro Tyr
85 90 95
Thr Phe Gly Gly Gly Thr Lys Leu Glu Ile Lys
100 105

<210> 157
<211> 11
<212> PRT
<213> Mus musculus

<400> 157
Lys Ala Ser Glu Asp Ile Tyr Asn Arg Leu Ala
1 5 10

<210> 158
<211> 7
<212> PRT
<213> Mus musculus

<400> 158
Gly Ala Thr Ser Leu Glu Thr
1 5

<210> 159
<211> 9
<212> PRT
<213> Mus musculus

<400> 159
Gln Gln Tyr Trp Ser Asn Pro Tyr Thr
1 5

<210> 160
<211> 321
<212> DNA
<213> Mus musculus

<400> 160
gacatccaga tgacacaatc ttcatcctcc ttttctgtat ctctaggaga cagagtcacc 60
attacttgca aggcaagtga ggacatatat aatcggttag cctggtatca gcagaaacca 120
ggaaatgctc ctaggctctt aatatctggt gcaaccagtt tggaaactgg ggttccttca 180
agattcagtg gcagtggatc tggaaaggat tacactctca gcattaccag tcttcagact 240
gaagatgttg ctacttatta ctgtcaacag tattggagta atccgtacac gttcggaggg 300
gggaccaagc tggaaataaa a 321

<210> 161
<211> 118
<212> PRT
<213> Mus musculus

<400> 161
Gln Val Gln Leu Gln Gln Ser Gly Pro Glu Leu Glu Lys Pro Gly Ala
1 5 10 15
Ser Val Lys Ile Ser Cys Lys Ala Ser Gly Tyr Ser Phe Thr Val Tyr
20 25 30
Asn Ile Asn Trp Val Lys Gln Ser Asn Gly Lys Ser Leu Glu Trp Ile
35 40 45
Gly Asn Ile Asp Pro Tyr Phe Gly Gly Thr Ile Tyr Asn Gln Lys Phe
50 55 60
Lys Asp Lys Ala Thr Leu Thr Val Asp Lys Ser Ser Ser Thr Ala Tyr
65 70 75 80
Met Glu Leu Lys Ser Leu Thr Ser Glu Asp Ser Ala Val Tyr Tyr Cys
85 90 95
Glu Arg Ser Arg Gly Thr Trp Arg Phe Ala Tyr Trp Gly Gln Gly Thr
100 105 110
Leu Val Thr Val Ser Ala
115

<210> 162
<211> 6
<212> PRT
<213> Mus musculus

<400> 162
Thr Val Tyr Asn Ile Asn
1 5

<210> 163
<211> 16
<212> PRT
<213> Mus musculus

<400> 163
Asn Ile Asp Pro Tyr Phe Gly Gly Thr Ile Tyr Asn Gln Lys Phe Lys
1 5 10 15

<210> 164
<211> 9
<212> PRT
<213> Mus musculus

<400> 164
Ser Arg Gly Thr Trp Arg Phe Ala Tyr
1 5

<210> 165
<211> 354
<212> DNA
<213> Mus musculus

<400> 165
caggtccagc tgcagcagtc tggacctgag ttggagaagc ctggcgcttc agtgaagata 60
tcctgcaagg cttctggtta ctcattcact gtctacaaca taaactgggt gaaacagagc 120
aatggaaaga gccttgagtg gattggaaat attgatcctt actttggtgg tactatttac 180
aaccagaaat tcaaggacaa ggccacattg actgtcgaca aatcctccag cacagcctac 240
atggagctca agagcctgac atctgaggac tctgcagtct attactgtga aagatcgagg 300
gggacgtgga ggtttgctta ctggggccaa gggactctgg tcactgtctc tgca 354

<210> 166
<211> 107
<212> PRT
<213> Mus musculus

<400> 166
Asp Ile Val Met Thr Gln Ser Pro Ser Ser Met Ser Val Ser Leu Gly
1 5 10 15
Asp Thr Val Thr Ile Thr Cys His Ala Ser Gln Gly Ile Ser Ser Asn
20 25 30
Ile Gly Trp Leu Gln Gln Lys Pro Gly Lys Ser Phe Lys Gly Leu Ile
35 40 45
Tyr His Gly Ala Thr Leu Glu Asp Gly Ile Pro Ser Arg Phe Ser Gly
50 55 60
Ser Gly Ser Gly Ala Asp Tyr Ser Leu Thr Ile Ser Ser Leu Asp Ser
65 70 75 80
Glu Asp Phe Ala Asp Tyr Tyr Cys Val Gln Tyr Ala Gln Phe Pro Tyr
85 90 95
Thr Phe Gly Gly Gly Thr Lys Leu Glu Ile Lys
100 105

<210> 167
<211> 11
<212> PRT
<213> Mus musculus

<400> 167
His Ala Ser Gln Gly Ile Ser Ser Asn Ile Gly
1 5 10

<210> 168
<211> 7
<212> PRT
<213> Mus musculus

<400> 168
His Gly Ala Thr Leu Glu Asp
1 5

<210> 169
<211> 9
<212> PRT
<213> Mus musculus

<400> 169
Val Gln Tyr Ala Gln Phe Pro Tyr Thr
1 5

<210> 170
<211> 321
<212> DNA
<213> Mus musculus

<400> 170
gacattgtga tgacccagtc tccatcctcc atgtctgtat ctctgggaga cacagtcacc 60
atcacttgcc atgcaagtca gggcattagc agtaatatag ggtggttgca gcagaaacca 120
gggaaatcat ttaagggcct gatctatcat ggagccacct tggaagatgg aattccatca 180
aggttcagtg gcagtggatc tggagcagat tattctctca ccatcagcag cctggactct 240
gaagattttg cagactatta ctgcgtacag tatgctcagt ttccgtacac gttcggaggg 300
gggaccaagc tggaaatcaa a 321

<210> 171
<211> 119

<212> PRT
<213> Mus musculus

<400> 171
Glu Val Gln Leu Lys Gln Ser Gly Pro Gly Leu Met Gln Pro Ser Gln
1 5 10 15
Ser Leu Ser Ile Thr Cys Thr Val Ser Gly Phe Ser Leu Thr Ser Tyr
20 25 30
Gly Ile His Trp Leu Arg Gln Ser Pro Gly Lys Gly Leu Glu Trp Leu
35 40 45
Gly Val Ile Trp Arg Gly Gly Ser Thr Asp Tyr Asn Ala Ala Phe Met
50 55 60
Ser Arg Leu Ser Ile Thr Lys Asp Asn Ser Lys Ser Gln Val Phe Phe
65 70 75 80
Lys Met Asn Ser Leu Gln Gly Asp Asp Thr Ala Ile Tyr Tyr Cys Ala
85 90 95
Lys Gly Lys Val Thr Thr Gly Phe Tyr Phe Asp Phe Trp Gly Gln Gly
100 105 110
Thr Thr Leu Thr Val Ser Ser
115

<210> 172
<211> 6
<212> PRT
<213> Mus musculus

<400> 172
Thr Ser Tyr Gly Ile His
1 5

<210> 173
<211> 16
<212> PRT
<213> Mus musculus

<400> 173
Val Ile Trp Arg Gly Gly Ser Thr Asp Tyr Asn Ala Ala Phe Met Ser
1 5 10 15

<210> 174
<211> 11
<212> PRT
<213> Mus musculus

<400> 174
Gly Lys Val Thr Thr Gly Phe Tyr Phe Asp Phe
1 5 10

<210> 175
<211> 357
<212> DNA
<213> Mus musculus

<400> 175
gaggtgcagc tgaagcagtc aggacctggc ctaatgcagc cctcacagag cctgtccata 60
acctgcacag tctctggttt ctcattaact agctatggta tacactggct tcgtcagtct 120
ccaggaaagg gtctggagtg gctgggagtg atatggagag gtggaagcac agactacaat 180
gcagctttca tgtccagact gagcatcacc aaggacaact ccaagagcca agttttcttt 240
aaaatgaaca gtctgcaagg tgatgacact gccatatact actgtgccaa agggaaggtt 300
acgacggggt tctactttga cttctggggc caaggcacca ctctcacagt ctcctca 357

<210> 176
<211> 107

<212> PRT
<213> Mus musculus

<400> 176
Asp Ile Gln Met Thr Gln Ser Ser Ser Ser Phe Ser Val Ser Leu Gly
1 5 10 15
Asp Arg Val Thr Ile Thr Cys Lys Ala Ser Glu Asp Ile Tyr Asn Arg
20 25 30
Leu Val Trp Tyr Gln Gln Lys Pro Gly Asn Ala Pro Gly Leu Leu Ile
35 40 45
Ser Gly Val Thr Ser Leu Glu Thr Gly Val Pro Ser Arg Phe Ser Gly
50 55 60
Ser Gly Ser Gly Lys Asp Tyr Thr Leu Thr Ile Thr Ser Leu Gln Thr
65 70 75 80
Glu Asp Val Ala Thr Tyr Tyr Cys Gln Gln Tyr Trp Ser Thr Pro Tyr
85 90 95
Thr Phe Gly Gly Gly Thr Lys Leu Glu Ile Lys
100 105

<210> 177
<211> 11
<212> PRT
<213> Mus musculus

<400> 177
Lys Ala Ser Glu Asp Ile Tyr Asn Arg Leu Val
1 5 10

<210> 178
<211> 7
<212> PRT
<213> Mus musculus

<400> 178
Gly Val Thr Ser Leu Glu Thr
1 5

<210> 179
<211> 9
<212> PRT
<213> Mus musculus

<400> 179
Gln Gln Tyr Trp Ser Thr Pro Tyr Thr
1 5

<210> 180
<211> 321
<212> DNA
<213> Mus musculus

<400> 180
gacatccaga tgacacaatc ttcatcctcc ttttctgtat ctctaggaga cagagtcacc 60
attacttgca aggcaagtga ggacatatat aatcggttag tctggtatca gcagaaacca 120
ggaaatgctc ctgggctctt aatatctggt gtaaccagtt tggaaactgg agttccttca 180
agattcagtg gcagtggatc tggaaaggat tacactctca ctattaccag tcttcagact 240
gaagatgttg ctacttatta ctgtcaacag tattggagta ctccgtacac gttcggaggg 300
ggaaccaagc tggaaataaa a 321

<210> 181
<211> 119
<212> PRT
<213> Homo sapiens

<400> 181
Gln Val Gln Leu Val Gln Ser Gly Ala Glu Leu Lys Lys Pro Gly Ser
1 5 10 15
Ser Val Lys Val Ser Cys Lys Val Ser Gly Phe Ser Leu Thr Ser Tyr
20 25 30
Gly Ile His Trp Leu Arg Gln Ala Pro Gly Gln Gly Leu Glu Trp Met
35 40 45
Gly Val Ile Trp Arg Gly Gly Ser Thr Asp Tyr Asn Ala Lys Phe Gln
50 55 60
Gly Arg Val Thr Ile Thr Lys Asp Asn Ser Lys Ser Thr Val Tyr Met
65 70 75 80
Glu Leu Ser Ser Leu Arg Ser Glu Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 182
<211> 10
<212> PRT
<213> Homo sapiens

<400> 182
Gly Phe Ser Leu Thr Ser Tyr Gly Ile His
1 5 10

<210> 183
<211> 16
<212> PRT
<213> Homo sapiens

<400> 183
Val Ile Trp Arg Gly Gly Ser Thr Asp Tyr Asn Ala Lys Phe Gln Gly
1 5 10 15

<210> 184
<211> 11
<212> PRT
<213> Homo sapiens

<400> 184
Gly Lys Val Thr Thr Gly Phe Tyr Phe Asp Phe
1 5 10

<210> 185
<211> 357
<212> DNA
<213> Homo sapiens

<400> 185
caggttcagc tggttcagtc tggcgccgag ctgaagaaac ctggcagctc tgtgaaggtg 60
tcctgcaagg tgtccggctt cagcctgaca agctacggca tccactggct gagacaggcc 120
cctggacaag gcttggaatg gatgggagtg atttggagag gcggcagcac cgactacaac 180
gccaagtttc agggcagagt gaccatcacc aaggacaaca gcaagagcac cgtgtacatg 240
gaactgagca gcctgagaag cgaggacacc gccgtgtact actgcgccaa gggcaaagtg 300
acaaccggct tctacttcga cttctggggc cagggaaccc tggtcacagt ctcttct 357

<210> 186
<211> 119
<212> PRT
<213> Homo sapiens

<400> 186
Gln Val Thr Leu Lys Glu Ser Gly Pro Val Leu Val Lys Pro Thr Glu
1 5 10 15
Thr Leu Thr Leu Thr Cys Thr Val Ser Gly Phe Ser Leu Thr Ser Tyr
20 25 30
Gly Ile His Trp Leu Arg Gln Pro Pro Gly Lys Ala Leu Glu Trp Leu
35 40 45
Ala Val Ile Trp Arg Gly Gly Ser Thr Asp Tyr Asn Ala Ser Leu Lys
50 55 60
Ser Arg Leu Thr Ile Ser Lys Asp Asn Ser Lys Ser Gln Val Val Leu
65 70 75 80
Thr Met Thr Asn Met Asp Pro Val Asp Thr Ala Thr Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 187
<211> 10
<212> PRT
<213> Homo sapiens

<400> 187
Gly Phe Ser Leu Thr Ser Tyr Gly Ile His
1 5 10

<210> 188
<211> 16
<212> PRT
<213> Homo sapiens

<400> 188
Val Ile Trp Arg Gly Gly Ser Thr Asp Tyr Asn Ala Ser Leu Lys Ser
1 5 10 15

<210> 189
<211> 11
<212> PRT
<213> Homo sapiens

<400> 189
Gly Lys Val Thr Thr Gly Phe Tyr Phe Asp Phe
1 5 10

<210> 190
<211> 357
<212> DNA
<213> Homo sapiens

<400> 190
caagtgaccc tgaaagaaag cggccctgtg ctggtcaagc ccaccgaaac actgaccctg 60
acctgtaccg tgtccggctt cagcctgaca agctacggaa tccactggct gagacagcct 120
ccaggcaagg ctctggaatg gctggccgtt atttggagag gcggcagcac agactacaac 180
gccagcctga agtccagact gaccatcagc aaggacaaca gcaagagcca ggtggtgctg 240
accatgacca acatggaccc tgtggacacc gccacctact actgcgctaa gggcaaagtg 300
accaccggct tctacttcga cttttggggc cagggcaccc tggtcacagt ctcttct 357

<210> 191
<211> 119
<212> PRT
<213> Homo sapiens

<400> 191
Glu Val Gln Leu Leu Glu Ser Gly Gly Gly Leu Val Gln Pro Gly Gly
1 5 10 15
Ser Leu Arg Leu Ser Cys Ala Val Ser Gly Phe Ser Leu Thr Ser Tyr
20 25 30
Gly Ile His Trp Leu Arg Gln Ala Pro Gly Lys Gly Leu Glu Trp Val
35 40 45
Ser Val Ile Trp Arg Gly Gly Ser Thr Asp Tyr Asn Ala Ser Val Lys
50 55 60
Gly Arg Phe Thr Ile Ser Lys Asp Asn Ser Lys Ser Thr Val Tyr Leu
65 70 75 80
Gln Met Asn Ser Leu Arg Ala Glu Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 192
<211> 10
<212> PRT
<213> Homo sapiens

<400> 192
Gly Phe Ser Leu Thr Ser Tyr Gly Ile His
1 5 10

<210> 193
<211> 16
<212> PRT
<213> Homo sapiens

<400> 193
Val Ile Trp Arg Gly Gly Ser Thr Asp Tyr Asn Ala Ser Val Lys Gly
1 5 10 15

<210> 194
<211> 11
<212> PRT
<213> Homo sapiens

<400> 194
Gly Lys Val Thr Thr Gly Phe Tyr Phe Asp Phe
1 5 10

<210> 195
<211> 357
<212> DNA
<213> Homo sapiens

<400> 195
gaagtgcagc tgctggaatc tggcggagga ctggttcaac ctggcggctc tctgagactg 60
agctgtgctg tgtctggctt cagcctgacc agctacggaa tccactggct gagacaggcc 120
cctggcaaag gactggaatg ggtgtccgtg atttggagag gcggcagcac agactacaac 180
gcctctgtga agggcagatt caccatcagc aaggacaaca gcaagagcac cgtgtacctg 240
cagatgaaca gcctgagagc cgaggacacc gccgtgtact actgtgccaa gggcaaagtg 300
accaccggct tctacttcga cttttggggc cagggcaccc tggtcacagt ctcttct 357

<210> 196
<211> 119
<212> PRT
<213> Homo sapiens

<400> 196
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Leu Arg Gln Pro Pro Gly Lys Gly Leu Glu Trp Ile
35 40 45
Gly Val Ile Trp Arg Gly Gly Ser Thr Asp Tyr Asn Ala Ser Leu Lys
50 55 60
Ser Arg Val Thr Ile Ser Lys Asp Asn Ser Lys Ser Gln Val Ser Leu
65 70 75 80
Lys Leu Ser Ser Val Thr Ala Ala Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 197
<211> 10
<212> PRT
<213> Homo sapiens

<400> 197
Gly Phe Ser Leu Thr Ser Tyr Gly Ile His
1 5 10

<210> 198
<211> 16
<212> PRT
<213> Homo sapiens

<400> 198
Val Ile Trp Arg Gly Gly Ser Thr Asp Tyr Asn Ala Ser Leu Lys Ser
1 5 10 15

<210> 199
<211> 11
<212> PRT
<213> Homo sapiens

<400> 199
Gly Lys Val Thr Thr Gly Phe Tyr Phe Asp Phe
1 5 10

<210> 200
<211> 357
<212> DNA
<213> Homo sapiens

<400> 200
caggttcagc tgcaagagtc tggacctggc ctggtcaagc ctagcgagac actgagcctg 60
acctgtaccg tgtctggctt cagcctgacc agctacggaa tccactggct gagacagcct 120
ccaggcaaag gcctggaatg gatcggagtg atttggagag gcggcagcac cgactacaac 180
gccagcctga agtccagagt gaccatcagc aaggacaaca gcaagagcca ggtgtccctg 240
aagctgagca gcgtgacagc cgctgatacc gccgtgtact actgcgccaa gggcaaagtg 300
accaccggct tctacttcga cttttggggc cagggcaccc tggtcacagt ctcttct 357

<210> 201
<211> 119
<212> PRT
<213> Homo sapiens

<400> 201
Glu Val Gln Leu Val Gln Ser Gly Ala Glu Leu Lys Lys Pro Gly Glu
1 5 10 15
Ser Leu Lys Ile Ser Cys Lys Val Ser Gly Phe Ser Leu Thr Ser Tyr
20 25 30
Gly Ile His Trp Leu Arg Gln Met Pro Gly Lys Gly Leu Glu Trp Met
35 40 45
Gly Val Ile Trp Arg Gly Gly Ser Thr Asp Tyr Asn Ala Ser Phe Gln
50 55 60
Gly Gln Val Thr Ile Ser Lys Asp Asn Ser Lys Ser Thr Val Tyr Leu
65 70 75 80
Gln Trp Ser Ser Leu Lys Ala Ser Asp Thr Ala Met Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 202
<211> 10
<212> PRT
<213> Homo sapiens

<400> 202
Gly Phe Ser Leu Thr Ser Tyr Gly Ile His
1 5 10

<210> 203
<211> 16
<212> PRT
<213> Homo sapiens

<400> 203
Val Ile Trp Arg Gly Gly Ser Thr Asp Tyr Asn Ala Ser Phe Gln Gly
1 5 10 15

<210> 204
<211> 11
<212> PRT
<213> Homo sapiens

<400> 204
Gly Lys Val Thr Thr Gly Phe Tyr Phe Asp Phe
1 5 10

<210> 205
<211> 357
<212> DNA
<213> Homo sapiens

<400> 205
gaagtgcagc tggttcagtc tggcgccgag ctgaagaagc ctggcgagag cctgaagatc 60
agctgcaagg tgtccggctt cagcctgacc tcttacggca tccactggct gagacagatg 120
cctggcaaag gcctggaatg gatgggagtg atttggagag gcggcagcac cgactacaac 180
gccagctttc agggccaagt gaccatcagc aaggacaaca gcaagagcac cgtgtacctg 240
cagtggtcca gcctgaaggc cagcgacaca gccatgtact actgcgccaa gggcaaagtg 300
acaaccggct tctacttcga cttctggggc cagggaaccc tggtcacagt ctcttct 357

<210> 206
<211> 119
<212> PRT
<213> Homo sapiens

<400> 206
Gln Val Gln Leu Val Gln Ser Gly Ser Glu Leu Lys Lys Pro Gly Ala
1 5 10 15
Ser Val Lys Val Ser Cys Lys Val Ser Gly Phe Ser Leu Thr Ser Tyr
20 25 30
Gly Ile His Trp Leu Arg Gln Ala Pro Gly Gln Gly Leu Glu Trp Met
35 40 45
Gly Val Ile Trp Arg Gly Gly Ser Thr Asp Tyr Asn Ala Gly Phe Thr
50 55 60
Gly Arg Phe Val Ile Ser Lys Asp Asn Ser Lys Ser Thr Val Tyr Leu
65 70 75 80
Gln Ile Cys Ser Leu Lys Ala Glu Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 207
<211> 10
<212> PRT
<213> Homo sapiens

<400> 207
Gly Phe Ser Leu Thr Ser Tyr Gly Ile His
1 5 10

<210> 208
<211> 16
<212> PRT
<213> Homo sapiens

<400> 208
Val Ile Trp Arg Gly Gly Ser Thr Asp Tyr Asn Ala Gly Phe Thr Gly
1 5 10 15

<210> 209
<211> 11
<212> PRT
<213> Homo sapiens

<400> 209
Gly Lys Val Thr Thr Gly Phe Tyr Phe Asp Phe
1 5 10

<210> 210
<211> 357
<212> DNA
<213> Homo sapiens

<400> 210
caggttcagc tggtgcagtc tggcagcgag ctgaagaaac ctggcgcctc tgtgaaggtg 60
tcctgcaagg tgtccggctt cagcctgaca agctacggca tccactggct gagacaggcc 120
cctggacaag gcttggaatg gatgggagtg atttggagag gcggcagcac cgattacaac 180
gccggcttca caggcagatt cgtgatcagc aaggacaaca gcaagagcac cgtgtacctg 240
cagatctgta gcctgaaggc cgaggacacc gccgtgtact actgtgccaa gggcaaagtg 300
accaccggct tctacttcga cttttggggc cagggcaccc tggtcacagt ctcttct 357

<210> 211
<211> 119
<212> PRT
<213> Homo sapiens

<400> 211
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Ile Arg Gln Pro Pro Gly Lys Gly Leu Glu Trp Ile
35 40 45
Gly Val Ile Trp Arg Gly Gly Ser Thr Asp Tyr Asn Pro Ser Leu Lys
50 55 60
Ser Arg Val Thr Ile Ser Lys Asp Asn Ser Lys Ser Gln Val Ser Leu
65 70 75 80
Lys Leu Ser Ser Val Thr Ala Ala Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 212
<211> 10
<212> PRT
<213> Homo sapiens

<400> 212
Gly Phe Ser Leu Thr Ser Tyr Gly Ile His
1 5 10

<210> 213
<211> 16
<212> PRT
<213> Homo sapiens

<400> 213
Val Ile Trp Arg Gly Gly Ser Thr Asp Tyr Asn Pro Ser Leu Lys Ser
1 5 10 15

<210> 214
<211> 11
<212> PRT
<213> Homo sapiens

<400> 214
Gly Lys Val Thr Thr Gly Phe Tyr Phe Asp Phe
1 5 10

<210> 215
<211> 357
<212> DNA
<213> Homo sapiens

<400> 215
caggttcagc tgcaagagtc tggacctggc ctggtcaagc ctagcgagac actgagcctg 60
acctgtaccg tgtctggctt cagcctgacc agctacggaa tccactggat cagacagcct 120
ccaggcaaag gcctggaatg gatcggagtg atttggagag gcggcagcac cgactacaac 180
cccagcctga agtccagagt gaccatcagc aaggacaaca gcaagagcca ggtgtccctg 240
aagctgagca gcgtgacagc cgctgatacc gccgtgtact actgcgccaa gggcaaagtg 300
accaccggct tctacttcga cttttggggc cagggcaccc tggtcacagt ctcttct 357

<210> 216
<211> 119
<212> PRT
<213> Homo sapiens

<400> 216
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Leu Arg Gln Pro Pro Gly Lys Gly Leu Glu Trp Ile
35 40 45
Gly Val Ile Trp Arg Gly Gly Ser Thr Asp Tyr Asn Pro Ser Leu Lys
50 55 60
Ser Arg Val Thr Ile Ser Lys Asp Thr Ser Lys Ser Gln Val Ser Leu
65 70 75 80
Lys Leu Ser Ser Val Thr Ala Ala Asp Thr Ala Val Tyr Tyr Cys Ala
85 90 95
Lys Gly Lys Val Thr Thr Gly Phe Tyr Phe Asp Phe Trp Gly Gln Gly
100 105 110
Thr Leu Val Thr Val Ser Ser
115

<210> 217
<211> 10
<212> PRT
<213> Homo sapiens

<400> 217
Gly Phe Ser Leu Thr Ser Tyr Gly Ile His
1 5 10

<210> 218
<211> 16
<212> PRT
<213> Homo sapiens

<400> 218
Val Ile Trp Arg Gly Gly Ser Thr Asp Tyr Asn Pro Ser Leu Lys Ser
1 5 10 15

<210> 219
<211> 11
<212> PRT
<213> Homo sapiens

<400> 219
Gly Lys Val Thr Thr Gly Phe Tyr Phe Asp Phe
1 5 10

<210> 220
<211> 357
<212> DNA
<213> Homo sapiens

<400> 220
caggttcagc tgcaagagtc tggacctggc ctggtcaagc ctagcgagac actgagcctg 60
acctgtaccg tgtctggctt cagcctgacc agctacggaa tccactggct gagacagcct 120
ccaggcaaag gcctggaatg gatcggagtg atttggagag gcggcagcac cgactacaac 180
cccagcctga agtccagagt gaccatcagc aaggacacca gcaagagcca ggtgtccctg 240
aagctgagca gcgtgacagc cgctgatacc gccgtgtact actgcgccaa gggcaaagtg 300
accaccggct tctacttcga cttttggggc cagggcaccc tggtcacagt ctcttct 357

<210> 221
<211> 119
<212> PRT
<213> Homo sapiens

<400> 221
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Ile Arg Gln Pro Pro Gly Lys Gly Leu Glu Trp Ile
35 40 45
Gly Val Ile Trp Arg Gly Gly Ser Thr Asp Tyr Asn Pro Ser Leu Lys
50 55 60
Ser Arg Val Thr Ile Ser Lys Asp Thr Ser Lys Ser Gln Val Ser Leu
65 70 75 80
Lys Leu Ser Ser Val Thr Ala Ala Asp Thr Ala Val Tyr Tyr Cys Ala
85 90 95
Arg Gly Lys Val Thr Thr Gly Phe Tyr Phe Asp Phe Trp Gly Gln Gly
100 105 110
Thr Leu Val Thr Val Ser Ser
115

<210> 222
<211> 10
<212> PRT
<213> Homo sapiens

<400> 222
Gly Phe Ser Leu Thr Ser Tyr Gly Ile His
1 5 10

<210> 223
<211> 16
<212> PRT
<213> Homo sapiens

<400> 223
Val Ile Trp Arg Gly Gly Ser Thr Asp Tyr Asn Pro Ser Leu Lys Ser
1 5 10 15

<210> 224
<211> 11
<212> PRT
<213> Homo sapiens

<400> 224
Gly Lys Val Thr Thr Gly Phe Tyr Phe Asp Phe
1 5 10

<210> 225
<211> 357
<212> DNA
<213> Homo sapiens

<400> 225
caggttcagc tgcaagagtc tggacctggc ctggtcaagc ctagcgagac actgagcctg 60
acctgtaccg tgtctggctt cagcctgacc agctacggaa tccactggat cagacagcct 120
ccaggcaaag gcctggaatg gatcggagtg atttggagag gcggcagcac cgactacaac 180
cccagcctga agtccagagt gaccatcagc aaggacacca gcaagagcca ggtgtccctg 240
aagctgagca gcgtgacagc cgctgatacc gccgtgtact actgcgccag aggcaaagtg 300
accaccggct tctacttcga cttttggggc cagggcaccc tggtcacagt ctcttct 357

<210> 226
<211> 119
<212> PRT
<213> Homo sapiens

<400> 226
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Ile Arg Gln Pro Pro Gly Lys Gly Leu Glu Trp Ile
35 40 45
Gly Val Ile Trp Ala Gly Gly Ser Thr Asp Tyr Asn Pro Ser Leu Lys
50 55 60
Ser Arg Val Thr Ile Ser Lys Asp Thr Ser Lys Ser Gln Val Ser Leu
65 70 75 80
Lys Leu Ser Ser Val Thr Ala Ala Asp Thr Ala Val Tyr Tyr Cys Ala
85 90 95
Arg Gly Lys Val Thr Thr Gly Phe Tyr Phe Asp Phe Trp Gly Gln Gly
100 105 110
Thr Leu Val Thr Val Ser Ser
115

<210> 227
<211> 10
<212> PRT
<213> Homo sapiens

<400> 227
Gly Phe Ser Leu Thr Ser Tyr Gly Ile His
1 5 10

<210> 228
<211> 16
<212> PRT
<213> Homo sapiens

<400> 228
Val Ile Trp Ala Gly Gly Ser Thr Asp Tyr Asn Pro Ser Leu Lys Ser
1 5 10 15

<210> 229
<211> 11
<212> PRT
<213> Homo sapiens

<400> 229
Gly Lys Val Thr Thr Gly Phe Tyr Phe Asp Phe
1 5 10

<210> 230
<211> 357
<212> DNA
<213> Homo sapiens

<400> 230
caggttcagc tgcaagagtc tggacctggc ctggtcaagc ctagcgagac actgagcctg 60
acctgtaccg tgtctggctt cagcctgacc agctacggaa tccactggat cagacagcct 120
ccaggcaaag gcctggaatg gatcggagtg atttgggctg gcggcagcac cgactacaac 180
cccagcctga agtccagagt gaccatcagc aaggacacca gcaagagcca ggtgtccctg 240
aagctgagca gcgtgacagc cgctgatacc gccgtgtact actgcgccag aggcaaagtg 300
accaccggct tctacttcga cttttggggc cagggcaccc tggtcacagt ctcttct 357

<210> 231
<211> 119
<212> PRT
<213> Homo sapiens

<400> 231
Gln Val Gln Leu Gln Glu Ser Gly Pro Gly Leu Val Lys Pro Ser Glu
1 5 10 15
Thr Leu Ser Leu Thr Cys Thr Val Ser Gly Phe Ser Leu Thr Ser Tyr
20 25 30
Gly Ile His Trp Ile Arg Gln Pro Pro Gly Lys Gly Leu Glu Trp Ile
35 40 45
Gly Val Ile Trp Arg Gly Gly Ser Thr Asp Tyr Asn Pro Ser Leu Gln
50 55 60
Ser Arg Val Thr Ile Ser Lys Asp Thr Ser Lys Ser Gln Val Ser Leu
65 70 75 80
Lys Leu Ser Ser Val Thr Ala Ala Asp Thr Ala Val Tyr Tyr Cys Ala
85 90 95
Arg Gly Lys Val Thr Thr Gly Phe Tyr Phe Asp Phe Trp Gly Gln Gly
100 105 110
Thr Leu Val Thr Val Ser Ser
115

<210> 232
<211> 10
<212> PRT
<213> Homo sapiens

<400> 232
Gly Phe Ser Leu Thr Ser Tyr Gly Ile His
1 5 10

<210> 233
<211> 16
<212> PRT
<213> Homo sapiens

<400> 233
Val Ile Trp Arg Gly Gly Ser Thr Asp Tyr Asn Pro Ser Leu Gln Ser
1 5 10 15

<210> 234
<211> 11
<212> PRT
<213> Homo sapiens

<400> 234
Gly Lys Val Thr Thr Gly Phe Tyr Phe Asp Phe
1 5 10

<210> 235
<211> 357
<212> DNA
<213> Homo sapiens

<400> 235
caggttcagc tgcaagagtc tggacctggc ctggtcaagc ctagcgagac actgagcctg 60
acctgtaccg tgtctggctt cagcctgacc agctacggaa tccactggat cagacagcct 120
ccaggcaaag gcctggaatg gatcggagtg atttggagag gcggcagcac cgactacaac 180
cccagcctgc agtccagagt gaccatcagc aaggacacca gcaagagcca ggtgtccctg 240
aagctgagca gcgtgacagc cgctgatacc gccgtgtact actgcgccag aggcaaagtg 300
accaccggct tctacttcga cttttggggc cagggcaccc tggtcacagt ctcttct 357

<210> 236
<211> 107
<212> PRT
<213> Homo sapiens

<400> 236
Asp Ile Gln Met Thr Gln Ser Pro Ser Ser Leu Ser Ala Ser Val Gly
1 5 10 15
Asp Arg Val Thr Ile Thr Cys Arg Ala Ser Glu Asp Ile Tyr Asn Arg
20 25 30
Leu Val Trp Tyr Gln Gln Lys Pro Gly Lys Ala Pro Lys Leu Leu Ile
35 40 45
Ser Gly Val Thr Ser Leu Glu Thr Gly Val Pro Ser Arg Phe Ser Gly
50 55 60
Ser Gly Ser Gly Lys Asp Tyr Thr Leu Thr Ile Ser Ser Leu Gln Pro
65 70 75 80
Glu Asp Phe Ala Thr Tyr Tyr Cys Gln Gln Tyr Trp Ser Thr Pro Tyr
85 90 95
Thr Phe Gly Gln Gly Thr Lys Val Glu Ile Lys
100 105

<210> 237
<211> 11
<212> PRT
<213> Homo sapiens

<400> 237
Arg Ala Ser Glu Asp Ile Tyr Asn Arg Leu Val
1 5 10

<210> 238
<211> 7
<212> PRT
<213> Homo sapiens

<400> 238
Gly Val Thr Ser Leu Glu Thr
1 5

<210> 239
<211> 9
<212> PRT
<213> Homo sapiens

<400> 239
Gln Gln Tyr Trp Ser Thr Pro Tyr Thr
1 5

<210> 240
<211> 321
<212> DNA
<213> Homo sapiens

<400> 240
gacatccaga tgacacagag ccctagcagc ctgtctgcca gcgtgggaga cagagtgacc 60
atcacctgta gagccagcga ggacatctac aacagactcg tgtggtatca gcagaagccc 120
ggcaaggctc ccaagctgct gattagcgga gtgaccagcc tggaaacagg cgtgccaagc 180
agattcagcg gctctggctc tggcaaggac tacaccctga ccatctctag cctgcagcct 240
gaggacttcg ccacctacta ctgccagcag tactggtcta ccccttacac cttcggccag 300
ggcaccaagg tggaaatcaa g 321

<210> 241
<211> 107
<212> PRT
<213> Homo sapiens

<400> 241

Asp Ile Val Met Thr Gln Ser Pro Leu Ser Leu Pro Val Thr Pro Gly
1               5                   10                  15
Glu Pro Ala Ser Ile Ser Cys Arg Ala Ser Glu Asp Ile Tyr Asn Arg
            20                  25                  30
Leu Val Trp Tyr Leu Gln Lys Pro Gly Gln Ser Pro Gln Leu Leu Ile
        35                  40                  45
Ser Gly Val Thr Ser Leu Glu Thr Gly Val Pro Asp Arg Phe Ser Gly
    50                  55                  60
Ser Gly Ser Gly Lys Asp Tyr Thr Leu Lys Ile Ser Arg Val Glu Ala
65                  70                  75                  80
Glu Asp Val Gly Val Tyr Tyr Cys Gln Gln Tyr Trp Ser Thr Pro Tyr
                85                  90                  95
Thr Phe Gly Gln Gly Thr Lys Val Glu Ile Lys
            100                 105

<210> 242
<211> 11
<212> PRT
<213> Homo sapiens

<400> 242
Arg Ala Ser Glu Asp Ile Tyr Asn Arg Leu Val
1               5                   10

<210> 243
<211> 7
<212> PRT
<213> Homo sapiens

<400> 243
Gly Val Thr Ser Leu Glu Thr
1               5

<210> 244
<211> 9
<212> PRT
<213> Homo sapiens

<400> 244
Gln Gln Tyr Trp Ser Thr Pro Tyr Thr
1               5

<210> 245
<211> 321
<212> DNA
<213> Homo sapiens

<400> 245
gacatcgtga tgacacagag ccctctgagc ctgcctgtga cacctggcga acctgccagc 60
atcagctgta gagccagcga ggacatctac aacagactcg tgtggtatct gcagaagccc 120
ggccagtctc ctcagctgct gattagcgga gtgaccagcc tggaaacagg cgtgccagac 180
agattcagcg gctctggctc tggcaaggac tacaccctga agatcagcag agtggaagcc 240
gaggacgtgg gcgtgtacta ctgccagcag tactggtcta ccccttacac cttcggccag 300
ggcaccaagg tggaaatcaa g 321

<210> 246
<211> 107
<212> PRT
<213> Homo sapiens

<400> 246
Glu Ile Val Met Thr Gln Ser Pro Pro Thr Leu Ser Leu Ser Pro Gly
1               5                   10                  15

```
Glu Arg Val Thr Leu Ser Cys Arg Ala Ser Glu Asp Ile Tyr Asn Arg
            20                  25                  30
Leu Val Trp Tyr Gln Gln Lys Pro Gly Gln Ala Pro Arg Leu Leu Ile
        35                  40                  45
Ser Gly Val Thr Ser Leu Glu Thr Ser Ile Pro Ala Arg Phe Ser Gly
    50                  55                  60
Ser Gly Ser Gly Lys Asp Tyr Thr Leu Thr Ile Ser Ser Leu Gln Pro
65                  70                  75                  80
Glu Asp Phe Ala Val Tyr Tyr Cys Gln Gln Tyr Trp Ser Thr Pro Tyr
            85                  90                  95
Thr Phe Gly Gln Gly Thr Lys Val Glu Ile Lys
            100                 105

<210>  247
<211>  11
<212>  PRT
<213>  Homo sapiens

<400>  247
Arg Ala Ser Glu Asp Ile Tyr Asn Arg Leu Val
1               5                   10

<210>  248
<211>  7
<212>  PRT
<213>  Homo sapiens

<400>  248
Gly Val Thr Ser Leu Glu Thr
1               5

<210>  249
<211>  9
<212>  PRT
<213>  Homo sapiens

<400>  249
Gln Gln Tyr Trp Ser Thr Pro Tyr Thr
1               5

<210>  250
<211>  321
<212>  DNA
<213>  Homo sapiens

<400>  250
gagatcgtga tgacacagag ccctccaaca ctgtctctga gccctggcga gagagtgacc       60
ctgagctgta gagccagcga ggacatctac aacagactcg tgtggtatca gcagaagccc      120
ggccaggctc ctagactgct gattagcgga gtgaccagcc tggaaactag catccccgcc      180
agattcagcg gctctggctc tggcaaggac tacaccctga caatcagcag cctgcagcct      240
gaggacttcg ccgtgtacta ctgccagcag tactggtcta ccccttacac cttcggccag      300
ggcaccaagg tggaaatcaa g                                                321

<210>  251
<211>  107
<212>  PRT
<213>  Homo sapiens

<400>  251
Glu Ile Val Met Thr Gln Ser Pro Gly Thr Leu Ser Leu Ser Pro Gly
1               5                   10                  15
Glu Arg Ala Thr Leu Ser Cys Arg Ala Ser Glu Asp Ile Tyr Asn Arg
            20                  25                  30
```

```
Leu Val Trp Tyr Gln Gln Lys Pro Gly Gln Ala Pro Arg Leu Leu Ile
        35                  40                  45
Ser Gly Val Thr Ser Leu Glu Thr Gly Ile Pro Asp Arg Phe Ser Gly
    50                  55                  60
Ser Gly Ser Gly Lys Asp Tyr Thr Leu Thr Ile Ser Arg Leu Glu Pro
65                  70                  75                  80
Glu Asp Phe Ala Val Tyr Tyr Cys Gln Gln Tyr Trp Ser Thr Pro Tyr
                85                  90                  95
Thr Phe Gly Gln Gly Thr Lys Val Glu Ile Lys
            100                 105

<210>  252
<211>  11
<212>  PRT
<213>  Homo sapiens

<400>  252
Arg Ala Ser Glu Asp Ile Tyr Asn Arg Leu Val
1               5                   10

<210>  253
<211>  7
<212>  PRT
<213>  Homo sapiens

<400>  253
Gly Val Thr Ser Leu Glu Thr
1               5

<210>  254
<211>  9
<212>  PRT
<213>  Homo sapiens

<400>  254
Gln Gln Tyr Trp Ser Thr Pro Tyr Thr
1               5

<210>  255
<211>  321
<212>  DNA
<213>  Homo sapiens

<400>  255
gagatcgtga tgacacagag ccccggcaca ctgtcactgt ctccaggcga aagagccaca        60
ctgagctgta gagccagcga ggacatctac aacagactcg tgtggtatca gcagaagccc       120
ggccaggctc ctagactgct gattagcgga gtgaccagcc tggaaacagg catccccgac       180
agattcagcg gctctggctc tggcaaggac tacaccctga ccatcagcag actggaaccc       240
gaggacttcg ccgtgtacta ctgccagcag tactggtcta ccccttacac cttcggccag       300
ggcaccaagg tggaaatcaa g                                                 321

<210>  256
<211>  107
<212>  PRT
<213>  Homo sapiens

<400>  256
Asp Ile Val Met Thr Gln Ser Pro Asp Ser Leu Ala Val Ser Leu Gly
1               5                   10                  15
Glu Arg Ala Thr Ile Asn Cys Lys Ala Ser Glu Asp Ile Tyr Asn Arg
            20                  25                  30
Leu Val Trp Tyr Gln Gln Lys Pro Gly Gln Pro Pro Lys Leu Leu Ile
        35                  40                  45
```

```
Ser Gly Val Thr Ser Leu Glu Thr Gly Val Pro Asp Arg Phe Ser Gly
    50                  55                  60
Ser Gly Ser Gly Lys Asp Tyr Thr Leu Thr Ile Ser Ser Leu Gln Ala
65                  70                  75                  80
Glu Asp Val Ala Val Tyr Tyr Cys Gln Gln Tyr Trp Ser Thr Pro Tyr
                85                  90                  95
Thr Phe Gly Gln Gly Thr Lys Val Glu Ile Lys
            100                 105
```

<210> 257
<211> 11
<212> PRT
<213> Homo sapiens

<400> 257

```
Lys Ala Ser Glu Asp Ile Tyr Asn Arg Leu Val
1               5                   10
```

<210> 258
<211> 7
<212> PRT
<213> Homo sapiens

<400> 258

```
Gly Val Thr Ser Leu Glu Thr
1               5
```

<210> 259
<211> 9
<212> PRT
<213> Homo sapiens

<400> 259

```
Gln Gln Tyr Trp Ser Thr Pro Tyr Thr
1               5
```

<210> 260
<211> 321
<212> DNA
<213> Homo sapiens

<400> 260

```
gacatcgtga tgacacagag ccctgatagc ctggccgtgt ctctgggaga gagagccacc        60
atcaactgca aggccagcga ggacatctac aacagactcg tgtggtatca gcagaagccc       120
ggccagcctc ctaagctgct gattagcgga gtgaccagcc tggaaacagg cgtgccagac       180
agattcagcg gctctggctc tggcaaggac tacaccctga caatcagctc cctgcaggcc       240
gaggatgtgg ccgtgtacta ctgccagcag tactggtcta ccccttacac cttcggccag       300
ggcaccaagg tggaaatcaa g                                                 321
```

<210> 261
<211> 107
<212> PRT
<213> Homo sapiens

<400> 261

```
Glu Ile Val Met Thr Gln Ser Pro Pro Thr Leu Ser Leu Ser Pro Gly
1               5                   10                  15
Glu Arg Val Thr Leu Ser Cys Arg Ala Ser Glu Asp Ile Tyr Asn Arg
            20                  25                  30
Leu Val Trp Tyr Gln Gln Lys Pro Gly Gln Ala Pro Arg Leu Leu Ile
        35                  40                  45
Ser Gly Val Thr Ser Leu Glu Thr Ser Ile Pro Ala Arg Phe Ser Gly
    50                  55                  60
```

Ser Gly Ser Gly Thr Asp Tyr Thr Leu Thr Ile Ser Ser Leu Gln Pro
65 70 75 80
Glu Asp Phe Ala Val Tyr Tyr Cys Gln Gln Tyr Trp Ser Thr Pro Tyr
85 90 95
Thr Phe Gly Gln Gly Thr Lys Val Glu Ile Lys
100 105

<210> 262
<211> 11
<212> PRT
<213> Homo sapiens

<400> 262
Arg Ala Ser Glu Asp Ile Tyr Asn Arg Leu Val
1 5 10

<210> 263
<211> 7
<212> PRT
<213> Homo sapiens

<400> 263
Gly Val Thr Ser Leu Glu Thr
1 5

<210> 264
<211> 9
<212> PRT
<213> Homo sapiens

<400> 264
Gln Gln Tyr Trp Ser Thr Pro Tyr Thr
1 5

<210> 265
<211> 321
<212> DNA
<213> Homo sapiens

<400> 265
gagatcgtga tgacacagag ccctccaaca ctgtctctga gccctggcga gagagtgacc 60
ctgagctgta gagccagcga ggacatctac aacagactcg tgtggtatca gcagaagccc 120
ggccaggctc ctagactgct gattagcgga gtgaccagcc tggaaactag catccccgcc 180
agattcagcg gctctggctc tggcacagac tacaccctga caatcagcag cctgcagcct 240
gaggacttcg ccgtgtacta ctgccagcag tactggtcta ccccttacac cttcggccag 300
ggcaccaagg tggaaatcaa g 321

<210> 266
<211> 107
<212> PRT
<213> Homo sapiens

<400> 266
Glu Ile Val Met Thr Gln Ser Pro Pro Thr Leu Ser Leu Ser Pro Gly
1 5 10 15
Glu Arg Val Thr Leu Ser Cys Arg Ala Ser Glu Asp Ile Tyr Asn Tyr
20 25 30
Leu Val Trp Tyr Gln Gln Lys Pro Gly Gln Ala Pro Arg Leu Leu Ile
35 40 45
Ser Gly Val Thr Ser Leu Glu Thr Ser Ile Pro Ala Arg Phe Ser Gly
50 55 60
Ser Gly Ser Gly Thr Asp Tyr Thr Leu Thr Ile Ser Ser Leu Gln Pro
65 70 75 80

```
Glu Asp Phe Ala Val Tyr Tyr Cys Gln Gln Tyr Trp Ser Thr Pro Tyr
                85                  90                  95
Thr Phe Gly Gln Gly Thr Lys Val Glu Ile Lys
            100                 105

<210>  267
<211>  11
<212>  PRT
<213>  Homo sapiens

<400>  267
Arg Ala Ser Glu Asp Ile Tyr Asn Tyr Leu Val
1               5                   10

<210>  268
<211>  7
<212>  PRT
<213>  Homo sapiens

<400>  268
Gly Val Thr Ser Leu Glu Thr
1               5

<210>  269
<211>  9
<212>  PRT
<213>  Homo sapiens

<400>  269
Gln Gln Tyr Trp Ser Thr Pro Tyr Thr
1               5

<210>  270
<211>  321
<212>  DNA
<213>  Homo sapiens

<400>  270
gagatcgtga tgacacagag ccctccaaca ctgtctctga gccctggcga gagagtgacc       60
ctgagctgta gagccagcga ggacatctac aactacctcg tgtggtatca gcagaagccc      120
ggccaggctc ctagactgct gattagcgga gtgaccagcc tggaaactag catccccgcc      180
agattcagcg gctctggctc tggcacagac tacaccctga caatcagcag cctgcagcct      240
gaggacttcg ccgtgtacta ctgccagcag tactggtcta ccccttacac cttcggccag      300
ggcaccaagg tggaaatcaa g                                                321
```